(19) Europäisches Patentamt
European Patent Office
Office européen des brevets

(11) EP 1 310 490 A1

(12) EUROPEAN PATENT APPLICATION
published in accordance with Art. 158(3) EPC

(43) Date of publication:
**14.05.2003 Bulletin 2003/20**

(21) Application number: **01949909.4**

(22) Date of filing: **04.07.2001**

(51) Int Cl.[7]: **C07D 223/16**, C07D 401/12, C07D 405/12, C07D 413/12, A61K 31/55, A61P 9/08, A61P 9/10, A61P 9/12, A61P 43/00

(86) International application number:
**PCT/JP01/05784**

(87) International publication number:
**WO 02/002530 (10.01.2002 Gazette 2002/02)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **04.07.2000 JP 2000206865**

(71) Applicant: **Takeda Chemical Industries, Ltd.**
**Osaka-shi, Osaka 541-0045 (JP)**

(72) Inventors:
- **TARUI, Naoki**
  **Nara-shi, Nara 631-0061 (JP)**
- **SANTO, Takashi**
  **Kobe-shi, Hyogo 658-0015 (JP)**
- **WATANABE, Hiroyuki**
  **Kobe-shi, Hyogo 651-2273 (JP)**
- **ASO, Kazuyoshi**
  **Takatsuki-shi, Osaka 569-1044 (JP)**
- **ISHIHARA, Yuji**
  **Itami-shi, Hyogo 664-0874 (JP)**

(74) Representative: **Rickard, Timothy Mark Adrian**
**Takeda Patent Office,**
**11-12 Charles II Street**
**London SW1Y 4QU (GB)**

# (54) GPR14 ANTAGONIST

(57) A novel GPR14 antagonist. The GPR14 antagonist comprises a compound represented by the formula (I) or a salt thereof wherein Ar represents optionally substituted aryl; X represents a spacer; n is an integer of 1 to 10; R represents an optionally substituted hydrocarbon group, etc., provided that R may be bonded to Ar, etc. to form a ring; and Y represents optionally substituted amino, etc.

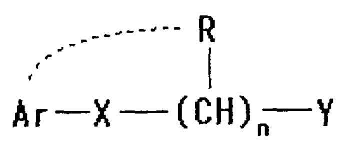

(I)

EP 1 310 490 A1

## Description

Technical field

The present invention relates to a novel GPR14 antagonistic agent and a novel benzazepine derivative having GPR14 antagonistic activity or a salt thereof.

Background Art

Urotensin II was found as one of peptide hormones having strong vasoconstrictive activity, and was revealed to have exceedingly stronger vasoconstrictive activity than endothelin which is the strongest vasopressor substance currently known to mammal artery. Also, a receptor for urotensin II was revealed to be a GPR14 protein which is one of orphan receptors [Nature, vol.401, p.p.282 (1999)].

On the other hand, as a benzazepine derivative, a compound useful as an acetylcholinesterase inhibitor is disclosed, for example, in EP-A-487071 and EP-A-560235, and a compound useful as an anti-obesity agent is disclosed in WO98/46590 and WO00/23437.

Summary of the invention

Although an antagonist of GPR14 which is a receptor for urotensin II is expected to be developed as a new vasoactive drug (e.g. therapeutic drug such as ischemic cardiac infarct and congestive heart failure), there is no report concerning such antagonist.

The present invention provides a vasoactive agent, in particular, a vasoconstriction inhibitor, useful as an prophylactic and therapeutic agent of hypertension, arteriosclerosis, cardiac hypertrophy, cardiac infarction and heart failure based on the GPR14 antagonistic activity; as well as a novel benzazepine derivative having GPR14 antagonistic activity or a salt thereof.

The present inventors intensively studied a compound having GPR antagonistic activity and, as a result, found that a compound represented by the following formula (I) or a salt thereof (hereinafter, referred to as compound (I) in some cases) has excellent GRP14 antagonistic activity and, based on this knowledge, the present invention was completed.

That is, the present invention relates to:

(1) a GPR14 antagonistic agent comprising a compound represented by the formula (I):

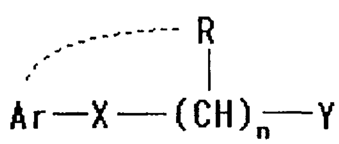 (I)

wherein Ar denotes an optionally substituted aryl group, X denotes a spacer wherein the number of atoms constituting a straight chain moiety is 1 to 4, n denotes an integer of 1 to 10, R is a hydrogen atom or an optionally substituted hydrocarbon group, and may be the same or different in repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

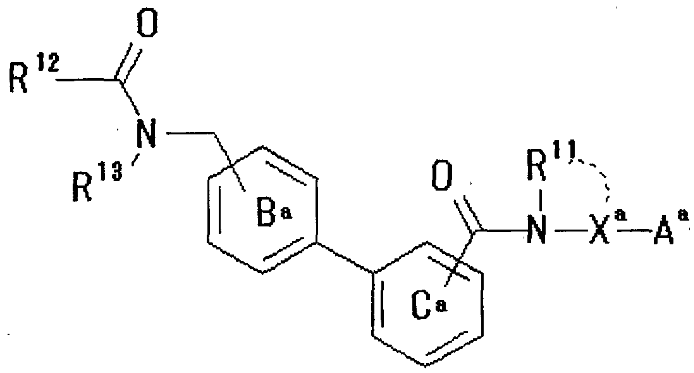

wherein $R^{11}$ denotes a hydrogen atom or an optionally substituted hydrocarbon group, $X^a$ denotes a spacer wherein the number of atoms constituting a straight chain moiety is 1 to 12, $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively;

(2) the agent according to the above-mentioned (1),
wherein Ar is an optionally substituted phenyl group;
(3) the agent according to the above-mentioned (1),
wherein Ar is a group represented by the formula:

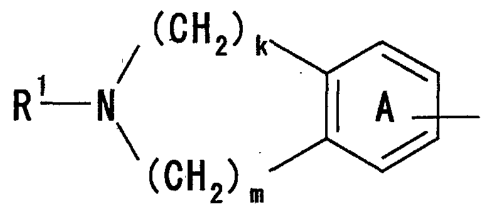

wherein $R^1$ denotes(1) a hydrogen atom,
(2) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$ alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a formyl group, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group having 1 to 6 hetero atoms selected from a nitrogen atom, an oxygen atom and a sulfur atom (this heterocyclic group may be substituted with a substituent group selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group and (xxi') a $C_{1-6}$alkylsulfonyl group (hereinafter, abbreviated as a substituent group P)), (xxvi) an ureido group (this ureido

group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or $C_{1-6}$alkoxy group) or a $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xviii) an amidino group (this amidino group may be mono- or di-substituted with a $C_{1-6}$alkly group or a $C_{6-14}$aryl group (this $C_{6-14}$aryl group may be substituted with a nitro group), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocabonyl and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsufonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphophoryl group, (xxxxii) a $C_{1-4}$alkylenedioxy, (xxxxiii) phenylthio (this phenylthio may be substituted with halogen) and (xxxxiv) phenoxy (this phenoxy may be substituted with halogen), or

(3) an acyl group selected from -(C=O)-$R^{2c}$, -$SO_2$-$R^{2c}$, -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$ alkyl group may be substituted with phenyl), (vii') a $C_{1-6}$alkoxy group (this $C_{1-6}$alkoxy group may be substituted with phenyl, (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may have substituent(s) selected from the substituent group P above), (xxv') phenylthio (this phenylthio may be substituted with halogen) or (xxvi') phenoxy (this phenoxy may be substituted with halogen) (hereainafter, abbreviated as substituent group A), or (iii) a monocyclic or 2 to 4 cyclic heterocyclic group containing 1 to 6 hetero atoms selected from a nitrogen atom, an oxygen atom and sulfur atom (this heterocyclic group may have 1 to 5 substituents selected from the substituent group A above), or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from the substituent group A above)],

ring A denotes a benzene ring optionally having substituent(s) selected from (i) an amino group, (ii) a mono-$C_{1-6}$alkylamino group, (iii) a di-$C_{1-6}$alkylamino group, (iv) a 5 to 7 membered cyclic amino group optionally having 1 to 3 hetero atoms selected from a nitrogen atom, an oxygen atom and a sulfur atom in addition to one nitrogen atom, (v) a $C_{1-6}$alkyl-carbonylamino group, (vi) an aminocarbonyloxy group, (vii) a mono-$C_{1-6}$alkylamino-carbonyloxy group, (viii) a di-$C_{1-6}$alkylamino-carbonyloxy group, (ix) a $C_{1-6}$alkylsulfonylamino group, (x) phenyl-$C_{1-6}$alkylamino, (xi) a phenyl-$C_{1-6}$alkyl-sulfonylamino group, (xii) a phenylsulfonylamino group, (xiii) a halogen atom, (xiv) an optionally halogenated $C_{1-6}$alkyl group, and (xv) an optionally halogenated $C_{1-6}$alkoxy group,

k and m denote independently an integer of 0 to 5, and $1 < k+m < 5$;

(4) the agent according to the above-mentioned (1),

wherein Ar is a group represented by the formula:

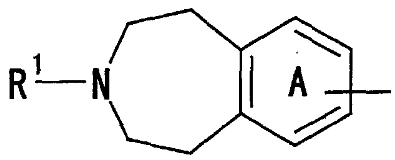

wherein $R^1$ denotes (1) a hydrogen atom,

(2) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group having 1 to 6 hetero atoms selected from a nitrogen atom, an oxygen atom and a sulfur atom(this heterocyclic group may be substituted with substituent (s) selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as a substituent group Q), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group, or a $C_{1-6}$alkoxy group) or a $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono-or di-substituted with a $C_{1-6}$alkyl group or a $C_{6-14}$aryl group (this $C_{6-14}$aryl group may be substituted with a nitro group)), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino) carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or a nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen), or

(3) an acyl group selected from -(C=O)-$R^{2c}$, -$SO_2$-$R^{2c}$, -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally

having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx' ) a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may have substituent(s) selected from the substituent group Q above), (xxv') phenylthio (this phenylthio may be substituted with halogen) or (xxvi') phenoxy (this phenoxy may be substituted with halogen), (hereinafter, abbreviated as substituent group B ), or (iii) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substitutent group B above), or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from the substituent group B above)], ring A denotes a benzene ring optionally having substituent(s) selected from (i) an amino group, (ii) a mono-$C_{1-6}$alkylamino group, (iii) a di-$C_{1-6}$alkylamino group, (iv) a 5 to 7 membered cyclic amino group optionally having 1 to 3 hetero atoms selected from a nitrogen atom, an oxygen atom and a sulfur atom in addition to one nitrogen atom, (v) a $C_{1-6}$alkyl-carbonylamino group, (vi) an aminocarbonyloxy group, (vii) a mono-$C_{1-6}$alkylamino-carbonyloxy group, (viii) a di-$C_{1-6}$alkylamino-carbonyloxy group, (ix) a $C_{1-6}$alkylsulfonylamino group, (x) phenyl-$C_{1-6}$alkylamino, (xi) a phenyl-$C_{1-6}$alkyl-sulfonylamino group, (xii) a phenylsulfonylamino group, (xiii) a halogen atom, (xiv) an optionally halogenated $C_{1-6}$alkyl group and (xv) optionally halogenated $C_{1-6}$alkoxy group;

(5) the agent according to the above-mentioned (1),

wherein X is a group represented by -CO-, -O-, $-NR^{3a}-$,$-NR^{3a}CO-$, -S-, -SO-, $-SO_2-$, $-SO_2NR^{3a}-$, $-SO_2NHCONR^{3a}-$, $-SO_2NHC(=NH)NR^{3a}-$, -CS-, $-CR^{3a}(R^{3b})-$, $-C(=CR^{3a}(R^{3b}))-$, $C(=NR^{3a})$-or $-CONR^{3a}-$ (wherein $R^{3a}$ and $R^{3b}$ denote independently a hydrogen atom, a cyano group, a hydroxyl group, an amino group, a $C_{1-6}$alkyl group or a $C_{1-6}$alkoxy group) ;

(6) the agent according to the above-mentioned (5),

wherein X is a group represented by -CO-, -O-, $-SO_2-$, $- SO_2NR^{3a}-$, $-CR^{3a}(R^{3b})-$ or $-CONR^{3a}-$ (wherein $R^{3a}$ and $R^{3b}$ denote independently a hydrogen atom, a cyano group, a hydroxyl group, an amino group, a $C_{1-6}$alkyl group or a $C_{1-6}$alkoxy group);

(7) the agent according to the above-mentioned (5),

wherein X is a group represented by the formula $-CONR^{3a}-$(wherein $R^{3a}$ denotes a hydrogen atom, a cyano group, a hydroxyl group, an amino group, a $C_{1-6}$ alkyl group or a $C_{1-6}$alkoxy group);

(8) the agent according to the above-mentioned (1),

wherein R is a hydrogen atom;

(9) the agent according to the above-mentioned (1),

wherein Y is a group represented by the formula:

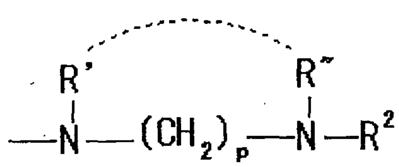

wherein $R^2$ denotes (1) a hydrogen atom,

(2) an acyl group selected from $-(C=O)-R^{2c}$, $-SO_2-R^{2c}$, $-SO-R^{2c}$, $-(C=O)NR^{3c}R^{2c}$, $-(C=O)O-R^{2c}$, $-(C=S)O-R^{2c}$ or $-(C=S) NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group,

(xix') a mono-$C_{1-6}$alkyl-carbamoyl group, (xx' ) a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) selected from (i") a halogen atom, (ii") a nitro group, (iii") a cyano group, (iv") an oxo group, (v") hydroxyl group, (vi") a $C_{1-6}$alkyl group, (vii") a $C_{1-6}$alkoxy group, (viii") a $C_{1-6}$alkylthio group, (ix") an amino group, (x") a mono-$C_{1-6}$alkylamino group, (xi") a di-$C_{1-6}$alkylamino group, (xii") a 5 to 7 membered cyclic amino group optionally having 1 to 3 hetero atoms selected from a nitrogen atom, an oxygen atom and a sulfur atom in addition to carbon atom and one nitrogen atom, (xiii") a $C_{1-6}$alkyl-carbonylamino group, (xiv") a $C_{1-6}$alkyl-carbonylamino group, (xv") a $C_{1-6}$alkoxy-carbonyl group, (xvi") a carboxyl group, (xvii") a $C_{1-6}$alkyl-carbonyl group, (xviii") a carbamoyl group, (xix") a mono-$C_{1-6}$alkylcarbamoyl group, (xx") a di-$C_{1-6}$alkylcarbamoyl group and (xxi") a $C_{1-6}$alkylsulfonyl group (hereinafter, abbreviated as substituent group R)), (xxv') phenylthio (this phenylthio may be substituted with halogen) or (xxvi') phenoxy (this phenoxy may be substituted with halogen) (hereinafter, abbreviated as substituent group C), or (iii) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group C above), or $R^{2c}$ and $R^{3c}$ may be bound to each other to form an optionally substituted 5 to 9 membered nitrogen-containing saturated heterocyclic group together with an adjacent atom (this nitrogen-containing saturated heterocyclic group may have 1 to 5 substituents selected from the substituent group C above)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group R above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4-(4-nitrophenyl)piperazino] carbonyl, (4-benzylpiperazino)carbonyl, morpholinocarbonyl and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group D), or

(4) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group D above), p denotes an integer of 1 to 3,

R' and R" denote a hydrogen atom or a $C_{1-6}$alkyl group (this $C_{1-6}$alkyl group may have 1 to 5 substituents selected from the aforementioned substituent group D), or R' and R" may be bound to each other to form a 5 to 9 membered nitrogen-containing heterocyclic ring optionally containing one hetero atom selected from a nitrogen atom, an

oxygen atom and a sulfur atom in addition to carbon atom and two nitrogen atoms;
(10) the agent according to the above-mentioned (1),
wherein, Y is a group represented by the formula:

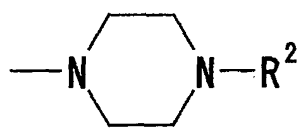

wherein $R^2$ denotes (1) a hydrogen atom, (2) an acyl group selected from -(C=O)-$R^{2c}$, -$SO_2$-$R^{2c}$, -SO-$R^{2c}$, -(C=O)$NR^{3c}R^{2c}$, - (C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl or (iii) a monocyclic or 2 to 4 cyclic heterocyclic group containing 1 to 6 hetero atoms selected from a nitrogen atom, an oxygen atom or a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom (this heterocyclic group may be substituted with substituent (s) selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as substituent group S), (xxv) phenylthio (this phenylthio may be substituted with halogen) or (xxvi) phenoxy (this phenoxy may be substituted with halogen)]
(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group S above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or a $C_{7-16}$aralkyl group), (xxvii) a thioureido group (this thioureido group may be

substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group E), or

(4) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group E above);

(11) the agent according to the above-mentioned (1),
wherein Y is a group represented by the formula:

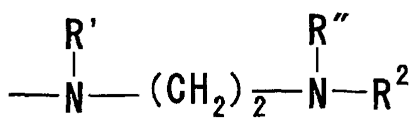

wherein $R^2$ denotes:

(1) a hydrogen atom,

(2) an acyl group selected from -(C=O)-$R^{2c}$, -$SO_2$-$R^{2c}$, -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom) (this heterocyclic group may be substituted with substituent(s) selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from an nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as a substituent group

T), (xxv) phenylthio (this phenylthio may be substituted with halogen and (xxvi) phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group T above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group, or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4-(4-nitrophenyl)piperazino] carbonyl, (4-benzylpiperazino)carbonyl, morpholinocarbonyl and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group F), or

(4) a monocyclic or 2 to 4 cyclic heterocyclic group containing 1 to 6 hetero atoms selected from a nitrogen atom, an oxygen atom and a sulfur atom (this heterocyclic group may have 1 to 5 substituents selected from the substituent group F above), R' and R'' denote a hydrogen atom or a $C_{1-6}$alkyl group respectively (this $C_{1-6}$alkyl group may have 1 to 5 substituents selected from the substituent group F above);

(12) the agent according to the above-mentioned (1), wherein Y is a piperidino group (this piperidino group may be substituted with:

(1) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may be substituted with substituent (s) selected from (i') a halogen atom, (ii') a nitro group, (iii') a cyano group, (iv') an oxo group, (v') a hydroxy group,

(vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as substituent group U), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) an aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group G),

(2) an acyl group selected from -(C=O)-$R^{2c}$, -$SO_2$-$R^{2c}$, -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom) (this heterocyclic group may be substituted with substituent(s) selected from the substituent group U above), (xxv) phenylthio (this phenylthio may be substituted with halogen) and (xxvi) phenoxy (this phenoxy may be substituted with halogen) ], or

(3) a monocyclic or a 2 to 4 cyclic heterocyclic group containing 1 to 6 hetero atoms selected from a nitrogen atom, an oxygen atom and a sulfur atom (this heterocyclic group may have 1 to 5 substituents selected from the substituent group G above);

(13) the agent according to the above-mentioned (1), wherein n is an integer of 1 to 5;

(14) the agent according to the above-mentioned (1), which is a vasoconstriction inhibitor;

(15) the agent according to the above-mentioned (1), which is a prophylactic and/or therapeutic agent of hypertension, arteriosclerosis, cardiac hypertrophy, cardiac infarction or heart failure;

(16) a compound represented by the formula (II):

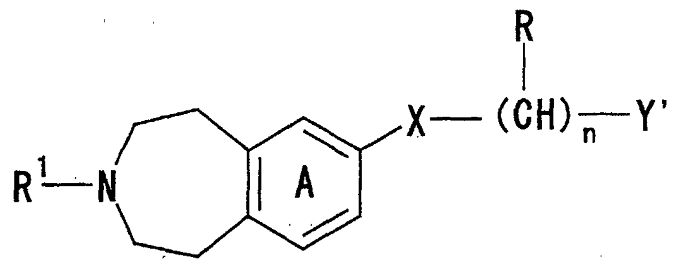

(II)

wherein $R^1$ denotes a hydrogen atom, an optionally substituted hydrocarbon group or an optionally substituted acyl group, ring A denotes a benzene ring optionally further having a substituent, X denotes a spacer wherein the number of atoms constituting a straight chain moiety is 1 to 4 (provided that -CO- is excluded), n denotes an integer of 1 to 10, R is a hydrogen atom or an optionally substituted hydrocarbon group and may be the same or different in the repetition of n, or R may be bound to ring A or a substituent of ring A to form a ring, and Y' denotes an optionally substituted amino group, or a salt thereof ;

(17) a prodrug of the compound or a salt thereof according to the above-mentioned (16);

(18) the compound according to the above-mentioned (16), wherein $R^1$ is a hydrogen atom or an optionally substituted hydrocarbon group;

(19) the compound according to the above-mentioned (16), wherein $R^1$ is a hydrogen atom;

(20) the compound according to the above-mentioned (16), wherein X is a group represented by the formula : -O-, $-NR^{3a}-$, $-NR^{3a}CO-$, -S-, -SO-, $-SO_2-$, $-SO_2NR^{3a}-$, $-SO_2NHCONR^{3a}-$, - $SO_2NHC(=NH)NR^{3a}-$, -CS-, $-CR^{3a}(R^{3b})-$, -C $(=CR^{3a}(R^{3b}))-$, -C $(=NR^{3a})$-or $-CONR^{3a}-$ (wherein $R^{3a}$ and $R^{3b}$ denote independently a hydrogen atom, a cyano group, a hydroxy group, an amino group, a $C_{1-6}$alkyl group or a $C_{1-6}$alkoxy group respectively);

(21) the compound according to the above-mentioned (20), wherein X is a group represented by the formula : - $SO_2NR^{3a}-$, $-CONR^{3a}-$ or $-CR^{3a}(R^{3b})-$ (wherein $R^{3a}$ and $R^{3b}$ denote independently a hydrogen atom, a cyano group, a hydroxy group, an amino group, a $C_{1-6}$alkyl group or a $C_{1-6}$alkoxy group respectively);

(22) the compound according to the above-mentioned (20), wherein X is a group represented by the formula : $-CONR^{3a}-$ (wherein $R^{3a}$ denotes a hydrogen atom, a cyano group, a hydroxy group, an amino group, a $C_{1-6}$alkyl group or a $C_{1-6}$alkoxy group);

(23) the compound according to the above-mentioned (16), wherein R is a hydrogen atom;

(24) the compound according to the above-mentioned (16), wherein Y' is a group represented by the formula:

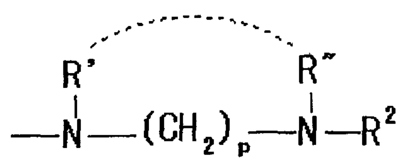

wherein $R^2$ denotes (1) a hydrogen atom,

(2) an acyl group selected from $-(C=O)-R^{2c}$, $-SO_2-R^{2c}$, $-SO-R^{2c}$, - $(C=O)NR^{3c}R^{2c}$, $-(C=O)O-R^{2c}$, $-(C=S)O-R^{2c}$ or - $(C=S)NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or a 2 to 4 cyclic heterocyclic group containing 1 to 6 hetero atoms selected from a nitrogen atom, an oxygen atom and a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i') a halogen atom, (ii') a nitro group, (iii') a cyano group, (iv') an oxo group, (v') a hydroxy group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) selected from (i'') a halogen atom, (ii'') a nitro group, (iii'') a cyano group, (iv'') an oxo group, (v'') hydroxy group, (vi'') a $C_{1-6}$alkyl group, (vii'') a $C_{1-6}$alkoxy group, (viii'') a $C_{1-6}$alkylthio group, (ix'') an amino group, (x'') a mono-$C_{1-6}$alkylamino group, (xi'') a di-$C_{1-6}$alkylamino group, (xii'') a 5 to 7 membered cyclic amino group optionally having 1 to 3 hetero atoms selected from a nitrogen atom, an oxygen atom and a sulfur atom in addition to carbon atom and one nitrogen atom, (xiii'') a $C_{1-6}$alkyl-carbonylamino group, (xiv'') a $C_{1-6}$alkyl-carbonylamino group, (xv'') a $C_{1-6}$alkoxy-carbonyl group, (xvi'') a carboxyl group, (xvii'') a $C_{1-6}$alkyl-carbonyl group, (xviii'') a carbamoyl group, (xix'') a mono-$C_{1-6}$alkylcarbamoyl group, (xx'') a di-$C_{1-6}$alkylcarbamoyl group and (xxi'') a $C_{1-6}$alkylsulfonyl group (hereinafter, abbreviated as substituent group V)), (xxv') phenylthio (this phenylthio may be substituted with halogen) or (xxvi') phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group V above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4- (4-nitrophenyl)piperazino] carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group H), or

(4) a monocyclic or 2 to 4 cyclic heterocyclic group containing 1 to 6 hetero atoms selected from a nitrogen atom, an oxygen atom and a sulfur atom (this heterocyclic group may have 1 to 5 substituents selected from the substituent group H above), p denotes an integer of 1 to 3, R' and R'' each denote a hydrogen atom or a $C_{1-6}$alkyl group (this $C_{1-6}$alkyl group may have 1 to 5 substituents selected from the aforementioned substituent group H), or R' and R'' may be bound to form a 5 to 9 membered nitrogen-containing heterocyclic ring optionally containing one hetero atom selected from a nitrogen atom, an oxygen atom and a sulfur atom in addition to carbon atom and two nitrogen atoms;

(25) the compound according to the above-mentioned (16), wherein, Y' is a group represented by the formula:

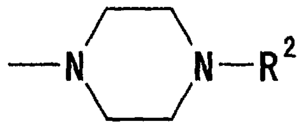

wherein $R^2$ denotes (1) a hydrogen atom,

(2) an acyl group selected from -(C=O)-$R^{2c}$, -$SO_2$-$R^{2c'}$ -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl , a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl or (iii) a monocyclic or 2 to 4 cyclic heterocyclic group containing 1 to 6 hetero atoms selected from a nitrogen atom, an oxygen atom and a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom (this heterocyclic group may be substituted with substituent(s) selected from (i') a halogen atom, (ii') a nitro group, (iii') a cyano group, (iv') an oxo group, (v') a hydroxy group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group), (xxv) phenylthio (this phenylthio may be substituted with halogen) or (xxvi) phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$Cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituent groups selected from (i) a halogen atom, (ii) a nitro group, (iii) a cyano group, (iv) a hydroxy group, (v) a $C_{1-6}$alkyl group and (vi) a $C_{1-6}$alkoxy group,or

(4) a monocyclic or 2 to 4 cyclic heterocyclic group containing 1 to 6 hetero atoms selected from a nitrogen atom, an oxygen atom or a sulfur atom];

(26) the compound according to the above-mentioned (25), wherein $R^2$ is a $C_{7-16}$aralkyl group optionally substituted with a halogen atom;

(27) the compound according to the above-mentioned (25), wherein $R^2$ is benzyl optionally substituted with a halogen atom, or diphenylmethyl optionally substituted with a halogen atom;

(28) the compound according to the above-mentioned (16), wherein Y' is a group represented by the formula:

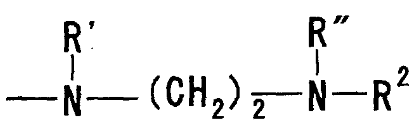

wherein $R^2$ denotes:

(1) a hydrogen atom,

(2) an acyl group selected from -(C=O)-$R^{2c}$, -$SO_2$-$R^{2c}$, -SO-$R^{2c}$, - (C=O)N$R^{3c}R^{2c}$, -(C=O)O-$R^{2c}$, -(C=S)O-$R^{2c}$ or

- (C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i') a halogen atom, (ii') a nitro group, (iii') a cyano group, (iv') an oxo group, (v') a hydroxy group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkylsulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) selected from (i") a halogen atom, (ii") a nitro group, (iii") a cyano group, (iv") an oxo group, (v") a hydroxy group, (vi") a $C_{1-6}$alkyl group, (vii") a $C_{1-6}$alkoxy group, (viii") a $C_{1-6}$alkylthio group, (ix") an amino group, (x") a mono-$C_{1-6}$alkylamino group, (xi") a di-$C_{1-6}$alkylamino group, (xii") a 5 to 7 membered cyclic amino group optionally having 1 to 3 hetero atoms selected from a nitrogen atom, an oxygen atom and a sulfur atom in addition to carbon atom and one nitrogen atom, (xiii") a $C_{1-6}$alkyl-carbonylamino group, (xiv") a $C_{1-6}$alkyl-carbonylamino group, (xv") a $C_{1-6}$alkoxy-carbonyl group, (xvi") a carboxyl group, (xvii") a $C_{1-6}$alkyl-carbonyl group, (xviii") a carbamoyl group, (xix") a mono-$C_{1-6}$alkylcarbamoyl group, (xx") a di-$C_{1-6}$alkylcarbamoyl group and (xxi") a $C_{1-6}$alkylsulfonyl group), (xxv') phenylthio (this phenylthio may be substituted with halogen) or (xxvi') phenoxy (this phenoxy may be substituted with halogen)];,

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) a hydroxyl group, (v) a $C_{1-6}$alkyl group and (vi) a $C_{1-6}$alkoxy group, or

(4) a monocyclic or 2 to 4 cyclic heterocyclic group containing 1 to 6 hetero atoms selected from a nitrogen atom, an oxygen atom or a sulfur atom,

R' and R" each denote a hydrogen atom or a $C_{1-6}$alkyl group;

(29) the compound according to the above-mentioned (16), wherein Y' is a piperidino group (this piperidino group may be substituted with (i) phenyl-$C_{1-6}$alkyl optionally substituted with $C_{1-6}$alkyl, $C_{1-6}$alkoxy, halogen atom, nitro, mono- or di-$C_{1-6}$alkyl-carbamoyloxy, hydroxyl, cyano, carboxyl, $C_{1-6}$alkoxycarbonyl, carbamoyl, cyclic aminocarbonyl, amino, $C_{1-6}$alkylcarbonylamino, phenylsulfonylamino, $C_{1-6}$alkylsulfonylamino, amidino, ureido or heterocycle, (ii) $C_{1-6}$alkyl group optionally substituted with halogen atom, hydroxyl, $C_{1-6}$alkoxy, amino, mono- or di-$C_{1-6}$alkylamino, carboxyl, cyano or $C_{1-6}$alkoxy-carbonyl, or (iii) $C_{1-6}$alkylcarbonyl group optionally substituted with mono or di-$C_{1-6}$alkylamino or $C_{1-6}$alkoxy-carbonyl;

(30) the compound according to the above-mentioned (16), wherein n is an integer of 1 to 5;

(31) N-[2-(4-benzhydrylpiperazin-1-yl)ethyl]-2,3,4,5-tetrahydro-1H-3-benzazepine-7-carboxamide or a salt thereof;

(32) N-[2-[4-(4-chlorobenzyl)piperazin-1-yl]ethyl]-2,3,4,5-tetrahydro-lH-3-benzazepine-7-carboxamide or a salt thereof;

(33) N-[2-{4-[bis(4-fluorophenyl)methyl]-1-piperazinyl}ethyl]-2,3,4,5-tetrahydro-lH-3-benzazepine-7-carboxamide or a salt thereof;

(34) a pharmaceutical composition comprising the compound according to the above-mentioned (16) or a salt thereof or a prodrug thereof;

(35) a GPR14 antagonistic agent comprising the compound according to the above-mentioned (16) or a salt thereof or a prodrug thereof;

(36) the composition according to the above-mentioned (34), which is a vasoconstriction inhibitor;

(37) the composition according to the above-mentioned (34), which is a prophylactic and/or therapeutic agent of hypertension, arteriosclerosis, cardiac hypertrophy, cardiac infarction or heart failure;

(38) a GPR14 antagonizing method, which comprises: administering to a mammal an effective dose of a compound represented by the formula (I):

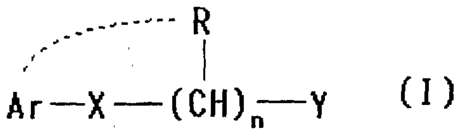

wherein Ar denotes an optionally substituted aryl group, X denotes a spacer wherein the number of atoms constituting a straight chain moiety is 1 to 4, n denotes an integer of 1 to 10, R denotes a hydrogen atom or an optionally substituted hydrocarbon group and may be the same or different in repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

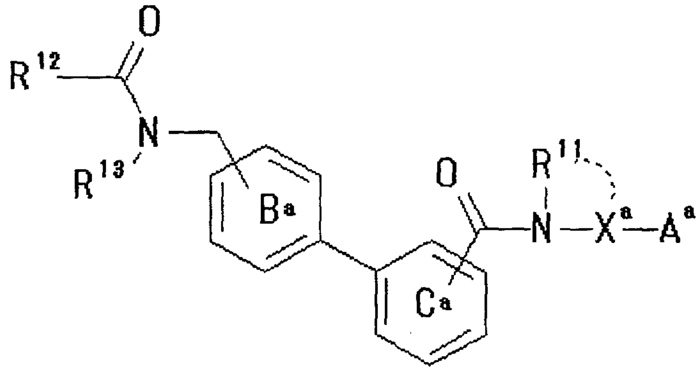

wherein $R^{11}$ denotes a hydrogen atom or an optionally substituted hydrocarbon group, $X^a$ denotes a spacer wherein the number of atoms constituting a straight chain moiety is 1 to 12, $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively;

(39) use of a compound represented by the formula (I):

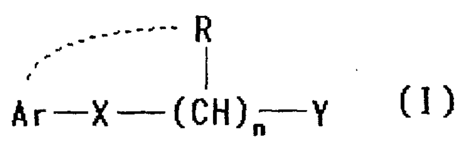

wherein Ar denotes an optionally substituted aryl group, X denotes a spacer wherein the number of atoms constituting a straight chain moiety is 1 to 4, n denotes an integer of 1 to 10, R denotes a hydrogen atom or an optionally substituted hydrocarbon group and may be the same or different in repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

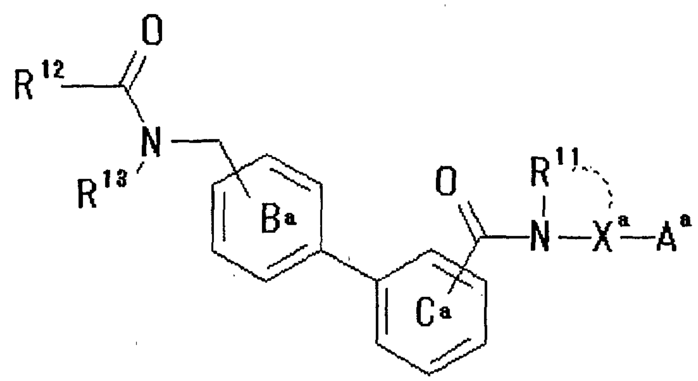

wherein $R^{11}$ denotes a hydrogen atom or an optionally substituted hydrocarbon group, $X^a$ denotes a spacer wherein the number of atoms constituting a straight chain moiety is 1 to 12, $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively, for the manufacture of a GPR14 antagonistic agent;
(40) a process for manufacturing a compound represented by the formula:

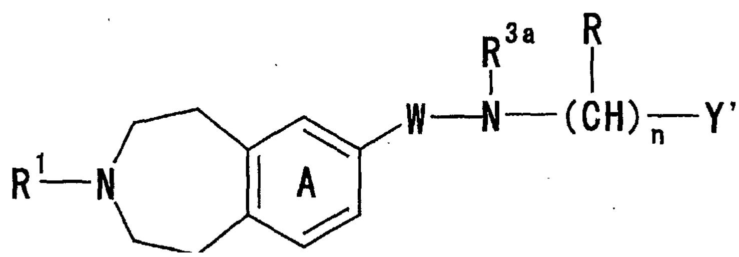

wherein $R^1$ denotes the same meaning as that described in the above-mentioned (1), W denotes $-SO_2-$ or -CO-, $R^{3a}$ denotes a hydrogen atom, a cyano group, a hydroxyl group, an amino group, a $C_{1-6}$alkyl group or a $C_{1-6}$alkoxy group, R denotes a hydrogen atom or an optionally substituted hydrocarbon group, Y' denotes an optionally substituted amino group, and n denotes an integer of 1 to 10, or a salt thereof, which comprises: reacting a compound represented by the formula:

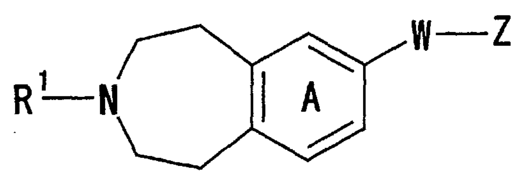

wherein Z denotes a leaving group and other symbols denote the same meanings as those described above, or a salt thereof with a compound represented by the formula:

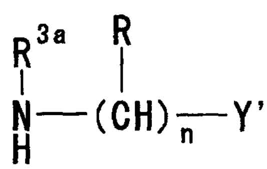

wherein respective symbols denote the same meanings as those described above, or a salt thereof; and the like.

Mode for Carrying Out the Invention

The GPR14 antagonistic activity in the present invention means the activity of competitively or non-competitively inhibiting binding of a ligand (urotensin II etc.) to a GPR14 protein on a cell membrane.

In the present invention, based on such GPR14 antagonistic activity, drugs expressing a variety of vasoactive activities (e.g. enhancement or inhibition of vasoconstriction) are provided. Inter alia, vasoconstriction inhibitor exhibiting the activity of alleviating the strong vasoconstriction activity induced by urotensin II are preferably used. Such vasoconstriction inhibitors can be applied as a prophylactic and therapeutic agent of various diseases and, inter alia, they are preferably used as a prophylactic and therapeutic agent of hypertension, arteriosclerosis, cardiac hypertrophy, cardiac infarction or heart failure, in particular, as a prophylactic and therapeutic agent of ischemic cardiac infarction and congestive heart failure.

In the formula above, Ar denotes an "optionally substituted aryl group".

Examples of the "substituent group" of the "optionally substituted aryl group" include (i) an optionally halogenated lower alkyl group, (ii) a halogen atom (e.g. fluoro, chloro, bromo, iodo etc.), (iii) a lower alkylenedioxy group (e.g. a $C_{1-3}$alkylenedioxy group such as methylenedioxy, ethylenedioxy etc.), (iv) a nitro group, (v) a cyano group, (vi) a hydroxyl group, (vii) an optionally halogenated lower alkoxy group, (viii) a lower cycloalkyl group (e.g. a $C_{3-6}$Cycloalkyl group such as cyclopropyl, cyclobutyl, cyclopentyl, cyclohexyl etc.), (ix) an optionally halogenated lower alkylthio group, (x) an amino group, (xi) a mono-lower alkylamino group (e.g. a mono-$C_{1-6}$alkylamino group such as methylamino, ethylamino, propylamino etc.), (xii) a di-lower alkylamino group (e.g. a di-$C_{1-6}$alkylamino group such as dimethylamino, diethylamino etc.), (xiii) a 5 to 7 membered cyclic amino group optionally having 1 to 3 hetero atoms selected from a nitrogen atom, an oxygen atom and a sulfur atom in addition to one nitrogen atom (e.g., pyrrolidino, piperidino, piperazino, morpholino, thiomorpholino etc.), (xiv) a lower alkyl-carbonylamino group (e.g. a $C_{1-6}$alkyl-carbonylamino group such as acetylamino, propionylamino, butyrylamino etc.), (xv) an aminocarbonyloxy group, (xvi) a mono-lower alkylamino-carbonyloxy group (e.g. a mono-$C_{1-6}$alkylamino-carbonyloxy group such as methylaminocarbonyloxy, ethylaminocarbonyloxy etc.), (xvii) a di-lower alkylamino-carbonyloxy group (e.g. a di-$C_{1-6}$alkylamino-carbonyloxy group such as dimethylaminocarbonyloxy, diethylaminocarbonyloxy etc.), (xviii) a lower alkylsulfonylamino group (e.g. a $C_{1-6}$alkylsulfonylamino group such as methylsulfonylamino, ethylsulfonylamino, propylsulfonylamino etc.), (xix) a lower alkoxy-carbonyl group (e.g. a $C_{1-6}$alkoxy-carbonyl group such as methoxycarbonyl, ethoxycarbonyl, propoxycarbonyl, isobutoxycarbonyl etc.), (xx) a carboxyl group, '(xxi) a lower alkyl-carbonyl group (e.g. a $C_{1-6}$alkyl-carbonyl group such as methylcarbonyl, ethylcarbonyl, butylcarbonyl etc.), (xxii) a lower cycloalkyl-carbonyl (e.g. a $C_{3-6}$Cycloalkyl-carbonyl group such as cyclopropylcarbonyl, cyclobutylcarbonyl, cyclopentylcarbonyl, cyclohexylcarbonyl etc.), (xxiii) a carbamoyl group, (xxiv) a mono-lower alkyl-carbamoyl group (e.g. a mono-$C_{1-6}$alkyl-carbamoyl group such as methylcarbamoyl, ethylcarbamoyl, propylcarbamoyl, butylcarbamoyl etc.), (xxv) a di-lower alkyl-carbamoyl group (e.g. a di-$C_{1-6}$alkyl-carbamoyl group such as diethylcarbamoyl, dibuthylcarbamoyl etc.), (xxvi) a lower alkylsulfonyl group (e. g. a $C_{1-6}$alkylsulfonyl group such as methylsulfonyl, ethylsulfonyl, propylsulfonyl etc.), (xxvii) a lower cycloalkylsulfonyl (e.g. a $C_{3-6}$cycloalkylsulfonyl such as cyclopentylsulfonyl, cyclohexylsulfonyl etc.), (xxviii) a phenyl group, (xxix) a naphthyl group, (xxx) a mono-phenyl-lower alkyl group (e.g. a mono-phenyl-$C_{1-6}$alkyl group such as benzyl, phenylethyl etc.), (xxxi) a di-phenyl-lower alkyl group (e.g. a diphenyl-$C_{1-6}$alkyl group such as diphenylmethyl, diphenylethyl etc.), (xxxii) a mono-phenyl-lower alkyl-carbonyloxy group (e.g. a mono-phenyl-$C_{1-6}$alkyl-carbonyloxy group such as phenylmethylcarbonyloxy, phenylethylcarbonyloxy etc.), (xxxiii) a di-phenyl-lower alkyl-carbonyloxy group (e.g. a di-phenyl-$C_{1-6}$alkyl-carbonyloxy group such as diphenylmethylcarbonyloxy, diphenylethylcarbonyloxy etc.), (xxxiv) a phenoxy group, (xxxv) a mono-phenyl-lower alkyl-carbonyl group (e.g. a mono-phenyl-$C_{1-6}$alkyl-carbonyl group such as phenylmethylcarbonyl, phenylethylcarbonyl etc.), (xxxvi) a di-phenyl-lower alkyl-carbonyl group (e.g. a diphenyl-$C_{1-6}$alkyl-carbonyl group such as diphenylmethylcarbonyl, diphenylethylcarbonyl etc.), (xxxvii) a benzoyl group, (xxxviii) a phenoxycarbonyl group, (xxxix) a phenyl-lower alkyl-carbamoyl group (e.g. a phenyl-$C_{1-6}$alkyl-carbamoyl group such as phenyl-methylcarbamoyl, phenyl-ethylcarbamoyl etc.), (xxxx) a phenylcarbamoyl group, (xxxxi) a phenyl-lower alkyl-carbonylamino group (e.g. a phenyl-$C_{1-6}$alkyl-carbonylamino such as phenyl-methylcarbonylamino, phenyl-ethylcarbonylamino etc.), (xxxxii) a phenyl-lower alkylamino (e.g. a phenyl-$C_{1-6}$alkylamino such as phenyl-methylamino, phenyl-ethylamino etc.), (xxxxiii) a phenyl-lower alkylsulfonyl group (e.g. a phenyl-$C_{1-6}$alkylsulfonyl group such as phenyl-methylsulfonyl, phenyl-ethylsulfonyl etc.), (xxxxiv) a phenylsulfonyl group, (xxxxv) a phenyl-lower alkylsulfinyl group (e.g. a phenyl-$C_{1-6}$alkylsulfinyl group such as phenyl-methylsulfinyl, phenyl-ethylsulfinyl etc.), (xxxxvi) a phenyl-lower alkylsulfonylamino group (e.g. a phenyl-$C_{1-6}$alkylsulfonylamino group such as phenyl-methylsulfonylamino, phenyl-ethylsulfonylamino etc.) and (xxxxvii) phenylsulfonylamino group [the (xxviii) phenyl group, the (xxix) naphthyl group, the (xxx) mono-phenyl-lower alkyl group, the (xxxi) di-phenyl-lower alkyl group, the (xxxii) mono-phenyl-lower alkyl-carbonyloxy group, the (xxxiii) di-phenyl-lower alkyl-carbonyloxy group, the (xxxiv) phenoxy group, the (xxxv) mono-phenyl-lower alkyl-carbonyl group, the (xxxvi) di-phenyl-lower alkyl-carbonyl group, the (xxxvii) benzoyl group, the (xxxviii) phenoxycarbonyl group, the (xxxix) phenyl-lower alkyl-carbamoyl group, the (xxxx) phenylcarbamoyl group, the (xxxxi) phenyl-lower alkyl-carbonylamino group, the (xxxxii) phenyl-lower alkylamino, the (xxxxiii) phenyl-lower

alkylsulfonyl group, the (xxxxiv) phenylsulfonyl group, the (xxxxv) phenyl-lower alkylsulfinyl group, the (xxxxvi) phenyl-lower alkylsulfonylamino group and the (xxxxvii) phenylsulfonylamino group may have further 1 to 4 substituents selected from, for example, a lower alkyl (e.g. a $C_{1-6}$alkyl such as methyl, ethyl, propyl, isopropyl, butyl, sec-butyl, tert-butyl, pentyl, hexyl etc.), lower alkoxy (e.g. $C_{1-6}$alkoxy such as methoxy, ethoxy, propoxy, isopropoxy, n-butoxy, isobutoxy, sec-butoxy, tert-butoxy etc.), a halogen atom (e.g. chloro, bromo, iodo etc.), hydroxyl, benzyloxy, amino, mono-lower alkylamino (e.g. a mono-$C_{1-6}$alkylamino such as methylamino, ethylamino, propylamino etc.), di-lower alkylamino (e.g. di-$C_{1-6}$alkylamino such as dimethylamino, diethylamino etc.), nitro, lower alkyl-carbonyl (e.g. $C_{1-6}$alkyl-carbonyl such as methylcarbonyl, ethylcarbonyl, butylcarbonyl etc.), and benzoyl].

Examples of the aforementioned "optionally halogenated lower alkyl group" include lower alkyl groups (e.g. a $C_{1-6}$alkyl group such as methyl, ethyl, propyl, isopropyl, butyl, sec-butyl, tert-butyl, pentyl, hexyl etc.) optionally having 1 to 3 halogen atoms(e.g. chloro, bromo, iodo etc.), more particularly, methyl, chloromethyl, difluoromethyl, trichloromethyl, trifluoromethyl, ethyl, 2-bromoethyl, 2,2,2-trifluoroethyl, propyl, 3,3,3-trifluoropropyi, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl and the like.

Examples of the aforementioned "optionally halogenated lower alkoxy group" include lower alkoxy groups (e.g. a $C_{1-6}$alkoxy group such as methoxy, ethoxy, propoxy, isopropoxy, n-butoxy, isobutoxy, sec-butoxy, tert-butoxy etc.) optionally having 1 to 3 halogen atoms (e.g. chloro, bromo, iodo etc.), more particularly, methoxy, difluoromethoxy, trifluoromethoxy, ethoxy, 2,2,2-trifluoroethoxy, n-propoxy, isopropoxy, n-butoxy, 4,4,4-trifluorobutoxy, isobutoxy, sec-butoxy, pentyloxy, hexyloxy and the like.

Examples of the aforementioned "optionally halogenated lower-alkylthio group" include lower alkylthio groups (e.g. a $C_{1-6}$alkylthio group such as methylthio, ethylthio, n-propylthio, isopropylthio, n-butylthio, isobutylthio, secbutylthio, tert-butylthio etc.) optionally having 1 to 3 halogen atoms(e.g. chloro, bromo, iodo etc.), more particularly, methylthio, difluoromethylthio, trifluoromethylthio, ethylthio, n-propylthio, isopropylthio, n-butylthio, 4,4,4-trifluorobutylthio, isobutylthio, secbutylthio, tert-butylthio, pentylthio, hexylthio and the like.

Examples of the "substituent group" of the "optionally substituted aryl group" include preferably (i) an amino group, (ii) a mono-lower alkylamino group (e.g. a mono-$C_{1-}$ 6alkylamino group such as methylamino, ethylamino, propylamino etc.), (iii) a di-lower alkylamino group (e.g. a di-$C_{1-6}$alkylamino group such as dimethylamino, diethylamino etc.), (iv) a 5 to 7 membered cyclic amino-group optionally having 1 to 3 hetero atoms selected from a nitrogen atom, an oxygen atom and a sulfur atom in addition to one nitrogen atom (e.g. pyrrolidino, piperidino, piperazino, morpholino, thiomorpholino etc.), (v) a lower alkyl-carbonylamino group (e.g. a $C_{1-6}$alkyl-carbonylamino group such as acetylamino, propionylamino, butyrylamino etc.), (vi) an aminocarbonyloxy group, (vii) a mono-lower alkylamino-carbonyloxy group (e.g. a mono-$C_{1-6}$alkylamino-carbonyloxy group such as methylaminocarbonyloxy, ethylaminocarbonyloxy etc.), (viii) a di-lower alkylamino-carbonyloxy group (e.g. a di-$C_{1-6}$alkylamino-carbonyloxy group such as dimethylaminocarbonyloxy, diethylaminocarbonyloxy etc.), (ix) a lower alkylsulfonylamino group (e.g. a $C_{1-6}$alkylsulfonylamino group such as methylsulfonylamino, ethylsulfonylamino, propylsulfonylamino etc.), (x) phenyl-lower alkylamino (e.g. phenyl-$C_{1-6}$alkylamino such as phenyl-methylamino, phenyl-ethylamino etc.) , (xi) a phenyl-lower alkylsulfonylamino group (e.g. a phenyl-$C_{1-6}$alkyl-sulfonylamino group such as phenyl-mehtylsulfonylamino, phenyl-ethylsulfonylamino etc.), (xii) a phenylsulfonylamino group, (xiii) a halogen atom (e.g. fluoro, chloro etc.), (xiv) an optionally halogenated lower (e.g. $C_{1-6}$) alkyl group (e.g. methyl, ethyl, isopropyl, tert-butyl, trifluoromethyl etc.) and (xv) an optionally halogenated lower (e.g. $C_{1-6}$)alkoxy group (e.g. methoxy, ethoxy, isopropoxy, tert-butoxy, trifluoromethoxy etc.) and, in particular, a 5 to 7 membered cyclic amino group (e.g. pyrrolidino, piperidino, piperazino, morpholino, thiomorpholino etc.) optionally having 1 to 3 hetero atoms selected from a nitrogen atom, an oxygen atom and a sulfur atom in addition to one nitrogen atom is preferred.

Examples of the "aryl group" in the "optionally substituted aryl group" represented by Ar in the formula above include $C_{6-14}$aryl such as phenyl, naphthyl and the like, preferably $C_{6-10}$aryl, more preferably phenyl. Herein, of the "optionally substituted aryl group", substituent groups in the "aryl group" may be bound to each other to form a fused ring, and examples of the case where an aryl group (preferably a phenyl group) as Ar forms a fused ring include:

(1) the case where an aryl group is fused with an optionally substituted monocyclic heterocyclic ring,
(2) the case where an aryl group is fused with an optionally substituted dicyclic heterocyclic ring, or is fused with two identical or different monocyclic rings (provided that at least one ring is a monocyclic heterocyclic ring), and
(3) the case where an aryl group is fused with an optionally substituted tricyclic heterocyclic ring.

Examples of the case where the "aryl group" in the "optionally substituted aryl group" is fused with an optionally substituted monocyclic heterocyclic ring include a group represented by the formula:

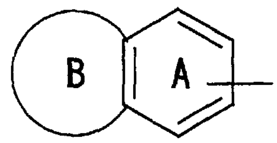

wherein ring B denotes an optionally substituted heterocyclic ring, and ring A denotes an optionally substituted benzene ring.

The substituent groups for ring A are exemplified by those for the aforementioned "optionally substituted aryl group".

As the "heterocyclic ring" of the "optionally substituted heterocyclic ring" represented by ring B, for example, 4 to 14 membered rings, preferably 5 to 9 membered rings are used, and the "heterocyclic ring" may be either aromatic or non-aromatic. As a hetero atom, for example, 1 to 3 or 4 selected from a nitrogen atom, an oxygen atom or a sulfur atom are used. More particularly, pyridine, pyrazine, pyrimidine, imidazole, furan, thiophene, dihydropyridine, azepine, diazepine, oxazepine, pyrrolidine, piperidine, hexamethyleneimine, heptamethyleneimine, tetrahydrofuran, piperazine, homopiperazine, tetrahydrooxazepine, morpholine, thiomorpholine, pyrrole, pyrazole, 1,2,3-triazole, oxazole, oxazolidine, thiazole, thiazolidine, isoxazole and imidazoline are used. In particular, 5 to 9 membered non-aromatic heterocyclic rings containing one hetero atom or same or different two hetero atoms (e.g. pyrrolidine, piperidine, hexamethyleneimine, heptamethyleneimine, tetrahydrofuran, piperazine, homopiperazine, tetrahydrooxazepine, morpholine, thiomorpholine etc.) are preferred. In particular, for example, a non-aromatic heterocyclic ring containing one hetero atom selected from a nitrogen atom, an oxygen atom and a sulfur atom, and a non-aromatic heterocyclic ring containing one nitrogen atom and one hetero atom selected from a nitrogen atom, an oxygen atom and a sulfur atom are frequently used.

The "substituent group" of the "optionally substituted heterocyclic ring" represented by ring B may be substituted on an arbitrary carbon atom of ring B. As the substituent group on an arbitrary carbon atom of ring B, for example, 1 to 5 substituent groups selected from (i) a halogen atom (e.g. fluoro, chloro, bromo, iodo etc.), (ii) a nitro group, (iii) a cyano group, (iv) an oxo group, (v) a hydroxyl group, (vi) a lower alkyl group (e.g. a $C_{1-6}$alkyl group such as methyl, ethyl, propyl, isopropyl, butyl, isobutyl, tert-butyl, sec-butyl etc.), (vii) a lower alkoxy group (e.g. a $C_{1-6}$alkoxy group such as methoxy, ethoxy, n-propyloxy, i-propyloxy, n-butyloxy etc.), (viii) a lower alkylthio group (e.g. a $C_{1-6}$alkylthio group such as methylthio, ethylthio, propylthio etc.), (ix) an amino group, (x) a mono-lower alkylamino group (e.g. a mono-$C_{1-6}$alkylamino group such as methylamino, ethylamino, propylamino etc.), (xi) a di-lower alkylamino group (e. g. a di-$C_{1-6}$alkylamino group such as dimethylamino, diethylamino etc.), (xii) a 5 to 7 membered cyclic amino group optionally having 1 to 3 hetero atoms selected from a nitrogen atom, an oxygen atom and a sulfur atom in addition to carbon atom and one nitrogen atom (e.g. pyrrolidino, piperidino, piperazino, morpholino, thiomorpholino etc.), (xiii) a lower alkyl-carbonylamino group (e.g. a $C_{1-6}$alkyl-carbonylamino group such as acetylamino, propionylamino, butyrylamino etc.), (xiv) a lower alkylsulfonylamino group (a $C_{1-6}$alkyl-carbonylamino group such as methylsulfonylamino, ethylsulfonylamino etc.), (xv) a lower alkoxy-carbonyl group (e.g. a $C_{1-6}$alkoxy-carbonyl group such as methoxycarbonyl, ethoxycarbonyl, propoxycarbonyl etc.), (xvi) a carboxyl group, (xvii) a lower alkyl-carbonyl group (e.g. a $C_{1-6}$alkyl-carbonyl group such as methylcarbonyl, ethylcarbonyl, propylcarbonyl etc.), (xviii) a carbamoyl group, (xix) a mono-lower alkylcarbamoyl group (e.g. a mono-$C_{1-6}$alkylcarbamoyl group such as methylcarbamoyl, ethylcarbamoyl etc.), (xx) a di-lower alkylcarbamoyl group (e.g. a di-$C_{1-6}$alkylcarbamoyl group such as dimethylcarbamoyl, diethylcarbamoyl etc.), (xxi) a lower alkylsulfonyl group (e.g. a $C_{1-6}$alkylsulfonyl group such as methylsulfonyl, ethylsulfonyl, propylsulfonyl etc.) are used.

Inter alia, an oxo group, a lower alkyl group (e.g. a $C_{1-6}$alkyl group such as methyl, ethyl, propyl, isopropyl, butyl, isobutyl, tert-butyl, sec-butyl etc.) and the like are preferred, and an oxo group and the like are used widely.

Further, when ring B has a nitrogen atom in the ring, ring B may have further a substituent on the nitrogen atom. That is, ring B may have in the ring:

$$>N-R^1$$

wherein $R^1$ denotes a hydrogen atom, an optionally substituted hydrocarbon group, an optionally substituted aryl group or an optionally substituted heterocyclic group.

The "hydrocarbon group" of the "optionally substituted hydrocarbon group" represented by the aforementioned $R^1$ denotes a group in which one hydrogen atom is removed from a hydrocarbon compound, and examples thereof include chain or cyclic hydrocarbon groups such as an alkyl group, an alkenyl group, an alkynyl group, a cycloalkyl group, an aryl group, an aralkyl group and the like. Inter alia, a chain or cyclic $C_{1-16}$hydrocarbon group or a combination

thereof and the like are preferably used.

As the chain or cyclic hydrocarbon group,

(1) a straight or branched lower alkyl group (e.g. a $C_{1-6}$alkyl group such as methyl, ethyl, propyl, isopropyl, butyl, isobutyl, tert-butyl, sec-butyl, pentyl, hexyl etc.),
(2) a straight or branched lower alkenyl group (e.g. a $C_{2-6}$alkenyl group such as vinyl, allyl, isopropenyl, butenyl, isobutenyl, sec-butenyl etc.),
(3) a straight or branched lower alkynyl group (e.g. a $C_{2-6}$alkynyl group such as propargyl, ethynyl, butynyl, 1-hexynyl etc.),
(4) a monocyclic lower cycloalkyl group (e.g. a monocyclic $C_{3-6}$cycloalkyl group such as cyclopropyl, cyclobutyl, cyclopentyl, cyclohexyl etc.),
(5) a bridged cyclic lower saturated hydrocarbon group (e.g. a bridged cyclic $C8_{-14}$ saturated hydrocarbon group such as bicyclo[3.2.1.]oct-2-yl, bicyclo[3.3.1]non-2-yl, adamantan-1-yl etc.) or
(6) an aryl group (e.g., a $C_{6-14}$aryl group such as phenyl, 1-naphthyl, 2-naphthyl, biphenyl, 2-indenyl, 2-anthryl etc., preferably, a phenyl group), in addition, as a hydrocarbon group comprising a combination of chain and cyclic groups,
(1) a lower aralkyl group (e.g. a $C_{7-16}$aralkyl group such as phenyl-$C_{1-10}$alkyl group (e.g. benzyl, phenylethyl, phenylpropyl, phenylbutyl, phenylpentyl, phenylhexyl etc.), naphthyl-$C_{1-6}$alkyl (e.g. $\alpha$-naphthylmethyl etc.) or diphenyl-$C_{1-3}$alkyl (e.g. diphenylmethyl, diphenylethyl etc.) etc.),
(2) an aryl-alkenyl group (e.g. a $C_{6-14}$aryl-$C_{2-12}$alkenyl group such as phenyl-$C_{2-12}$alkenyl such as styryl, cinnamyl, 4-phenyl-2-butenyl, 4-phenyl-3-butenyl etc.),
(3) an aryl-$C_{2-12}$alkynyl group (e.g. a $C_{6-14}$aryl-$C_{2-12}$alkynyl group such as phenyl-$C_{2-12}$alkynyl such as phenylethynyl, 3-phenyl-2-propynyl, 3-phenyl-1-propynyl etc.),
(4) a lower cycloalkyl-lower alkyl group (e.g. $C_{3-7}$cycloalkyl-$C_{1-6}$alkyl group such as cyclopropylmethyl, cyclobutylmethyl, cyclopentylmethyl, cyclohexylmethyl, cycloheptylmethyl, cyclopropylethyl, cyclobutylethyl, cyclopentylethyl, cyclohexylethyl, cycloheptylethyl, cyclopropylpropyl, cyclobutylpropyl, cyclopentylpropyl, cyclohexylpropyl, cycloheptylpropyl, cyclopropylbutyl, cyclobutylbutyl, cyclopentylbutyl, cyclohexylbutyl, cycloheptylbutyl, cyclopropylpentyl, cyclobutylpentyl, cyclopentylpentyl, cyclohexylpentyl, cycloheptylpentyl, cyclopropylhexyl, cyclobutylhexyl, cyclopentylhexyl, cyclohexylhexyl etc.),
(5) an aryl-$C_{1-10}$alkyl group (e.g. biphenyl-$C_{1-10}$alkyl such as biphenylmethyl, biphenylethyl etc.); are preferably used.

As a preferable "hydrocarbon group" in the "optionally substituted hydrocarbon group" represented by $R^1$, for example,
(1) a straight, branched or cyclic alkyl group, preferably a straight or branched $C_{1-6}$alkyl group (e.g. a $C_{1-6}$alkyl group such as methyl, ethyl, propyl, isopropyl, butyl, isobutyl, tert-butyl, sec-butyl, pentyl, hexyl etc.), a cyclic $C_{3-8}$alkyl group (e.g. cyclopropyl, cyclobutyl, cyclopentyl, cyclohexyl etc.), a $C_{4-12}$alkyl group comprising a combination of straight, branched and cyclic groups (e.g. cyclopropylmethyl, cyclopentylmethyl, cyclohexylmethyl, cyclohexylethyl, (4-methylcyclohexyl)methyl etc.) or
(2) a $C_{7-16}$aralkyl group (e.g. phenyl-$C_{1-10}$alkyl (e.g. benzyl, phenylethyl, phenylpropyl, phenylbutyl, phenylpentyl, phenylhexyl etc.), naphthyl-$C_{1-6}$alkyl (e.g. $\alpha$-naphthylmethyl etc.) or diphenyl-$C_{1-3}$alkyl (e.g. diphenylmethyl, diphenylethyl etc.) etc.), more preferably a $C_{7-10}$aralkyl group (e.g. phenyl-$C_{1-4}$alkyl such as benzyl, phenylethyl, phenylpropyl etc.) and the like are used.

The "hydrocarbon group" represented by $R^1$ may have a substituent group and, as such substituent groups, those which are generally used as a substituent group for a hydrocarbon group can be appropriately used. Specifically, 1 to 5 (preferably 1 to 3) substituet groups selected from (i) a halogen atom (e.g. fluoro, chloro, bromo, iodo etc.), (ii) a nitro group, (iii) a cyano group, (iv) an oxo group, (v) a hydroxyl group, (vi) a lower alkyl group optionally substituted with halogen or phenyl (e.g. a $C_{1-6}$alkyl group such as methyl, ethyl, propyl, isopropyl, butyl, isobutyl, tert-butyl, sec-butyl etc.), (vii) a lower alkoxy group optionally substituted with halogen or phenyl (e.g. a $C_{1-6}$alkoxy group such as methoxy, ethoxy, n-propyloxy, i-propyloxy, n-butyloxy etc.), (viii) a lower alkylthio group optioanally substituted with halogen or phenyl (e.g. a $C_{1-6}$alkylthio group such as methylthio, ethylthio, propylthio etc.), (ix) an amino group, (x) a mono-lower alkylamino group (e.g. a mono-$C_{1-6}$alkylamino group such as methylamino, ethylamino, propylamino etc.), (xi) a di-lower alkyamino group (e,g. a di-$C_{1-6}$alkylamino group such as dimethylamino, diethylamino etc.), (xii) a 5 to 7 membered cyclic amino group optionally having 1 to 3 hetero atoms selected from a nitrogen atom, an oxygen atom and a sulfur atom in addition to carbon atom and one nitrogen atom (e.g. pyrrolidino, piperidino, piperazino, morpholino, thiomorpholino etc.), (xiii) a lower alkyl-carbonylamino group (e.g. $C_{1-6}$alkyl-carbonylamino group such as acetylamino, propionylamino, butyrylamino etc.), (xiv) a lower alkylsulfonylamino group (e.g. a $C_{1-6}$alkyl-sulfonylamino group such as methylsulfonylamino, ethylsulfonylamino etc.), (xv) a lower alkoxy-carbonyl group (e.g. a $C_{1-6}$alkoxy-carbonyl group

such as methoxycarbonyl, ethoxycarbonyl, propoxycarbonyl etc.), (xvi) a carboxyl group, (xvii) formyl, a lower alkylcarbonyl group (e.g. a $C_{1-6}$alkyl-carbonyl group such as methylcarbonyl, ethylcarbonyl, propylcarbonyl etc.), (xviii) a carbamoyl group, (xix) a mono-lower alkyl-carbamoyl group (e.g. a mono-$C_{1-6}$alkyl-carbamoyl group such as methylcarbamoyl, ethylcarbamoyl etc.), (xx) a di-lower alkyl-carbamoyl group (e.g. a di-$C_{1-6}$alkyl-carbamoyl group such as dimethylcarbamoyl, diethylcarbamoyl etc.), (xxi) a lower alkylsulfonyl group (e.g. a $C_{1-6}$alkylsulfonyl group such as methylsulfonyl, ethylsulfonyl, propylsulfonyl etc.), (xxii) a lower alkoxy-carbonyl-lower alkyl group (e.g. a $C_{1-6}$alkoxy-carbonyl-$C_{1-6}$alkyl group such as methoxycarbonylmethyl, ethoxycarbonylmetyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl etc.), (xxiii) a carboxyl-lower alkyl group (e.g. a carboxyl-$C_{1-6}$alkyl group such as carboxylmethyl, carboxylethyl, carboxyl(dimethyl)methyl etc.), (xxiv) an optionally substituted heterocyclic group, (xxv) an optionally substituted alkyl group, (xxvi) an optionally substituted alkoxy group, (xxvii) an optionally substituted ureido group (e.g ureido, 3-methylureido, 3-ethylureido, 3-phenylureido, 3-(4-fluorophenyl)ureido, 3-(2-methylphenyl)ureido, 3-(4-methoxyphenyl)ureido, 3-(2,4-difluorophenyl)ureido, 3-[3,5-bis(trifluoromethyl)phenyl]ureido, 3-benzylureido, 3-(1-naphthyl)ureido, 3-(2-biphenylyl)ureido etc.), (xxviii) an optionally substituted thioureido group (e.g. thioureido, 3-methylthioureido, 3-ethylthioureido, 3-phenylthioureido, 3-(4-fluorophenyl)thioureido, 3-(4-methylphenyl)thioureido, 3-(4-methoxyphenyl)thioureido, 3- (2,4-dichlorophenyl)thioureido, 3-benzylthioureido, 3-(1-naphthyl)thioureido etc.), (xxix) an optionally substituted amidino group (e.g. amidino, $N^{1-}$methylamidino, $N^1$-ethylamidino, $N^1$-phenylamidino, $N^1$,$N^1$-dimethylamidino, $N^1$,$N^2$-dimethylamidino, $N^1$-methyl-$N^1$-ethylamidino, $N^1$,$N^1$-diethylamidino, $N^1$-methyl-$N^1$-phenylamidino, $N^1$,$N^1$-di(4-nitrophenyl)amidino etc.), (xxx) an optionally substituted guanidino group (e.g. guanidino, 3-methylguanidino, 3,3-dimethylguanidino, 3,3-diethylguanidino etc.), (xxxi) an optionally substituted cyclic aminocarbonyl group (e.g. pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, thiomorpholinocarbonyl etc.), (xxxii) an optionally substituted aminothiocarbonyl group (e.g. aminothiocarbonyl, methylaminothiocarbonyl, dimethylaminothiocarbonyl etc.), (xxxiii) an optionally substituted aminosulfonyl (e.g. aminosulfonyl, methylaminosulfonyl, dimethylaminosulfonyl etc.), (xxxiv) an optionally substituted phenylsulfonylamino (e.g. phenylsulfonylamino, (4-methylphenyl)sulfonylamino, (4-chlorophenyl)sulfonylamino, (2,5-dichlorophenyl)sulfonylamino, (4-methoxyphenyl)sulfonylamino, (4-acetylaminophenyl)sulfonylamino, (4-nitrophenyl)phenylsulfonylamino etc.), (xxxv) a sulfo group, (xxxvi) a sulfino group, (xxxvii) a sulfeno group, (xxxviii) a $C_{1-6}$alkylsulfo group (e.g. methylsulfo, ethylsulfo, propylsulfo etc.), (xxxix) a $C_{1-6}$alkylsulfino group (e.g. methylsulfino, ethylsulfino, propylsulfino etc.), (xxxx) a $C_{1-6}$alkylsulfeno group (e.g. methylsulfeno, ethylsulfeno, propylsulfeno etc.), (xxxxi) a phosphono group, (xxxxii) a di-$C_{1-6}$alkoxyphosphoryl group (e.g. dimethoxyphosphoryl, diethoxyphosphoryl, dipropoxyphosphoryl etc.), (xxxxiii) $C_{1-4}$alkylenedioxy (e.g. $-O-CH_2-O-$, $-O-CH_2-CH_2-O-$ etc.), (xxxxiv) phenylthio optionally substituted with halogen, and (xxxxv) phenoxy optionally substituted with halogen are used.

As the "substituent group" in the "optionally substituted hydrocarbon group" represented by $R^1$, preferably, a halogen atom, an optionally substituted alkyl group , an optionally substituted alkoxy group, a hydroxyl group, a nitro group, a cyano group, a carboxyl group, a $C_{1-6}$alkoxycarbonyl group, a carbamoyl group, an aminothiocarbonyl group, a mono-lower alkyl-carbamoyl group, a di-lower alkyl-carbamoyl group, an optionally substituted cyclic aminocarbonyl group, an amino group, a mono-lower alkylamino group, a di-lower alkylamino group, a 5 to 7 membered cyclic amino group optionally having 1 to 3 hetero atoms selected from a nitrogen atom, an oxygen atom and a sulfur atom in addition to carbon atom and one nitrogen atom, a $C_{1-6}$alkylcarbonylamino group, an optionally substituted phenylsulfonylamino group, a $C_{1-6}$alkylsulfonylamino group, an optionally substituted amidino group, an optionally substituted ureido group, and an optionally substituted heterocyclic group are used.

As the "heterocyclic group" in the "optionally substituted heterocyclic group", groups obtained by removing one hydrogen atom from a monocyclic heterocylic ring and polycyclic heterocyclic rings such as di-, tri-and tetracyclic heterocyclic rings are used. The heterocyclic rings may be either aromatic or non-aromatic. As a heteroatom, for example, 1 to 6 hetero atoms selected from a nitrogen atom, an oxygen atom and a sulfur atom are used. Specifically, as a monocyclic heterocyclic group, groups obtained by removing one hydrogen atom from the "heterocyclic ring" in the "optionally substituted heterocyclic ring" represented by the aforementioned ring B are used. In addition, groups obtained by removing one hydrogen atom from a monocyclic heterocyclic ring such as triazole, thiadiazole, oxadiazole, oxathiadiazole, triazine and tetrazole are also used. As a dicyclic heterocyclic group, for example, groups obtained by removing one hydrogen atom from a dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine and imidazopyridine are used. As a polycyclic heterocyclic group such as tricyclic and tetracyclic heterocyclic groups, groups obtained by removing one hydrogen atom from a polycyclic heterocyclic ring such as acridine, tetrahydroacridine, pyrroloquinoline, pyrroloindole, cyclopentoindole and

isoindolobenzazepine are used.

As the "heterocyclic group" in the "optionally substituted heterocyclic group", in particular, groups obtained by removing one hydrogen atom from the aforementioned monocyclic heterocyclic ring or dicyclic heterocyclic ring are frequently used.

In addition, as the "substituent group" in the "optionally substituted heterocyclic group", the "substituent groups (provided that the "optionally substituted heterocyclic group" is excluded)" in the "optionally substituted heterocyclic ring " represented by the aforementioned ring B are used.

As the "substituent group" in the "optionally substituted alkyl (preferably an optionally substituted $C_{1-6}$alkyl)" or the "optionally substituted alkoxy (preferably an optionally substituted $C_{1-6}$alkoxy)", for example, "substituent groups" represented by (i) to (xxiv) or (xxvii) to (xxxxii) exemplified as the "substituent group" in the "optionally substituted hydrocarbon group" represented by the aforementioned $R^1$ are used.

The "substituent group" in the "optionally substituted uerido group", the "optionally substituted thioureido group", the "optionally substituted amidino group", the "optionally substituted guanidino group", the "optionally substituted cyclic aminocarbonyl group", the "optionally substituted aminothiocarbonyl group", the "optionally substituted aminosulfonyl" or the "optionally substituted phenylsulfonylamino", for example, the "substituent groups" shown in (i) to (xxvi) or (xxxv) to (xxxxii) exemplified as the "substituent group" in the "optionally substituted hydrocarbon group" represented by the aforementioned $R^1$, a $C_{6-14}$aryl group (this $C_{6-14}$aryl group may have a substituent group selected from halogen, a $C_{1-6}$alkyl group, a halo$C_{1-6}$alkyl group, a $C_{1-6}$alkoxy group and a nitro group) and a $C_{7-16}$aralkyl group are used.

The "optionally substituted hydrocarbon group" represented by $R^1$ include preferably (i) a $C_{1-6}$alkyl group and (ii) a phenyl-$C_{1-6}$alkyl group optionally substituted with a halogen atom, a nitro, $C_{1-6}$alkyl, or $C_{1-6}$alkoxy, more preferably, a benzyl group optionally substituted with $C_{1-4}$alkyl(methyl etc.), trihalogeno$C_{1-4}$alkyl (methyl etc.), halogen atom (fluoro, chloro etc.), nitro, cyano, $C_{1-4}$alkoxy (methoxy etc.), trihalogeno$C_{1-4}$alkoxy (methoxy etc.), hydroxyl, carbamoyl, (4-$C_{1-4}$alkyl (methyl etc.)-1-piperazinyl)carbonyl, aminothiocarbonyl, morpholinocarbonyl, carboxyl, $C_{1-4}$alkoxy (methoxy etc.) carbonyl, $C_{1-4}$alkoxy(ethoxy etc.) carbonyl$C_{1-4}$alkoxy(methoxy etc.), carboxyl$C_{1-4}$alkoxy (methoxy etc.), $C_{1-4}$alkoxy(ethoxy etc.)carbonyl$C_{1-6}$alkyl(isopropyl etc.), carboxyl$C_{1-6}$alkyl (isopropyl etc.), amino, acetylamino, $C_{1-4}$alkyl(methyl etc.)sulfonylamino, (4-$C_{1-4}$alkyl(methyl etc.)phenyl)sulfonylamino, ureido, 3-$C_{1-4}$alkyl(methyl etc.) ureido, amidino, dihydrothiazolyl or dihydroimidazolyl.

Inter alia, $R^1$ is preferably a benzyl group optionally substituted with $C_{1-4}$alkyl (methyl etc.), trihalogeno(fluoro etc.)$C_{1-4}$alkyl (methyl etc.), halogen atom (fluoro, chloro etc.), nitro, cyano, carbamoyl, $C_{1-4}$alkoxy(methoxy etc.) carbonyl, $C_{1-4}$alkoxy (ethoxy etc.) carbonyl$C_{1-4}$alkoxy (methoxy etc.), amino, acetylamino, $C_{1-4}$alkyl(methyl etc.) sulfonylamino, 3-$C_{1-4}$alkyl(methyl etc.) ureido, amidino, or dihydroimidazolyl, and in particular, a benzyl group optionally substituted with $C_{1-4}$alkyl, more particularly, a benzyl group optionally substituted with methyl is preferred.

Examples of the "optionally substituted acyl group" represented by the aforementioend $R^1$ include -(C=O) -$R^{2c}$,-$SO_2$-$R^{2c}$, -SO-$R^{2c}$, -(C=O)$NR^{3c}R^{2c}$, -(C=O)O-$R^{2c}$, -(C=S)O-$R^{2c}$ or-(C=S)$NR^{3c}R^{2c}$ [$R^{2c}$ and $R^{3c}$ are the same or different and denote (i) a hydrogen atom, (ii) an optionally substituted hydrocarbon group or (iii) an optionally substituted heterocyclic group or $R^{2c}$ and $R^{3c}$ may be bound to each other to form a nitrogen-containing saturated heterocyclic group optionally having a substituent group together with an adjacent nitrogen atom].

Among them, preferable are -(C=O)-$R^{2c}$, -$SO_2$-$R^{2c}$, -SO-$R^{2c}$, - (C=O)$NR^{3c}R^{2c}$ and -(C=O)O-$R^{2c}$ ($R^{2c}$ and $R^{3c}$ have the same meanings as those described above) and, inter alia, -(C=O)-$R^{2c}$ and -(C=O)$NR^{3c}R^{2c}$ ($R^{2c}$ and $R^{3c}$ have the same meanings as those described above) are used widely.

The "hydrocarbon group" in the "optionally substituted hydrocarbon group" represented by $R^{2c}$ and $R^{3c}$ denotes a group in which one hydrogen atom is removed from a hydrocarbon compound, and examples thereof include chain or cyclic hydrocarbon groups such as an alkyl group, an alkenyl group, an alkynyl group, a cycloalkyl group, an aryl group, and an aralkyl group. Specifically, the "hydrocarbon group" are exemplified by those for the "optionally substituted hydrocarbon group" represented by $R^1$ described above and, inter alia, chain or cyclic $C_{1-16}$hydrocarbon groups are preferred, in particular, a lower ($C_{1-6}$)alkyl group, a lower ($C_{2-6}$)alkenyl group, a $C_{7-16}$aralkyl group and a $C_{6-14}$aryl group are preferred. Inter alia, a lower ($C_{1-6}$)alkyl group, a $C_{7-16}$aralkyl group and a $C_{6-14}$aryl group are used widely.

As the "heterocyclic group" in the "optionally substituted heterocyclic group" represented by $R^{2c}$ and $R^{3c}$, groups obtained by removing one hydrogen atom from a monocyclic heterocyclic ring and polycyclic heterocyclic ring such as di, tri-or tetracyclic heterocyclic ring are used. The heterocyclic rings may be either aromatic or non-aromatic. As a hetero atom, for example, 1 to 6 hetero atoms selected from a nitrogen atom, an oxygen atom and a sulfur atom are used. Specifically, as a monocyclic heterocyclic group, groups obtained by removing one hydrogen atom from the "heterocyclic ring" in the "optionally substituted heterocyclic ring" represented by the aformentioend ring B are used. In addition, for example, groups obtained by removing one hydrogen atom from a monocylic heterocyclic ring such as triazole, thiadiazole, oxadiazole, oxathiadiazole, triazine and tetrazole are also used. As a dicyclic heterocyclic group, for example, groups obtained by removing one hydrogen atom from a dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-1H-1-benzazepine, tetrahydro-lH-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine and imidazopyridine are used. As a popycyclic heterocyclic group such as tri- or tetracyclic heterocyclic groups, groups obtained by removing one hydrogen atom from a polycyclic heterocyclic ring such as acridine, tetrahydroacridine, pyrroloquinoline, pyrroloindole, cyclopentoindole and isoindolobenzazepine are used.

As the "heterocyclic group" in the "optionally substituted heterocyclic group", in particular, groups obtained by removing one hydrogen atom from the aforementioned monocyclic heterocyclic ring or dicyclic heterocyclic ring are frequently used.

As the "optionally substituted nitrogen-containing saturated heterocylic group" which may be formed by $R^{2c}$ and $R^{3c}$ together with an adjacent nitrogen atom, a 5 to 9 membered nitrogen-containing saturated heterocyclic group optionally containing 1 to 3 hetero atoms such as a nitrogen atom, an oxygen atom and a sulfur atom in addition to carbon atom and one nitrogen atom is used. As the nitrogen-containing saturated heterocyclic group, groups having a bond on a ring-constituting nitrogen atom are preferred. As a group having a bond on a ring-constituting nitrogen atom, for example, a group represented by the formula:

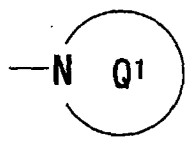

wherein ring $Q^1$ denotes a 5 to 9 membered nitrogen-containing saturated heterocyclic group optionally containing 1 to 2 hetero atoms selected from a nitrogen atom, an oxygen atom and a sulfur atom in addition to carbon atom and one nitrogen atom, is used. More specifically, for example,

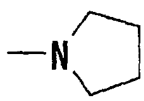 , 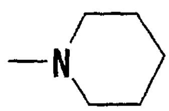 , 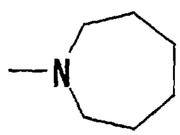 , 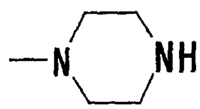

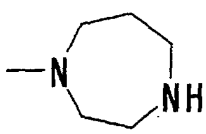 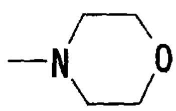 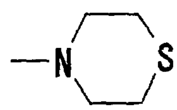

or is frequently used.

As a preferable substituent group which may be possessed by the "hydrocarbon group" or the "heterocyclic group" represented by $R^{2c}$ and $R^{3c}$, or the "nitrogen-containing saturated heterocyclic group" represented by $NR^{3c}R^{2c}$, for example, 1 to 5 (preferably 1 to 3) substituent groups selected from (i) a halogen atom (e.g. fluoro, chloro, bromo, iodo etc.), (ii) a nitro group, (iii) a cyano group, (iv) an oxo group, (v) a hydroxyl group, (vi) an optionally substituted hydrocarbon group, (vii) a lower alkoxy group optionally substituted with a phenyl group (e.g. a $C_{1-6}$alkoxy group such as methoxy, ethoxy, n-propyloxy, i-propyloxy, n-butyloxy etc.), (viii) a lower alkylthio group optionally substituted with a phenyl group (a $C_{1-6}$alkylthio group such as methylthio, ethylthio, propylthio etc.), (ix) an amino group, (x) a mono-lower alkylamino group (e.g. a mono-$C_{1-6}$alkylamino group such as methylamino, ethylamino, propylamino etc.), (xi) a di-lower alkylamino group (e.g. a di-$C_{1-6}$alkylamino group such as dimethylamino, a diethylamino etc.), (xii) a 5 to 7 membered cyclic amino group optionally having 1 to 3 hetero atoms selected from a nitrogen atom, an oxygen atom and a sulfur atom in addition to carbon atom and one nitrogen atom (e.g. pyrrolidino, piperidino, piperazino, morpholino, thiomorpholino etc.), (xiii) a lower alkyl-carbonylamino group (e.g. a $C_{1-6}$alkyl-carbonylamino group such as acetylamino, propionylamino, butyrylamino etc.), (xiv) a lower alkyl-sulfonylamino group (e.g. a $C_{1-6}$alkyl-sulfonylamino group such as methylsulfonylamino, ethylsulfonylamiono etc.), (xv) a lower alkoxy-carbonyl group (e.g. a $C_{1-6}$alkoxy-carbonyl group such as methoxycarbonyl, ethoxycarbonyl, propoxycarbonyl etc.), (xvi) a carboxyl group, (xvii) a lower alkyl-carbonyl group (e.g. a $C_{1-6}$alkyl-carbonyl group such as methylcarbonyl, ethylcarbonyl, propylcarbonyl etc.), (xviii) a carbamoyl group, (xix) a mono-lower alkyl-carbamoyl group (e.g. a mono-$C_{1-6}$alkyl-carbamoyl group such as methylcarbamoyl, ethylcarbamoyl etc.), (xx) a di-lower alkyl-carbamoyl group (e.g. a di-$C_{1-6}$alkyl-carbamoyl group such as dimethylcarbamoyl, diethylcarbamoyl etc.), (xxi) a lower alkylsulfonyl group (e.g. a $C_{1-6}$alkylsulfonyl group such as methylsulfonyl, ethylsulfonyl, propylsulfonyl etc.), (xxii) a lower alkoxy-carbonyl-lower alkyl group (e.g. a $C_{1-6}$alkoxy-carbonyl-$C_{1-6}$alkyl group such as methoxycarbonylmethyl, ethoxycarbonylmethyl, tert-butoxycarbonylmethyl, methoxycarbonylethyl, methoxycarbonylmethyl, methoxycarbonyl(dimethyl)methyl, ethoxycarbonyl(dimethyl)methyl, tert-butoxycarbonyl(dimethyl)methyl etc.), (xxiii) a carboxyl-lower alkyl group (e.g. a carboxyl-$C_{1-6}$alkyl group such as carboxylmethyl, carboxylethyl, carboxyl(dimethyl)methyl etc.), (xxiv) an optionally substituted heterocyclic group, (xxv) phenylthio optionally substituted with halogen, and (xxvi) phenoxy optionally substituted with halogen are used.

The "lower alkoxy group" and the "lower alkylthio group" may further have a phenyl group as a substituent group.

As the "substituent group" and the "hydrocarbon group" in the "optionally substituted hydrocarbon group", the "substituent group" and the "hydrocarbon group" in the "optionally substituted hydrocarbon group" represented by the aforementioned $R^1$ are used.

As the "heterocyclic group" in the "optionally substituted heterocyclic group", a group obtained by removing one hydrogen atom from the "heterocyclic ring" in the "optionally substituted heterocyclic ring" represented by the aforementioned ring B is used.

In addition, as the "substituent group" in the "optionally substituted heterocyclic group", the "substituent group (provided that the "optionally substituted heterocyclic group" is excluded)" in the "optionally substituted heterocyclic ring" represented by the aforementioned ring B is used.

Preferable examples of $R^{2c}$ and $R^{3c}$ include phenyl optionally substituted with $C_{1-4}$alkyl (methyl, ethyl etc.) or $C_{1-4}$alkoxy (methoxy, ethoxy etc.), $C_{1-4}$alkyl (methyl, ethyl etc.), halogeno(fluoro, chloro etc.)$C_{1-4}$alkyl (methyl, ethyl etc.), benzyl, naphthyl, pyridyl, thienyl, furyl and a hydrogen atom.

Preferable examples of the "optionally substituted acyl group" represented by the aforementioned $R^1$ include formyl, acetyl, trihalogeno(fluoro etc.)acetyl, pyridylcarbonyl, thienylcarbonyl, furylcarbonyl, phenacyl, benzoyl, $C_{1-4}$alkyl (methyl etc.)benzoyl, $C_{1-4}$alkoxy (methoxy etc.)benzoyl, benzenesulfonyl, naphthylsulfonyl and thienylsulfonyl, more preferably, -(C=O)-$R^{2c}$ [wherein $R^{2c}$ denotes a $C_{1-6}$alkyl group, a phenyl group optionally substituted with a $C_{1-6}$alkoxy group, or a phenyl-$C_{1-6}$alkyl group].

As the "heterocyclic group" in the "optionally substituted heterocyclic group" represented by $R^1$, groups obtained by removing one hydrogen atom from a monocyclic heterocyclic ring or polycyclic heterocyclic ring such as tricyclic or tetracyclic heterocyclic ring are used. The heterocyclic ring may be aromatic or non-aromatic. As a hetero atom, for example, 1 to 6 hetero atoms selected from a nitrogen atom, an oxygen atom and a sulfur atom are used. Specifically, as the monocyclic heterocyclic group, groups obtained by removing one hydrogen atom from the "heterocyclic ring" in the "optionally substituted heterocyclic ring" represented by the aforementioned ring B are used. Further, besides them, for example, groups obtained by removing one hydrogen atom from a monocyclic heterocyclic ring such as triazole, thiadiazole, oxadiazole, oxathiadiazole, triazine and tetrazole are used. As the dicyclic heterocyclic group, for example, groups obtained by removing one hydrogen atom from a dicyclic heterocyclic ring such as indole, dihydroindole, isoindole, dihydroisoindole, benzofuran, dihydrobenzofuran, benzimidazole, benzoxazole, benzisoxazole, benzothiazole, indazole, quinoline, tetrahydroquinoline, isoquinoline, tetrahydroisoquinoline, tetrahydro-lH-1-benzazepine, tetrahydro-1H-2-benzazepine, tetrahydro-1H-3-benzazepine, tetrahydrobenzoxazepine, quinazoline, tetrahydroquinazoline, quinoxaline, tetrahydroquinoxaline, benzodioxane, benzodioxole, benzothiazine and imidazopyridine are used. As polycyclic heterocyclic groups such as tricyclic or tetracyclic heterocyclic group, groups obtained by removing one hydrogen atom from polycyclic heterocyclic rings such as acridine, tetrahydroacridine, pyrroloquinoline, pyrroloindole, cyclopentoindole and isoindolobenzazepine are used.

As the "heterocyclic group" in the "optionally substituted heterocyclic group", in particular, groups obtained by removing one hydrogen atom from the monocyclic heterocyclic ring or the dicyclic heterocyclic ring are frequently used, and, inter alia, a pyridyl group is preferred.

In addition, as the "substituent group" in the "optionally substituted heterocyclic group", the "substituent group (provided that "optionally substituted heterocyclic group" is excluded)" in the "optionally substituted heterocyclic ring" represented by the aforementioned ring B and the "substituent group" in the "optionally substituted hydrocarbon group" represented by the aforementioned $R^1$ are used.

Preferable examples of $R^1$ include (i) a hydrogen atom, (ii) a $C_{1-6}$alkyl group, (iii) a phenyl-$C_{1-6}$alkyl group optionally substituted with a halogen atom, nitro, $C_{1-6}$alkyl or $C_{1-6}$alkoxy or (iv) (C=O)-$R^{2c}$ [wherein $R^{2c}$ denotes a $C_{1-6}$alkyl group, a phenyl group optionally substituted with a $C_{1-6}$alkoxy group, or a phenyl-$C_{1-6}$alkyl group].

More specific examples of the case where the "aryl group" in the "optionally substituted aryl group" is fused with a monocyclic heterocyclic ring optionally having a substituent group include, as a phenyl group fused with a monocyclic heterocyclic ring represented by the formula:

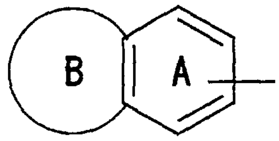

, for example, groups obtained by removing one hydrogen atom from a dicyclic fused benzene ring such as 2,3-dihydrobenzofuran; 3,4-dihydro-2H-1-benzothiopyran; 2,3-dihydro-1H-indole; 1,2,3,4-tetrahydroquinoline; 2,3-dihydro-1H-isoindole; 1,2,3,4-tetrahydroisoquinoline; benzazepine such as 2,3,4,5-tetrahydro-lH-1-benzazepine, 2,3,4,5-tetrahydro-lH-2-benzazepine, 2,3,4,5-tetrahydro-lH-3-benzazepine and the like; benzazocine such as 1,2,3,4,5,6-hexahydro-1-benzazocine, 1,2,3,4,5,6-hexahydro-2-benzazocine, 1,2,3,4,5,6-hexahydro-3-benzazocine and the like; benzazonine such as 2,3,4,5,6,7-hexahydro-1H-1-benzazonine, 2,3,4,5,6,7-hexahydro-lH-2-benzazonine, 2,3,4,5,6,7-hexahydro-1H-3-benzazonine, 2,3,4,5,6,7-hexahydro-lH-4-benzazonine and the like; benzoxazole such as 2,3-dihydrobenzoxazole and the like; benzothiazole such as 2,3-dihydrobenzothiazole; benzimidazole such as 2,3-dihydro-1H-benzimidazole and the like; benzoxazine such as 3,4,dihydro-1H-2,1-benzoxazine, 3,4-dihydro-lH-2,3-benzoxazine, 3,4-dihydro-2H-1,2-benzoxazine, 3,4-dihydro-2H-1,4-benzoxazine, 3,4-dihydro-2H-l,3-benzoxazine, 3,4-dihydro-2H-3,1-benzoxazine and the like; benzothiazine such as 3,4-dihydro-1H-2,1-benzothiazine, 3,4-dihydro-1H-2,3-benzothiazine, 3,4-dihydro-2H-1,2-benzothiazine, 3,4-dihydro-2H-1,4-benzothiazine, 3,4-dihydro-2H-1,3-benzothiazine, 3,4-dihydro-2H-3,1-benzothiazine and the like; benzodiazine such as 1,2,3,4-tetrahydrocinnoline, 1,2,3,4-tetrahydrophthalazine, 1,2,3,4-tetrahydroquinazoline, 1,2,3,4-tetrahydroquinoxaline and the like; benzoxathiin such as 3,4-dihydro-1,2-benzoxathiin, 3,4-dihydro-2,1-benzoxathiin, 2,3-dihydro-1,4-benzoxathiin, 1,4-dihydro-2,3-benzoxathiin, 4H-1,3-benzoxathiin, 4H-3,1-benzoxathiin and the like; benzodioxin such as 3,4-dihydro-1,2-benzodioxin, 2,3-dihydro-1,4-benzodioxin, 1,4-dihydro-2,3-benzodioxin, 4H-1,3-benzodioxin and the like; benzodithiin such as 3,4-dihydro-1,2-benzodithiin, 2,3-dihydro-1,4-benzodithiin, 1,4-dihydro-2,3-benzodithiin, 4H-1,3-benzodithiin and the like; benzoxazepine such as 2,3,4,5-tetrahydro-1,2-benzoxazepine, 2,3,4,5-tetrahydro-1,3-benzoxazepine, 2,3,4,5-tetrahydro-1,4-benzoxazepine, 2,3,4,5-tetrahydro-1,5-benzoxazepine, 1,3,4,5-tetrahydro-2,1-benzoxazepine, 1,3,4,5-tetrahydro-2,3-benzoxazepine, 1,3,4,5-tetrahydro-2,4-benzoxazepine, 1,2,4,5-tetrahydro-3,1-benzoxazepine, 1,2,4,5-tetrahydro-3,2-benzoxazepine, 1,2,3,5-tetrahydro-4,1-benzoxazepine and the like; benzothiazepine such as 2,3,4,5-tetrahydro-1,2-benzothiazepine, 2,3,4,5-tetrahydro-1,4-benzothiazepine, 2,3,4,5-tetrahydro-1,5-benzothiazepine, 1,3,4,5-tetrahydro-2,1-benzothiazepine, 1,3,4,5-tetrahydro-2,4-benzothiazepine, 1,2,4,5-tetrahydro-3,1-benzothiazepine, 1,2,4,5-tetrahydro-3,2-benzothiazepine, 1,2,3,5-tetrahydro-4,1-benzothiazepine and the like; benzodiazepine such as ,2,3,4,5-tetrahydro-1H-1,2-benzodiazepine, 2,3,4,5-tetrahydro-lH-1,3-benzodiazepine, 2,3,4,5-tetrahydro-lH-1,4-benzodiazepine, 2,3,4,5-tetrahydro-lH-1,5-benzodiazepine, 2,3,4,5-tetrahydro-lH-2,3-benzodiazepine, 2,3,4,5-tetrahydro-lH-2,4-benzodiazepine and the like; benzodioxepin such as 4,5-dihydro-1,3-benzodioxepin, 4,5-dihydro-3H-1,2-benzodioxepin, 2,3-dihydro-5H-1,4-benzodioxepin, 3,4-dihydro-2H-1,5-benzodioxepin, 4,5-dihydro-lH-2,3-benzodioxepin, 1,5-dihydro-2,4-benzodioxepin and the like; benzodithiepin such as 4,5-dihydro-lH-2,3-benzothiepin, 1,5-dihydro-2,4-benzodithiepin, 3,4-dihydro-2H-1,5-benzodithiepin, 2,3-dihydro-5H-1,4-benzodithiepin and the like; benzoxazocine such as 3,4,5,6-tetrahydro-2H-1,5-benzoxazocine, 3,4,5,6-tetrahydro-2H-1,6-benzoxazocine and the like; benzothiazocine such as 3,4,5,6-tetrahydro-2H-1,5-benzothiazocine, 3,4,5,6-tetrahydro-2H-1,6-benzothiazocine and the like; benzodiazocine such as 1,2,3,4,5,6-hexahydro-1,6-benzodiazocine and the like; benzoxathiocine such as 2,3,4,5-tetrahydro-1,6-benzoxathiocine and the like; benzodioxocine such as 2,3,4,5-tetrahydro-1,6-benzodioxocine and the like; benzotrioxepin such as 1,3,5-benzotrioxepin, 5H-1,3,4-benzotrioxepin and the like; benzoxathiazepine such as 3,4-dihydro-lH-5,2,1-benzoxathiazepine, 3,4-dihydro-2H-5,1,2-benzoxathiazepine, 4,5-dihydro-3,1,4-benzoxathiazepine, 4,5-dihydro-3H-1,2,5-benzoxathiazepine and the like; benzoxadiazepine such as 2,3,4,5-tetrahydro-1,3,4-benzoxadiazepine and the like; benzothiadiazepine such as 2,3,4,5-tetrahydro-1,3,5-benzothiadiazepine and the like; benzotriazepine such as 2,3,4,5-tetrahydro-lH-1,2,5-benzotriazepine and the like; 4,5-dihydro-1,3,2-benzoxathiepin, 4,5-dihydro-1H-2,3-benzoxathiepin, 3,4-dihydro-2H-1,5-benzoxathiepin, 4,5-dihydro-3H-1,2-benzoxathiepin, 4,5-dihydro-3H-2,1-benzoxathiepin, 2,3-dihydro-5H-1,4-benzoxathiepin, 2,3-dihydro-5H-4,1-benzoxathiepin and the like, inter alia, 2,3,4,5-tetrahydro-1H-3-benzazepine, 2,3,4,5-tetrahydro-1H-2-benzazepine, 2,3-dihydro-1H-indole and 2,3,4,5-tetrahydro-1,4-benzoxazepine.

Preferable examples of the case where the "aryl group" in the "optionally substituted aryl group" is fused with a monocyclic heterocyclic ring optionally having a substituent group include a group represented by the formula:

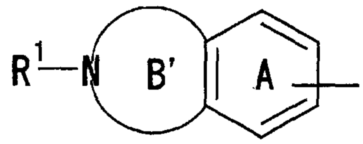

wherein ring B' denotes a 5 to 9 membered nitrogen-containing heterocyclic ring optionally substituted with an oxo group besides $R^1$, and ring A and $R^1$ are as defined above.

Examples of the "5 to 9 membered nitrogen-containing heterocyclic ring" in the "5 to 9 membered nitrogen-containing heterocyclic ring optionally substituted with an oxo group" include a 5 to 9 membered nitrogen-containing heterocyclic group optionally containing 1 to 3 hetero atoms such as a nitrogen atom, an oxygen atom and a sulfur atom in addition to carbon atom and one nitrogen atom, and a 5 to 9 membered non-aromatic nitrogen-containing heterocyclic ring (e.g. pyrrolidine, piperidine, hexamethyleneimine, heptamethyleneimine, piperazine, homopiperazine, tetrahydrooxazepine, morpholine, thiomorpholine etc.) is preferably used. Preferable examples of the case where the "aryl group" in the "optionally substituted aryl group" is fused with a monocyclic heterocyclic ring optionally having a substituent group include, in addition to a group represented by the formula:

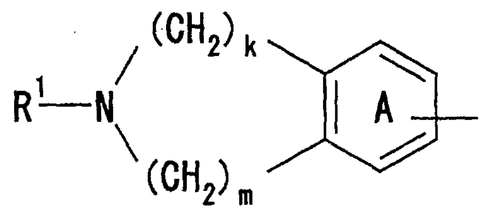

wherein ring A and $R^1$ are as defined above, k and m denote independently an integer of 0 to 5 and $1 < k+m < 5$, groups represented by the formula:

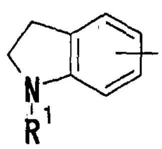
,
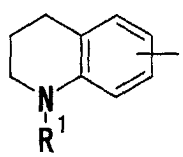
,
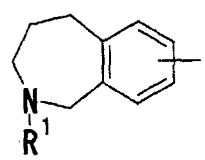
,
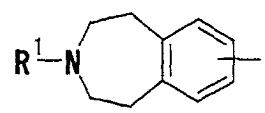

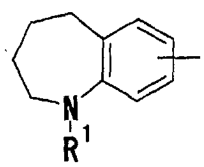
,
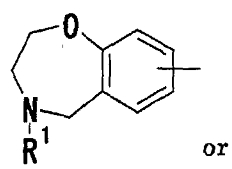
or
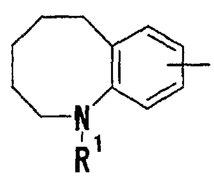

wherein $R^1$ is as defined above, and particularly preferable examples include, in addition to a group represented by the formula:

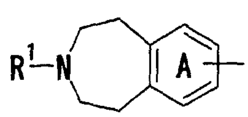

wherein ring A and $R^1$ are as defined above, groups represented by the formula:

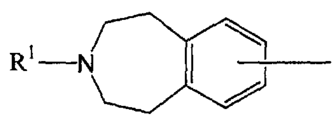
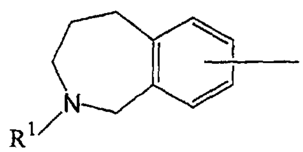
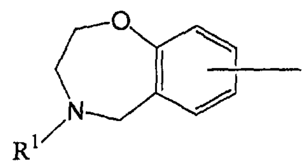

wherein $R^1$ is as defined above.

Specific examples of the case where the "aryl group" in the "optionally substituted aryl group" represented by Ar is fused with a dicyclic heterocyclic ring optionally having a substituent group or the case where the "aryl group" is fused with two identical or different monocyclic rings (provided that at least one ring is a monocyclic heterocyclic ring) include groups represented by the formula:

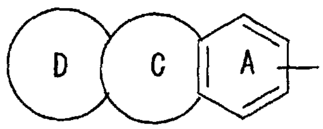
,
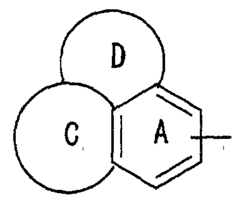
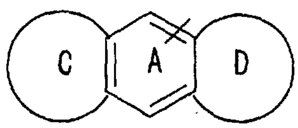
or
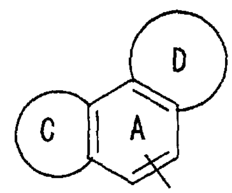

wherein ring A is as defined above, ring C and ring D denote a 5 to 9 membered ring wherein one of them is an optionally substituted heterocyclic ring and the other may have a substituent group and may contain a hetero atom.

As the "heterocyclic ring" in the "optionally substituted heterocyclic ring" represented by the ring C and the ring D, for example, a 4 to 14 membered heterocyclic ring, preferably a 5 to 9 membered heterocyclic ring is used and, as a heteroatom, for example, 1 to 3 heteroatoms selected from a nitrogen atom, an oxygen atom and a sulfur atom are used. In addition, the heterocyclic ring may be aromatic or non-aromatic. Specifically, for example, pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiparazine, tetrahydrooxazepine, morpholine and thiomorpholine are used.

The "substituent group" in the "optionally substituted heterocyclic ring" denotes the same meaning as that of the "substituent group" in the "optionally substituted heterocyclic ring" represented by the aforementioned ring B.

As the "5 to 9 membered ring optionally containing a heteroatom" in the "5 to 9 membered ring optionally having a substituent group and optionally containing a hetero atom" represented by the ring C and the ring D, a 5 to 9 membered heterocyclic ring (e.g. a saturated or unsaturated 5 to 9 membered heterocyclic ring such as pyridine, pyrazine, pyrimidine, imidazole, furan, thiophene, dihydropyridine, diazepine, oxazepine, pyrrolidine, piperidine, hexamethyleneimine, heptamethyleneimine, tetrahydrofuran, piperazine, homopiperazine, tetrahydrooxazepine, morpholine and thiomorpholine) or a 5 to 9 membered carbocyclic ring is used. The "5 to 9 membered carbocyclic ring" may be a saturated or unsaturated ring and, for example, benzene, cyclopentane, cyclopentene, cyclohexane, cyclohexene, cyclohexadiene, cycloheptane, cycloheptene and cycloheptadiene are used. Inter alia, benzene and cyclohexane are preferred.

The "substituent group" in the "5 to 9 membered ring optionally having a substituent group and optionally containing a hetero atom" denotes the same meaning as that of the "substituent group on an arbitrary carbon atom of ring B" in the "optionally substituted heterocyclic ring" represented by the aforementioned ring B.

Specific examples of the case where the "aryl group" in the "optionally substituted aryl group" represented by Ar is fused with a dicyclic heterocyclic ring optionally having a substituent group include:

(1) as a phenyl group fused with a dicyclic heterocyclic ring represented by the formula:

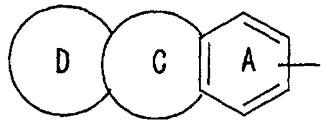

, groups obtained by removing one hydrogen atom from a tricyclic fused benzene ring such as carbazole, 1,2,3,4,4a, 9a-hexahydrocarbazole, 9,10-dihydroacridine, 1,2,3,4-tetrahydroacridine, 10,11-dihydro-5H-dibenz[b,f]azepine, 5,6,7,12-tetrahydrodibenz[b,g]azocine, 6,11-dihydro-5H-dibenz[b,e]azepine, 6,7-dihydro-5H-dibenz[c,e]azepine, 5,6,11,12-tetrahydrodibenz[b,f]azocine, dibenzofuran, 9H-xanthene, 10,11-dihydrodibenz[b,f]oxepin, 6,11-dihydrodibenz[b,e]oxepin, 6,7-dihydro-5H-dibenz[b,g]oxocine, dibenzothiophene, 9H-thioxanthene, 10,11-dihydrodibenzo[b,f]thiepin, 6,11-dihydrodibenzo[b,e]thiepin, 6,7-dihydro-5H-dibenzo[b,g]thiocine, 10H-phenothiazine, 10H-phenoxazine, 5,10-dihydrophenazine, 10,11-dibenzo[b,f][1,4]thiazepine, 10,11-dihydrodibenz[b,f][1,4]oxazepine, 2,3,5,6,11,11a-hexahydro-lH-pyrrolo[2,1-b][3]benzazepine, 10,11-dihydro-5H-dibenzo[b,e][1,4]diazepine, 5,11-dihydrodibenz[b,e][1,4]oxazepine, 5,11-dihydrodibenzo[b,f][1,4]thiazepine, 10,11-dihydro-5H-dibenzo[b,e][1,4]diazepine and 1,2,3,3a,8,8a-hexahydropyrrolo[2,3-b]indole,

(2) as a phenyl group fused with a dicyclic heterocyclic ring represented by the formula:

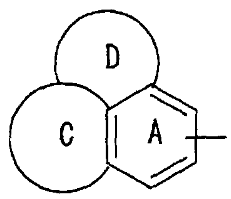

, groups obtained by removing one hydrogen atom from a tricyclic fused benzene ring such as 1H,3H-naphtho [1,8-cd][1,2]oxazine, naphtho[1,8-de]-1,3-oxazine, naphtho[1,8-de]-1,2-oxazine, 1,2,2a,3,4,5-hexahydrobenz[cd] indole, 2,3,3a,4,5,6-hexahydro-lH-benzo[de]quinoline, 4H-pyrrolo[3,2,1-ij]quinoline, 1,2,5,6-tetrahydro-4H-pyrrolo [3,2,1-ij]quinoline, 5,6-dihydro-4H-pyrrolo[3,2,1-ij]quinoline, 1H,5H-benzo[ij]quinolizine, azepino[3,2,1-hi]indole, 1,2,4,5,6,7-hexahydroazepino[3,2,1-hi]indole, 1H-pyrido[3,2,1-jk][1]benzazepine, 5,6,7,8-tetrahydro-1H-pyrido [3,2,1-jk][1]benzazepine, 1,2,5,6,7,8-hexahydro-1H-pyrido[3,2,1-jk][1]benzazepine, 2,3,dihydro-1H-benz[de]isoquinoline, 1,2,3,4,4a,5,6,7-octahydronaphtho[1,8-bc]azepine, and 2,3,5,6,7,8-hexahydro-lH-pyrido[3,2,1-jk][1] benzazepine,

(3) as a phenyl group fused with two identical or different monocyclic rings (provided that at least one ring is a monocyclic heterocyclic ring) represented by the formula:

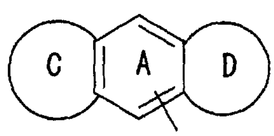

, groups obtained by removing one hydrogen atom from a tricyclic fused benzene ring such as 1,2,3,5,6,7-hexahydrobenzo[1,2-b:4,5-b']dipyrrole and 1,2,3,5,6,7-hexahydrocyclopent[f]indole, and

(4) as a phenyl group fused with two identical or different rings (provided that at least one ring is a monocyclic heterocyclic ring) represented by the formula:

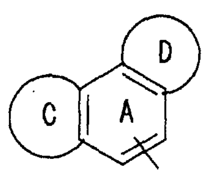

, groups obtained by removing one hydrogen atom from a tricyclic fused benzene ring such as 1,2,3,6,7,8-hexahydrocyclopent[e]indole and 2,3,4,7,8,9-hexahydro-lH-cyclopenta[f]quinoline.

Preferable examples of the case where the "aryl group" in the "optionally substituted aryl group" represented by Ar is fused with a dicyclic heterocyclic ring optionally having a substituent group include groups represented by the formula:

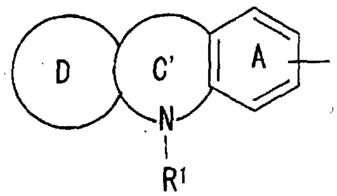
,
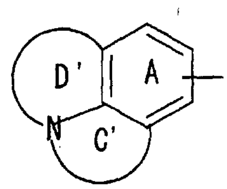

or

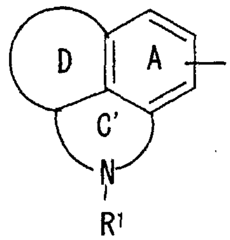

wherein ring C'and ring D'denote a 5 to 9 membered nitrogen-containing heterocyclic ring wherein each may be substituted with an oxo group besides $R^1$, and ring A, ring D and $R^1$ denotes the same meanings as those described above.

Examples of the "5 to 9 membered nitrogen-containing heterocyclic ring" in the "5 to 9 membered nitrogen-containing heterocyclic ring optionally substituted with an oxo group" include a 5 to 9 membered nitrogen-containing heterocyclic group optionally containing 1 to 3 heteroatoms such as a nitrogen atom, an oxygen atom and a sulfur atom in addition to carbon atom and one nitrogen atom, and a 5 to 9 membered non-aromatic nitrogen-containing heterocyclic ring (e.g. pyrrolidine, piperidine, hexamethyleneimine, heptamethyleneimine, piperazine, homopiperazine, tetrahydrooxazepine, morpholine, thiomorpholine etc.) is preferably used.

Preferable examples of the case where the "aryl group" in the "optionally substituted aryl group" represented by Ar is fused with a dicyclic heterocyclic ring optionally having a substituent group include groups represented by the formula:

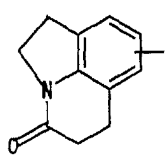
,
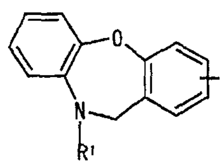
,
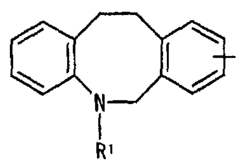
,
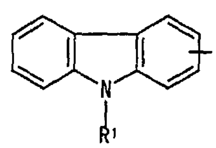
,

or

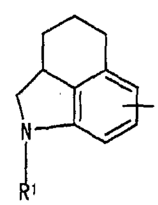

wherein $R^1$ is as defined above.

Specific examples of the case where the "phenyl group" in the "phenyl group which may have a substituent group and may be fused" is fused with a tricyclic heterocyclic ring optionally having a substituent group include groups represented by the formula:

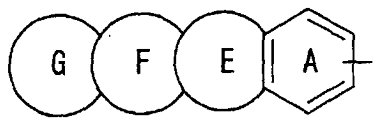 or 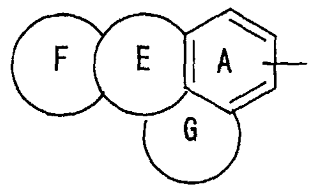

wherein ring A is as defined above, and ring E, ring F and ring G denotes a 5 to 9 membered ring wherein at least one ring of the ring E, the ring F and the ring G is a heterocyclic ring optionally having a substituent group and other rings may have a substituent group and may contain a hetero atom.

As the "heterocyclic ring" and the "substituent group" in the "heterocyclic ring optionally having a substituent group" represented by the ring E, the ring F and the ring G, the "heterocyclic ring" and the "substituent group" in the "optionally substituted heterocyclic ring" represented by the aforementioned ring C and ring D are used.

As the "5 to 9 membered ring optionally containing a hetero atom" and the "substituent group" in the "5 to 9 membered ring optionally having a substituent group and optionally containing a hetero atom" represented by the ring E, the ring F and the ring G, the "5 to 9 membered ring optionally containing a hetero atom" and the "substituent group" in the "5 to 9 membered ring optionally having a substituent group and optionally containing a hetero atom" represented by the aforementioned ring C and ring D are used.

More specific examples of the case where the "phenyl group" in the "phenyl group which may have a substituent group and may be fused" is fused with a tricyclic heterocyclic ring optionally having a substituent group include:

(1) as a phenyl group fused with a tricyclic heterocyclic ring represented by the formula:

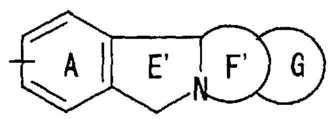

wherein ring E'and ring F'are as defined later, groups obtained by removing one hydrogen atom from a tetracyclic fused benzene ring such as 2H-isoindolo[2,1-e]purine, 1H-pyrazolo[4',3':3,4]pyrido[2,1-a]isoindole, 1H-pyrido[2',3':4,5]imidazo[2,1-a]isoindole, 2H,6H-pyrido[1',2':3,4]imidazo[5,1-a]isoindole, 1H-isoindolo[2,1-a]benzimidazole, 1H-pyrido[3',4':4,5]pyrrolo[2,1-a]isoindole, 2H-pyrido[4',3':4,5]pyrrolo[2,1-a]isoindole, 1H-isoindolo[2,1-a]indole, 2H-isoindolo[1,2-a]isoindole, 1H-cyclopenta[4,5]pyrimido[2,1-a]isoindole, 2H,4H-pyrano[4',3':4,5][1,3]oxazino[2,3-a]isoindole, 2H-isoindolo[2,1-a] [3,1]benzoxazine, 7H-isoindolo[1,2-b][1,3]benzoxazine, 2H-pyrido[2',1':3,4]pyrazino[2,1:a]isoindole, pyrido[2',3':4,5]pyrimido[2,1-a]isoindole, pyrido[3',2':5,6]pyrimido[2,1-a]isoindole, 1H-pyrido[1',2':3,4]pyrimido[2,1-a]isoindole, isoindolo[2,1-a]quinazoline, isoindolo[2,1-a]quinoxaline, isoindolo[1,2-a]isoquinoline, isoindolo[2,1-b]isoquinoline, isoindolo[2,1-a]quinoline, 6H-oxazino[3',4':3,4][1,4]diazepino[2,1-a]isoindole, azepino[2',1':3,4]pyrazino[2,1-a]isoindole, 2H,6H-pyrido[2',1':3,4][1,4]diazepino[2,1-a]isoindole, 1H-isoindolo[1,2-b][1,3,4]benzotriazepine, 2H-isoindolo[2,1-a][1,3,4]benzotriazepine, isoindolo[2,1-d][1,4]benzoxazepine, 1H-isoindolo[2,1-b][2,4]benzodiazepine, 1H-isoindolo[2,1-c][2,3]benzodiazepine, 2H-isoindolo[1,2-a][2,4]benzodiazepine, 2H-isoindolo[2,1-d][1,4]benzodiazepine, 5H-indolo[2,1-b][3]benzazepine, 2H-isoindolo[1,2-a] [2]benzazepine, 2H-isoindolo[1,2-h][3]benzazepine, 2H-isoindolo[2,1-b][2]benzazepine, 2H-isoindolo[1,2-b][1,3,4]benzoxadiazocine, isoindolo[2,1-b][1,2,6]benzotriazocine and 5H-4,8-methano-1H-[1,5]diazacycloundecino[1,11-a]indole,

(2) as a phenyl group fused with a tricyclic heterocyclic ring represented by the formula:

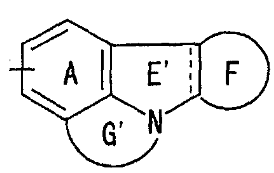

wherein ---- denotes a single bond or a double bond, and ring E'and ring G'are as defined later, groups obtained

by removing one hydrogen atom from a tetracyclic fused benzene ring such as 1H,4H-pyrrolo[3',2':4,5]pyrrolo[3,2,1-ij]quinoline, pyrrolo[3,2,1-jk]carbazole, 1H-furo[2',3':4,5]pyrrolo[3,2,1-ij]quinoline, 1H,4H-cyclopenta[4,5]pyrrolo[1,2,3-de]quinoxaline, 1H,4H-cyclopenta[4,5]pyrrolo[3,2,1-ij]quinoline, pyrido[3',4':4,5]pyrrolo[1,2,3-de]benzoxazine, [1,4]oxazino[2,3,4-jk]carbazole, 1H,3H-[1,3]oxazino[5,4,3-jk]carbazole, pyrido[3',4':4,5]pyrrolo[1,2,3-de][1,4]benzothiazine, 4H-pyrrolo[3,2,1-de]phenanthridine, 4H,5H-pyrido [3,2,1-de]phenanthridine, 1H,4H-3a,6a-diazafluoroanthene, 1-oxa-4,6a-diazafluoroanthene, 4-oxa-2,10b-diazafluoroanthene, 1-thia-4,6a-diazafluoroanthene, 1H-pyrazino[3,2,1-jk]carbazole, 1H-indolo[3,2,1-de][1,5]naphthylidine, benzo[b]pyrano[2,3,4-hi]indolizine, 1H,3H-benzo[b]pyrano[3,4,5-hi]indolizine, 1H,4H-pyrano[2',3':4,5]pyrrolo[3,2,1-ij]quinoline, 1H,3H-benzo[b]thiopyrano[3,4,5-hi]indolizine, 1H-pyrido[3,2,1-jk]carbazole, 4H-3-oxa-11b-azacyclohepta[jk]fluorene, 2H-azepino[1',2':1,2]pyrimidino[4,5-b]indole, 1H,4H-cyclohepta[4,5]pyrrolo[1,2,3-de]quinoxaline, 5H-pyrido[3',4':4,5]pyrrolo[1,2,3-ef][1,5]benzoxazepine, 4H-pyrido[3',4':4,5]pyrrolo[3,2,1-jk][4,1]benzothiazepine, 5H-pyrido[3',4':4,5]pyrrolo[1,2,3-ef][1,5]benzothiazepine, 5H-pyrido[4',3':4,5]pyrrolo[1,2,3-ef][1,5]benzothiazepine, [1,2,4]triazepino[6,5,4-jk]carbazole, [1,2,4]triazepino[6,7,1-jk]carbazole, [1,2,5]triazepino[3,4,5-jk]carbazole, 5H-[1,4]oxazepino[2,3,4-jk]carbazole, 5H-[1,4]thiazepino[2,3,4-jk]carbazole, [1,4]diazepino[3,2,1-jk]carbazole, [1,4]diazepino[6,7,1-jk]carbazole, azepino[3,2,1-jk]carbazole, 1H-cycloocta[4,5]pyrrolo[1,2,3-de]quinoxaline and 1H-cycloocta[4,5]pyrrolo[3,2,1-ij]quinoline,

(3) as a phenyl group fused with a tricyclic heterocyclic ring represented by the formula:

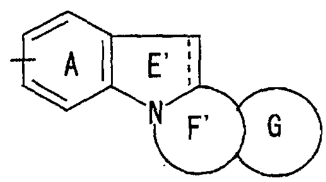

wherein - - - - denotes a single bond or double bond, and ring E'and ring F'are as defined later, groups obtained by removing one hydrogen atom from a tetracyclic fused benzene ring such as lH-indolo[1,2-a]benzimidazole, 1H-indolo[1,2-b]indazole, pyrrolo[2',1':3,4]pyrazino[1,2-a]indole, 1H,5H-pyrrolo[1',2':4,5]pyrazino[1,2-a]indole, 2H-pyrido[2',3':3,4]pyrrolo[1,2-a]indole, 1H-pyrrolo[2',3':3,4]pyrido[1,2-a]indole, 1H-indolo[1,2-a]indole, 6H-isoindolo[2,1-a]indole, 6H-indolo[1,2-c][1,3]benzoxazine, lH-indolo[1,2-b][1,2]benzothiazine, pyrimido[4',5':4,5]pyrimido[1,6-a]indole, pyrazino[2',3':3,4]pyrido[1,2-a]indole, 6H-pyrido[1',2':3,4]pyrimido[1,6-a]indole, indolo[1,2-b]cinnoline, indolo[1,2-a]quinazoline, indolo[1,2-c]quinazoline, indolo[2,1-b]quinazoline, indolo[1,2-a]quinoxaline, indolo[1,2-a][1,8]naphthylidine, indolo[1,2-b]-2,6-naphthylidine, indolo[1,2-b][2,7]naphthylidine, indolo[1,2-h]-1,7-naphthylidine, indolo[1,2-b]isoquinoline, indolo[2,1-a]isoquinoline, indolo[1,2-a]quinoline, 2H,6H-pyrido[2',1':3,4][1,4]diazepino[1,2-a]indole, 1H-indolo[2,1-c][1,4]benzodiazepine, 2H-indoio[1,2-d][1,4]benzodiazepine, 2H-indolo[2,1-a][2,3]benzodiazepine, 2H-indolo[2,1-b][1,3]benzodiazepine, 1H-indolo[1,2-b] [2]benzazepine, 2H-indolo[1,2-a][1]benzazepine, 2H-indolo[2,1-a][2]benzazepine, indolo[1,2-e][1,5]benzodiazocine and indolo[2,1-b][3]benzazocine,

(4) as a phenyl group fused with a tricyclic heterocyclic ring represented by the formula:

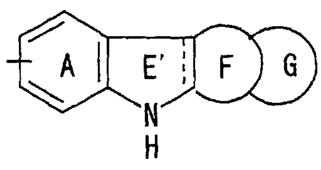

wherein - - - - denotes a single bond or a double bond, and ring E'is as defined later, groups obtained by removing one hydrogen atom from a tetracyclic fused benzene ring such as lH-imidazo[1',2':1,2]pyrido[3,4-b]indole, 1H-imidazo[1',2':1,6]pyrido[4,3-b]indole, 1H-imidazo[1',5':1,2]pyrido[3,4-b]indole, 1H-imidazo[1',5':1,6]pyrido[4,3-b]indole, 1H-pyrido[2',1':2,3]imidazo[4,5-b]indole, imidazo[4,5-a]carbazole, imidazo[4,5-c]carbazole, pyrazolo[3,4-c]carbazole, 2H-pyrazino[1',2':1,5]pyrrolo[2,3-b]indole, 1H-pyrrolo[1',2':1,2]pyrimido[4,5-b]indole, 1H-indolizino[6,7-b]indole, 1H-indolizino[8,7-b]indole, indolo[2,3-b]indole, indolo[3,2-b]indole, pyrrolo[2,3-a]carbazole, pyrrolo[2,3-b]carbazole, pyrrolo[2,3-c]carbazole, pyrrolo[3,2-a]carbazole, pyrrolo[3,2-b]carbazole, pyrrolo[3,2-c]carbazole, pyrrolo[3,4-a]carbazole, pyrrolo[3,4-b]carbazole, pyrrolo[3,4-c]carbazole, 1H-pyrido[3',4':4,5]furo[3,2-b]indole, 1H-furo[3,4-a]carbazole, lH-furo[3,4-b]carbazole, 1H-furo[3,4-c]carbazole, 2H-furo[2,3-a]carbazole, 2H-furo[2,3-c]carbazole, 2H-furo[3,2-a]carbazole, 2H-furo[3,2-c]carbazole, 1H-pyrido[3',4':4,5]thieno[2,3-b]indole, thieno[3',2':5,6]thiopyrano[4,3-b]indole, thieno[3',4':5,6]thiopyrano[4,3-b]indole, 1H-[1]benzothieno[2,3-b]indole, 1H-[1]benzothieno[3,2-b]indole, 1H-thieno[3,4-a]carbazole, 2H-thieno[2,3-b]carbazole, 2H-thieno[3,2-a]carbazole, 2H-thieno[3,2-b]carbazole, cyclopenta[4,5]pyrrolo[2,3-f]qunioxaline, cyclopenta[5,6]pyrido[2,3-b]indole, pyrido[2',3':3,4]cyclopenta[1,2-b]indole, pyrido[2',3':4,5]cyclopenta[1,2-b]indole, pyrido[3',4':3,4]cyclopenta[1,2-b]indole, pyrido[3',4':4,5]cyclopenta[1,2-b]indole, pyrido[4',3':4,5]cyclopenta[1,2-b]indole, 1H-cyclopenta[5,6]pyrano[2,3-b]indole, 1H-cyclopenta[5,6]thiopyrano[4,3-b]indole, cyclopenta[a]carbazole, cyclopenta[c]carbazole, indeno[1,2-b]indole, indeno[2,1-b]indole, [1,2,4]triazino[4',3':1,2]pyrido[3,4-b]indole, 1,3,5-triazino[1',2',1,1]pyrido[3,4-b]indole, 1H-[1,4]oxazino[4',3':1,2]pyrido[3,4-b]indole, 1H-[1,4]oxazino[4',3':1,6]pyrido[3,4-b]indole, 4H-[1,3]oxazino[3',4':1,2]pyrido[3,4-b]indole, indolo[3,2-b][1,4]benzoxazine, 1,3-oxazino[6,5-b]carbazole, 2H-pyrimido[2',1':2,3][1,3]thiazino[5,6-b]indole, 2H-[1,3]thiazino[3',2':1,2]pyrido[3,4-b]indole, 4H-[1,3]thiazino[3',4':1,2]pyrido[3,4-b]indole, indolo[2,3-b] [1,4]benzothiazine, indolo[3,2-b] [1,4]benzothiazine, indolo[3,2-c][2,1]benzothiazine, 1,4-thiazino[2,3-a]carbazole, [1,4]thiazino[2,3-b]carbazole, [1,4]thiazino[2,3-c]carbazole, 1,4-thiazino[3,2-b]carbazole, 1,4-thiazino[3,2-c]carbazole, 1H-indolo[2,3-g]pteridine, 1H-indolo[3,2-g]pteridine, pyrazino[1',2':1,2]pyrido[3,4-b]indole, pyradino[1',2':1,2]pyrido[4,3-b]indole, 1H-pyrido[2',3':5,6]pyrazino[2,3-b]indole, 1H-pyrido[3',2':5,6]pyrazino[2,3-b]indole, 1H-pyrido[3',4':5,6]pyrazino[2,3-b]indole, pyrido[1',2':1,2]pyrimido[4,5-b]indole, pyrido[1',2':1,2]pyrimido[5,4-b]indole, pyrido[2',1':2,3]pyrimido[4,5-b]indole, pyrimido[1',2':1,2]pyrido[3,4-b]indole, pyrimido[1',2':1,6]pyrido[3,4-b]indole, pyrimido[5',4':5,6]pyrano[2,3-b]indole, piridazino[4',5':5,6]thiopyrano[4,5-b]indole, 1H-indolo[3,2-c]cinnoline, 1H-indolo[2,3-b]quinoxaline, 1H-pyrazino[2,3-a]carbazole, 1H-pyrazino[2,3-b]carbazole, 1H-pyrazino[2,3-c]carbazole, 1H-piridazino[3,4-c]carbazole, 1H-piridazino[4,5-b]carbazole, 1H-pyrimido[4,5-a]carbazole, lH-pyrimido[4,5-c]carbazole, 1H-pyrimido[5,4-a]carbazole, 1H-pyrimido[5,4-b]carbazole, 1H-pyrimido[5,4-c]carbazole, 7H-1,4-dioxino[2',3':5,6][1,2]dioxino[3,4-b]indole, 6H-[1,4]benzodioxino[2,3-b]indole, 6H-[1,4]benzodithiino[2,3-b]indole, 1H-indolo[2,3-b]-1,5-naphthylidine, 1H-indolo[2,3-b][1,6]naphthylidine, 1H-indolo[2,3-b][1,8]naphthylidine, 1H-indolo[2,3-c]-1,5-naphthylidine, 1H-indolo[2,3-c][1,6]naphthylidine, 1H-indolo[2,3-c][1,7]naphthylidine, 1H-indolo[2,3-c][1,8]naphthylidine, 1H-indolo[3,2-b]-1,5-naphthylidine, 1H-indolo[3,2-b][1,7]naphthylidine, lH-indolo[3,2-b][1,8]naphthylidine, 1H-indolo[3,2-c][1,8]naphthylidine, indolo[2,3-a]quinolizine, indolo[2,3-b]quinolizine, indolo[3,2-a]quinolizine, indolo[3,2-b]qunolizine, pyrano[4',3':5,6]pyrido[3,4-b]indole, pyrido[4',3':4,5]pyrano[3,2-b]indole, pyrido[4',3':5,6]pyrano[2,3-b]indole, pyrido[4',3':5,6]pyrano[3,4-b]indole, 1H-indolo[2,3-c]isoquinoline, 1H-indolo[3,2-c]isoquinoline, 1H-indolo[2,3-c]quinoline, 1H-indolo[3,2-c]quinoline, 1H-pyrido[2,3-a]carbazole, 1H-pyrido[2,3-b]carbazole, 1H-pyrido[2,3-c]carbazole, 1H-pyrido[3,2-a]carbazole, 1H-pyrido[3,2-b]carbazole, 1H-pyrido[3,2-c]carbazole, 1H-pyrido[3,4-a]carbazole, 1H-pyrido[3,4-b]carbazole, 1H-pyrido[3,4-c]carbazole, 1H-pyrido[4,3-a]carbazole, 1H-pyrido[4,3-b]carbazole, 1H-pyrido[4,3-c]carbazole, 1H-quindoline, 1H-quinindoline, 1H-pyrano[3',4':5,6]pyrano[4,3-b]indole, [1]benzopyrano[2,3-b]indole, [1]benzopyrano[3,2-b]indole, [1]benzopyrano[3,4-b]indole, [l]benzopyrano[4,3-b]indole, [2]benzopyrano[4,3-b]indole, pyrano[2,3-a]carbazole, pyrano[2,3-b]carbazole, pyrano[2,3-c]carbazole, pyrano[3,2-a]carbazole, pyrano[3,2-c]carbazole, pyrano[3,4-a]carbazole, 1H-phosphinolino[4,3-b]indole, [l]benzothiopyrano[2,3-b]indole, [1]benzothiopyrano[3,2-b]indole, [1]benzothiopyrano[3,4-b]indole, [1]benzothiopyrano[4,3-b]indole, [2]benzothiopyrano[4,3-b]indole, 1H-benzo[a]carbazole, 1H-benzo[b]carbazole, lH-benzo[c]carbazole, [1,6,2]oxathiazepino[2',3':1,2]pyrido[3,4-b]indole, 1H-azepino[1',2':1,2]pyrido[3,4-b]indole, 1H-pyrido[1',2':1,2]azepino[4,5-b]indole, 2H-pyrido[1',2':1,2]azepino[3,4-b]indole, 1H-pyrido[3',2':5,6]oxazepino[3,2-b]indole, 1H-pyrido[4',3':5,6]oxepino[3,2-b]indole, 2H-pyrido[2',3':5,6]oxepino[2,3-b]indole, 2H-pyrido[2',3':5,6]oxepino[3,2-b]indole, 2H-pyrido[3',4':5,6]oxepino[3,2-b]indole, pyrido[2',3':4,5]cyclohepta[1,2-b]indole, pyrido[3',2':3,4]cyclohepta[1,2-b]indole, pyrido[3',4':4,5]cyclohepta[1,2-b]indole, pyrido[3',4':5,6]cyclohepta[1,2-b]indole, 2H-pyrano[3',2':2,3]azepino[4,5-b]indole, 1H-indolo[3,2-b][1,5]benzoxazepine, lH-indolo[3,2-d][1,2]benzoxazepine, 1H-indolo[2,3-c][1,5]benzothiazepine, [1,4]diazepino[2,3-a]carbazole, indolo[2,3-b][1,5]benzodiazepine, indolo[2,3-d][1,3]benzodiazepine, indolo[3,2-b][1,4]benzodiazepine, indolo[3,2-b][1,5]benzodiazepine, indolo[3,2-d] [1,3]benzodiazepine, indolo[3,2-d][2,3]benzodiazepine, indolo[2,3-a][3]benzazepine, indolo[2,3-c][1]benzazepine, indolo[2,3-d][1]benzazepine, indolo[2,3-d][2]benzazepine, indolo[3,2-b][1]benzazepine, indolo[3,2-c][1]benzazepine, indolo[3,2-d][1]benzazepine, 1H-indolo[2,1-b][3]benzazepine, 1H-[1]benzoxepino[5,4-b]indole, 1H-[2]benzoxepino[4,3-b]indole, 1H-[1]benzothiepino[4,5-b]indole, 1H-[1]benzothiepino[5,4-b]indole, benzo[3,4]cyclohepta[1,2-b]indole, benzo[4,5]cyclohepta[1,2-b]indole, benzo [5, 6] cyclohepta [1,2-b] indole, benzo[6,7]cyclohepta[1,2-b]indole, cyclohepta[b]carbazole, 4H-[1,5]oxazocino[5',4':1,6]pyrido[3,4-b]indole, azocino[1',2':1,2]pyrido[3,4-b]indole, 2,6-methano-2H-azecino[4,3-b]indole, 3,7-methano-3H-azecino[5,6-b]indole, pyrido[1',2':1,8]azocino[5,4-b]indole, pyrido[4',3':6,7]oxocino[2,3-b]indole, pyrido[4',3':6,7]oxocino[4,3-b]indole, 1,5-methano-1H-azecino[3,4-b]indole, 2,6-methano-1H-azecino[5,4-b]indole, 1H-pyrido[3',4':5,6]cycloocta[1,2-b]indole, 1,4-ethanooxocino[3,4-b]indole, pyrano[3',4':5,6]cycloocta[1,2-b]indole, 1H-indolo[2,3-c][1,2,5,6]benzotetrazocine, 1H-indolo[2,3-c] [1,6]benzodiazocin, 6,13b-methano-13bH-azecino[5,4-b]indole, oxocino[3,2-a]carbazole, 1H-benzo[g]cycloocta[b]indole, 6,3-(iminomethano)-2H-1,4-thiazonino[9,8-b]indole, 1H,3H-[1,4]oxazonino[4',3':1,2]pyrido[3,4-b]indole, 2H-3,6-ethanoazonino[5,4-b]indole, 2H-3,7-methanoazacycloundecino[5,4-b]indole, 1H-6,12b-ethanoazonino[5,4-b]indole, indolo[3,2-e][2]benzazonine, 5,9-methanoazacycloundecino[5,4-b]indole, 3,6-ethano-3H-azecino[5,4-b]indole, 3,7-methano-3H-azacycloundecino[5,4-b]indole, pyrano[4',3':8,9]azecino[5,4-b]indole, 1H-indolo[2,3-c][1,7]benzodiazecine and 1H-indolo[3,2-e] [2]benzazecine.

Further examples include groups obtained by removing one hydrogen atom from a tetracyclic fused benzene ring such as benzo[e]pyrrolo[3,2-b]indole, benzo[e]pyrrolo[3,2-g]indole, benzo[e]pyrrolo[3,2,1-hi]indole, benzo[e]pyrrolo[3,4-b]indole, benzo[g]pyrrolo[3,4-b]indole, 1H-benzo[f]pyrrolo[1,2-a]indole, 1H-benzo[g]pyrrolo[1,2-a]indole, 2H-benzo[e]pyrrolo[1,2-a]indole, 1H-benzo[f]pyrrolo[2,1-a]isoindole, 1H-benzo[g]pyrrolo[2,1-a]isoindole, 2H-benzo[e]pyrrolo[2,1-a]isoindole, isoindolo[6,7,1-cde]indole, spiro[cyclohexane-1,5'-[5H]pyrrolo[2,1-a]isoindole], isoindolo[7,1,2-hij]quinoline, 7,11-methanoazocino[1,2-a]indole, 7,11-methanoazocino[2,1-a]isoindole, dibenz[cd,f]indole, dibenz[cd,g]indole, dibenz[d,f]indole, lH-dibenz[e,g]indole, 1H-dibenz[e,g]isoindole, naphtho[1,2,3-cd]indole, naphtho[1,8-ef]indole, naphtho[1,8-fg]indole, naphtho[3,2,1-cd]indole, 1H-naphtho[1,2-e]indole, 1H-naphtha[1,2-f]indole, 1H-naphtho[1,2-g]indole, 1H-naphto[2,1-e]indole, 1H-naphtho[2,3-e]indole, 1H-naphtho[1,2-f]isoindole, 1H-naphtho[2,3-e]isoindole, spiro[1H-carbazole-1,1'-cyclohexane], spiro[2H-carbazole-2,1'-cyclohexane], spiro[3H-carbazole-3,1'-cyclohexane], cyclohepta[4,5]pyrrolo[3,2-f]quinoline, cyclohepta[4,5]pyrrolo[3,2-h]quinoline, azepino[4,5-b]benz[e]indole, 1H-azepino[1,2-a]benz[f]indole, 1H-azepino[2,1-a]benz[f]isoindole, benzo[e]cyclohepta[b]indole and benzo[g]cyclohepta[b]indole, or

(5) as a phenyl group fused with a tricyclic heterocyclic ring represented by the formula:

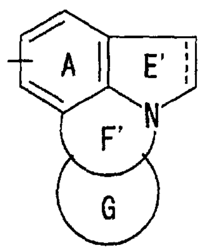

wherein - - - - denotes a single bond or a double bond, and ring E'and ring F'are as defined later, groups obtained by removing one hydrogen atom from a tetracyclic fused benzene ring such as 1H-dipyrrolo[2,3-b:3',2',1'-hi]indole, spiro[cyclopentane-1,2'(1'H)-pyrrolo[3,2,1-hi]indole], spiro[imidazolidine-4,1'(2'H)-[4H]pyrrolo[3,2,1-ij]quinoline], pyrido[2,3-b]pyrrolo[3,2,1-hi]indole, pyrido[4,3-b]pyrrolo[3,2,1-hi]indole, benzo[de]pyrrolo[3,2,1-ij]quinoline, 3H-pyrrolo[3,2,1-de]acridine, 1H-pyrrolo[3,2,1-de]phenanthridine, spiro[cyclohexane-1,6'-[6H]pyrrolo[3,2,1-ij]quinoline], 4,9-methanopyrrolo[3,2,1-1m][1]benzoazocine, spiro[cycloheptane-1,6'-[6H]pyrrolo[3,2,1-ij]quinoline], 1H-pyrano[3,4-d]pyrrolo[3,2,1-jk][1]benzazepine, 3H-benzo[b]pyrrolo[3,2,1-jk][4,1]benzoxazepine, 7H-indolo[1,7-ab][4,1]benzoxazepine, benzo[b]pyrrolo[3,2,1-jk] [1,4]benzodiazepine, indolo[1,7-ab][1,4]benzodiazepine, indolo[1,7-ab][1]benzazepine, indolo[7,1-ab][3]benzazepine, 1H-cyclohepta[d][3,2,1-jk][1]benzazepine, spiro[azepino[3,2,1-hi]indole-7(4H),1'-cycloheptane], 4H-5,11-methanopyrrolo[3,2,1-no][1]benzazacycloundecyne, and spiro[azepino[3,2,1-hi]indole-7(4H),1'-cyclooctane].

In addition, as the "phenyl group fused with a tricyclic heterocyclic ring", as well as the aforementioned phenyl group fused with a tricyclic heterocyclic ring including optionally hydrogenated indole ring and isoindole ring, phenyl groups fused with the following exemplified tricyclic heterocyclic rings and a dihydro compound, a tetrahydro compound, a hexahydro compound, an octahydro compound and a decahydro compound thereof are used. Specifically, examples thereof include fluoranthene, acephenanthrylene, aceanthrylene, triphenylene, pyrene, chrysene, naphthacene, pleiadene, benzo[a]anthracene, indeno[1,2-a]indene, cyclopenta[a]phenanthrene, pyrido[1',1':1,2]imidazo[4,5-b]quinoxaline, 1H-2-oxapyrene and spiro[piperidine-4,9'-xanthene].

Preferable examples of the case where the "phenyl group" in the "phenyl group which may have a substituent group and may be fused" is fused with a tricyclic heterocyclic ring optionally having a substituent group include groups represented by the formula:

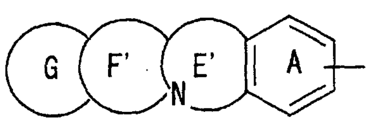

or

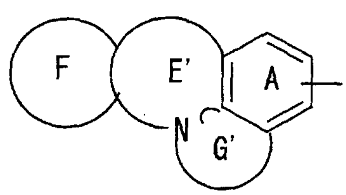

wherein ring E', ring F'and ring G'denote a 5 to 9 membered nitrogen-containing heterocyclic ring optionally substituted with an oxo group in addition to $R^1$, and ring A, ring F, ring G and $R^1$ denote the same meanings as described above.

Inter alia, a group represented by the formula:

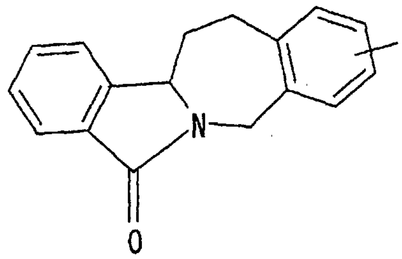

is particularly preferred.

As the "5 to 9 membered nitrogen-containing heterocyclic ring" in the "5 to 9 membered nitrogen-containing heterocyclic ring optionally substituted with an oxo group", the "5 to 9 membered nitrogen-containing heterocyclic ring" represented by the aforementioned ring C' and ring D' are used.

Preferable examples of the case where the "optionally substituted aryl group" represented by Ar is fused with (2) a dicyclic heterocyclic ring optionally having a substituent group, or two identical or different monocyclic rings (provided that at least one ring is a monocyclic heterocyclic ring), and the case where the "optionally substituted aryl group" is fused with (3) a tricyclic heterocyclic ring optionally having a substituent group, include groups wherein Ar is represented by the formulas:

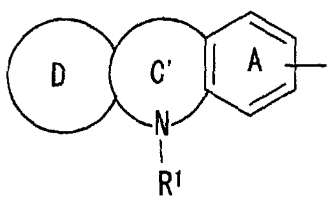
,
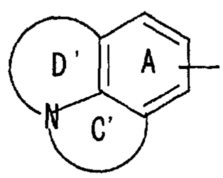
,
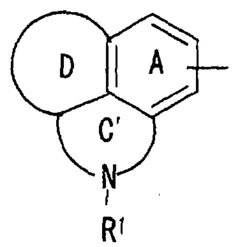
,

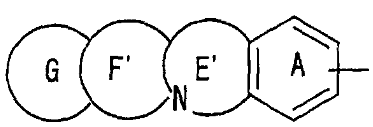
or
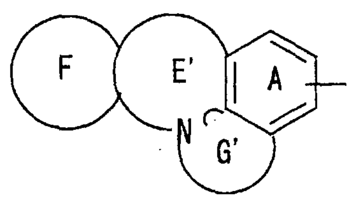

wherein respective symbols are as defined above.

Particularly preferable examples of the "optionally substituted aryl group" represented by Ar include groups represented by the formulas:

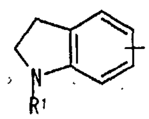 , 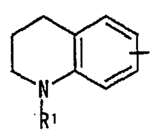 , 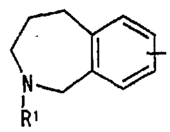 , 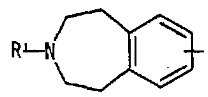 ,

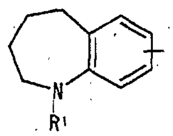 , 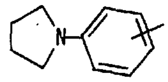 , 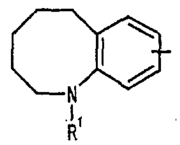 ,

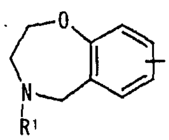 , 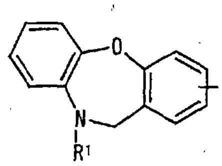 , 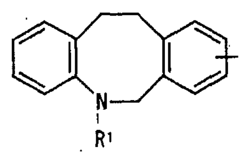 , 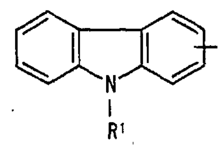 ,

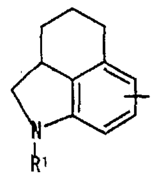 , 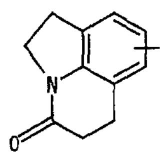 or 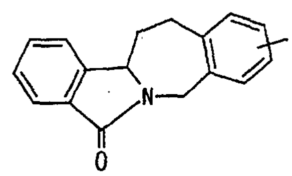

wherein $R^1$ is as defined above and, inter alia, groups represented by the formulas:

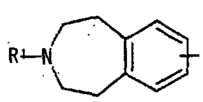 , 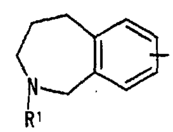 or 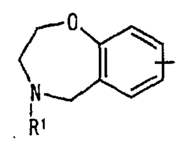

wherein $R^1$ is as defined above, are preferred.

In the formulas above, n denotes an integer of 1 to 10. Preferable n is an integer of 1 to 6, particularly preferably 1 to 5, more preferably 2 to 5, further preferably 3,4 or 5.

In the formulas above, R denotes a hydrogen atom or an optionally substituted hydrocarbon group, and may be different in repetition of n.

The "hydrocarbon group" and the "substituent group" in the "optionally substituted hydrocarbon group" represented by R denote the same meanings as those of the "hydrocarbon group" and the "substituent group" in the "optionally substituted hydrocarbon group" represented by the aforementioned $R^1$.

In addition, R may be bound to Ar or a substituent group of Ar.

Examples of the compound, represented by the formula

[I] wherein R is bound to Ar or a substituent group of Ar include a compound represented by the formula:

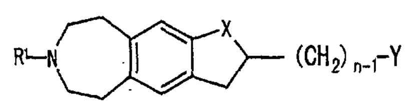

wherein $R^1$, n, X and Y denote the same meanings as those described above, a compound represented by the formula:

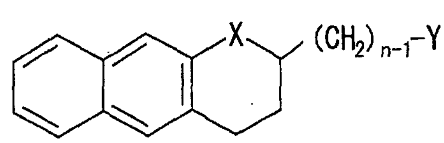

wherein n, X and Y denote the same meanings as those described above, and a compound represented by the formula:

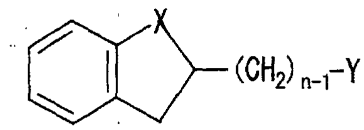

wherein n, X and Y denote the same meanings as those described above.

As R, a hydrogen atom is preferred.

In the formulas above, Y denotes an optionally substituted amino group or an optionally substituted nitrogen-containing heterocyclic group (preferably nitrogen-containing saturated heterocyclic group) [Y is preferably an optionally substituted amino group]. In addition, Y' denotes an optionally substituted amino group.

As the "optionally substituted amino group" represented by Y and Y', for example, a group represented by the formula:

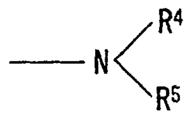

wherein $R^4$ and $R^5$ are the same or different and denote a hydrogen atom, an optionally substituted hydrocarbon group or an optionally substituted acyl group, and $R^4$ and $R^5$ may be bound to each other to form a ring, is used.

As the "substituent group" and the "hydrocarbon group" in the "optionally substituted hydrocarbon group" represented by $R^4$ and $R^5$, for example, the "substituent group" and "hydrocarbon group" in the "optionally substituted hydrocarbon group" described for the aforementioned $R^1$ are used.

Preferable examples of the optionally substituted hydrocarbon group represented by $R^4$ and $R^5$ include ① a straignt or branched lower alkyl group (e.g. a $C_{1-6}$alkyl group such as methyl, ethyl, propyl, isopropyl, butyl, isobutyl, tert-butyl, sec-butyl, pentyl, hexyl etc.) optionally having 1 to 3 substituents selected from (i) a halogen atom (e.g. fluoro, chloro, bromo, iodo etc.) (ii) a lower alkoxy group (e.g. a $C_{1-6}$alkoxy group such as methoxy, ethoxy, n-propyloxy, i-propyloxy, n-butyloxy etc.), and (iii) a hydroxyl group, and ② a lower aralkyl group (e.g. a $C_{7-16}$aralkyl group such as pheny-$C_{1-10}$alkyl (e.g. benzyl, phenylethyl, phenylpropyl, phenylbutyl, phenylpentyl, phenylhexyl etc.), naphthyl-$C_{1-6}$alkyl group (e.g. α-naphthylmethyl etc.) or diphenyl-$C_{1-3}$alkyl (e.g. diphenylmethyl, diphenylethyl etc.)) optionally having 1 to 3 substituents selected from (i) a halogen atom (e.g. fluoro, chloro, bromo, iodo etc.) (ii) a lower alkoxy group (e.g. a $C_{1-6}$alkoxy group such as methoxy, ethoxy, n-propyloxy, i-propyloxy, n-butyloxy etc.), and (iii) a hydroxyl group.

More preferably, examples thereof include ① an unsubstituted straight or branched lower alkyl group (e.g. a $C_{1-6}$alkyl group such as methyl, ethyl, propyl, isopropyl, butyl, isobutyl, tert-butyl, sec-butyl, pentyl, hexyl etc.) and ② an unsubstituted lower aralkyl group (e.g. a $C_{7-16}$aralkyl group such as phenyl-$C_{1-10}$alkyl (e.g. benzyl, phenylethyl, phenylpropyl, phenylbutyl, phenylpentyl, phenylhexyl etc.), naphthyl-$C_{1-6}$alkyl (e.g. α-naphthylmethyl etc.) and diphenyl-$C_{1-3}$alkyl (e.g. diphenylmethyl, diphenylethyl etc.))

As the "optionally substituted acyl group" represented by $R^4$ and $R^5$, for example, the "optionally substituted acyl group" described for the aforementioned $R^1$ is used.

In addition, as the specific examples of the case where $R^4$ and $R^5$ are bound to each other to form a ring in the "optionally substituted amino group" represented by Y and Y', that is, the case where the "optionally substituted amino group" represented by Y and Y' denotes an "optionally substituted cyclic amino group", a group represented by the formula:

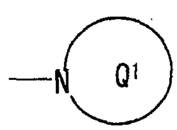

wherein ring $Q^1$ denotes a 5 to 9 membered nitrogen-containing heterocyclic group (preferably nitrogen-containing saturated heterocyclic group) optionally containing 1 to 2 hetero atoms selected from a nitrogen atom, an oxygen atom and a sulfur atom in addition to carbon atom and one nitrogen atom, is used. More specifically, for example,

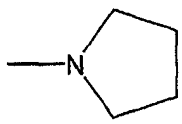 , 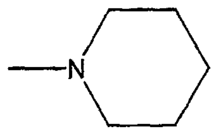 , 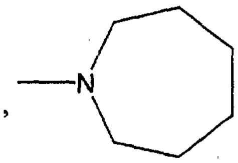 , 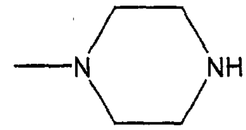 ,

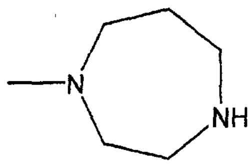 , 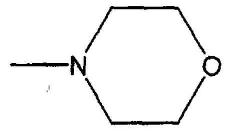 or 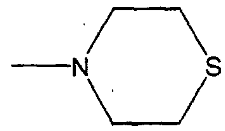

are frequently used.

As the "substituent group" in the "optionally substituted cyclic amino group" as the "optionally substituted amino group" represented by Y and Y', for example, the "substituent group" in the "nitrogen-containing heterocyclic ring optionally having a substituent" which may be formed by the aforementioned $R^{2c}$ and $R^{3c}$ together with an adjacent nitrogen atom, and the "optionally substituted hydrocarbon group, optionally substituted acyl group or optionally substituted heterocyclic group" represented by the aforementioned $R^1$ are used.

As the "optionally substituted amino group" represented by Y and Y',

(1) a group represented by the formula:

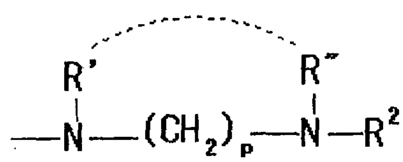

wherein $R^2$ denotes a hydrogen atom, an optionally substituted acyl group, an optionally substituted hydrocarbon group or an optionally substituted heterocyclic group, p denotes an integer of 1 to 3, R' and R" denote a hydrogen atom or an optionally substituted alkyl group, respectively, or R' and R" may be bound to each other to form a ring; and (2) an optionally substituted piperidino group are preferable and, inter alia,

(1a) a group represented by the formula:

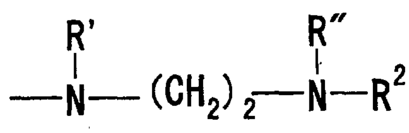

wherein $R^2$ denotes a hydrogen atom, an optionally substituted acyl group, an optionally substituted hydrocarbon group or an optionally substituted heterocyclic group, and R' and R" denote a hydrogen atom or an optionally substituted alkyl group, respectively, and

(1b) a group represented by the formula:

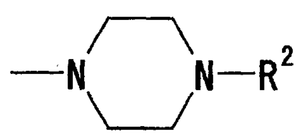

wherein $R^2$ denotes a hydrogen atom, an optionally substituted acyl group, an optionally substituted hydrocarbon group, or an optionally substituted heterocyclic group; are preferably used.

Herein, examples of the "optionally substituted acyl group", the "optionally substituted hydrocarbon group" and the "optionally substituted heterocyclic group" represented by $R^2$ include the same as the "optionally substituted acyl group", the "optionally substituted hydrocarbon group" and the "optionally substituted heterocycli group" represented by $R^1$.

Examples of an "alkyl group" in the "optionally substituted alkyl group" represented by R' and R" include a $C_{1-6}$alkyl group, and examples of the "substituent group" of the "alkyl group" include the same substituent group as the "substituent group" of the "optionally substituted hydrocarbon group" represented by the aforementioned $R^1$.

In addition, when R' and R" are bound to each other to form a ring, among the "nitrogen-containing heterocyclic groups" exemplified as the aforementioned ring $Q^1$, a preferable example is a 5 to 9 membered nitrogen-containing heterocyclic group (preferably nitrogen-containing saturated heterocyclic group) optionally containing one hetero atom selected from a nitrogen atom, an oxygen atom and a sulfur atom in addition to carbon atom and two nitrogen atoms and, as such the ring, a 5 to 9 membered nitrogen-containing heterocylic ring (preferably nitrogen-containing saturated heterocyclic ring) composed of carbon atoms and two nitrogen atoms is preferable, and these rings may further have the same substituent groups as those of the aforementioned ring $Q^1$.

The optionally substituted piperidino group as Y may have, as a substituent group, the "optionally substituted acyl group", the "optionally substituted hydrocarbon group" and the "optionally substituted heterocyclic group" represented by the aforementioned $R^1$.

As the "nitrogen-containing heterocyclic group" in the "optionally substituted nitrogen-containing heterocyclic group" represented by Y, a 5 to 9 membered nitrogen-containing heterocyclic group (preferably nitrogen-containing saturated heterocyclic group) optionally containing 1 to 3 hetero atoms such as a nitrogen atom, an oxygen atom and a sulfur atom in addition to carbon atom and one nitrogen atom, is used. These nitrogen-containing heterocyclic groups may be a group having a bond on a ring-constituting nitrogen atom, or a group having a bond on a ring-constituting carbon atom. As the group having a bond on a ring-constituting nitrogen atom, for example, a group represented by the formula:

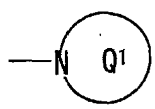

wherein ring $Q^1$ denotes a 5 to 9 membered nitrogen-containing heterocyclic group (preferably nitrogen-containing saturated heterocyclic group) optionally containing 1 to 2 hetero atoms selected from a nitrogen atom, an oxygen atom and a sulfur atom in addition to carbon atom and one nitrogen atom, is used. More specifically, for example,

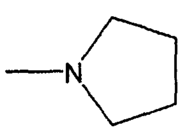 , 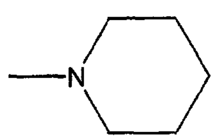 , 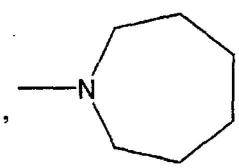 , 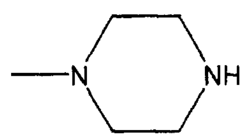 ,

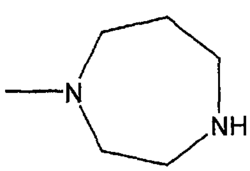 , 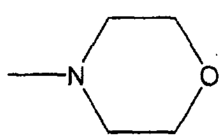 or 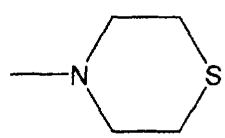

are frequently used.

In addition, as the group having a bond on a ring-constituting carbon atom, for example, a group represented by the formula;

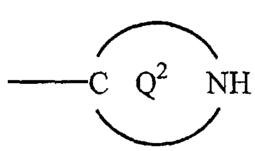

wherein ring $Q^2$ denote a 5 to 9 membered nitrogen-containing heterocyclic group (preferably nitrogen-containing saturated heterocyclic group) optionally containing 1 to 2 hetero atoms selected from a nitrogen atom, an oxygen atom and a sulfur atom in addition to carbon atom and one nitrogen atom, is used. More specifically, for example,

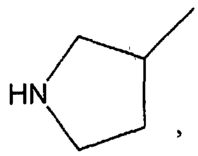 , 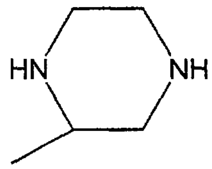 , 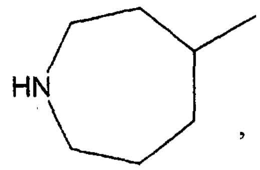 , 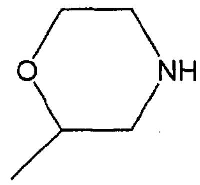 ,

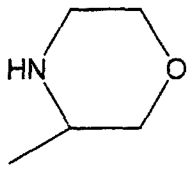 or 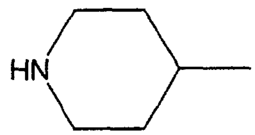

are frequently used.

As the "substituent group" in the "optionally substituted nitrogen-containing heterocyclic group (preferably nitrogen-containing saturated heterocyclic group)" represented by Y, for example, the "substituent group" in the "optionally substituted nitrogen-containing heterocyclic ring" which may be formed by the aforementioned $R^{2c}$ and $R^{3c}$ together with an adjacent nitrogen atom, and the "optionally substituted hydrocarbon group, optionally substituted acyl group or optionally substituted heterocyclic group" represented by the aforementioned $R^1$ are used.

In addition, in circumstances where an "optionally substituted cyclic amino group" as the "optionally substituted amino group" represented by Y and Y'; and the "optionally substituted nitrogen-containing heterocyclic group" represented by Y have two or more substituent groups, the substituent groups may be bound to each other to form a ring, and examples of such the ring include a benzene ring, a 5 to 8 membered (preferably 5 to 6 membered) aromatic monocyclic heterocyclic ring (e.g. pyrrole, oxazole, isoxazole, thiazole, isothiazole, imidazole, pyrazole, 1,2,3-oxadiazole, 1,2,4-oxadiazole, 1,3,4-oxadiazole, 1,2,3-thiadiazole, 1,2,4-thiadiazole, 1,3,4-thiadiazole, 1,2,3-triazole, 1,2,4-triazole, tetrazole, pyridine, piridazine, pirimidine, pyrazine, triazine, etc.), and rings in which a part or all of unsaturated bonds of these rings are converted into saturated bonds.

Further, when an "optionally substituted cyclic amino group" as the "optionally substituted amino group" represented by Y and Y'; as well as the "optionally substituted nitrogen-containing heterocyclic group" represented by Y have two or more substituent groups on one carbon atom, the substituent groups may be bound to each other to form a spiro ring, and examples of the case such the spiro ring is formed include a spiro (1H-indene-1,4'-piperidinyl) ring.

Preferable examples of the "nitrogen-containing heterocyclic group" in the "optionally substituted nitrogen-containing heterocyclic group" represented by Y include a 4-piperidinyl group, 1-piperidinyl group and 1-piperazinyl group.

That is, as Y, a group represented by the formula:

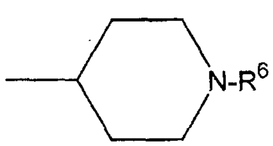 , 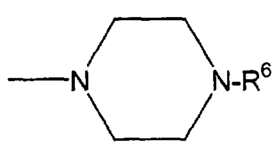 or 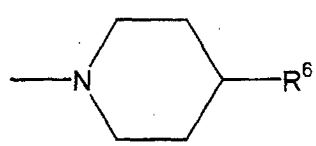

wherein $R^6$ denotes the same meaning as that of $R^1$, is preferred.

More preferable examples of Y include groups represented by the formula:

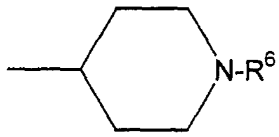 , 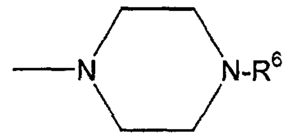 or 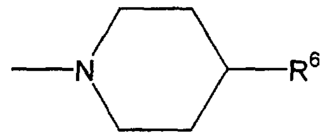

wherein $R^6$ denotes (i) phenyl-$C_{1-6}$alkyl optionally substituted with $C_{1-6}$alkyl, $C_{1-6}$alkoxy, halogen atom, nitro, mono-or di-$C_{1-6}$alkyl-carbamoyloxy, hydroxyl, cyano, carboxyl, $C_{1-6}$alkoxycarbonyl, carbamoyl, cyclic aminocarbonyl, amino, $C_{1-6}$alkylcarbonylamino, phenylsulfonylamino, $C_{1-6}$alkylsulfonylamino, amidino, ureido or heterocyclic ring (the aforementioned $C_{1-6}$alkyl and $C_{1-6}$alkoxy, carbamoyl, cyclic aminocarbonyl, amino, phenylsulfonylamino, amidino, ureido and heterocyclic ring may further have a substituent group and, as the "substituent group", for example, the "substituent group" of the "optionally substituted hydrocarbon group" represented by $R^1$ is used), (ii) a hydrogen atom, (iii) a $C_{1-6}$alkyl group optionally substituted with halogen atom, hydroxyl, $C_{1-6}$alkoxy, amino, mono- or di-$C_{1-6}$alkylamino, carboxyl, cyano or $C_{1-6}$alkoxy-carbaonyl or (iv) a $C_{1-6}$alkylcarbonyl group optionally substituted with mono or di-$C_{1-6}$alkylamino or $C_{1-6}$alkoxy-carbonyl, preferably, a benzyl group optionally substituted with $C_{1-4}$alkyl (e.g. methyl), trihalogeno$C_{1-4}$alkyl (e.g. methyl), halogen atom (e.g. fluoro, chloro), nitro, cyano, $C_{1-4}$alkoxy (e.g. methoxy), hydroxyl, carbamoyl, (4-$C_{1-4}$alkyl (e.g. methyl)-1-piperazinyl)carbonyl, aminothiocarbonyl, morpholinocarbonyl, carboxyl, $C_{1-4}$alkoxy (e.g. methoxy)carbonyl, $C_{1-4}$alkoxy (e.g. ethoxy)carbonyl$C_{1-4}$alkoxy(e.g. methoxy), carboxyl$C_{1-4}$alkoxy (e.g. methoxy), $C_{1-4}$alkoxy(e.g. ethoxy)carbonyl$C_{1-6}$alkyl(e.g. isopropyl), carboxyl$C_{1-6}$alkyl(e.g. isopropyl), amino, acetylamino, $C_{1-4}$alkyl(e.g. methyl)sulfonylamino, (4-$C_{1-4}$alkyl(e.g. methyl)phenyl)sulfonylamino, uerido, 3-$C_{1-4}$alkyl (e.g. methyl)ureido, amidino, dihydrothiazolyl or dihydroimidazolyl.

Inter alia, it is preferable that $R^6$ is a benzyl group optionally substituted with $C_{1-4}$alkyl(e.g. methyl), trihalogeno (e.g. fluoro)$C_{1-4}$alkyl(e.g. methyl), halogen atom(e.g. fluoro, chloro), nitro, hydroxyl, carbamoyl, amino, amidino or dihydroimidazolyl.

As Y, in particular, a 1-benzyl-4-piperidinyl group, a 4-benzyl-1-piperidinyl group, a 4-benzyl-1-piperazinyl group, a 1-acetyl-4-piperidinyl group, a 1-[(2-methylphenyl)methyl]-4-piperidinyl group, a 1-[(3-chlorophenyl)methyl]-4-piperidinyl group, a 1-[(2-chlorophenyl)methyl]-4-piperidinyl group, a 1-[(3-nitrophenyl)methyl]-4-piperidinyl group, and 1-[[3-(trifluoromethyl)phenyl]methyl]-4-piperidinyl group are preferable, and a 1-benzyl-4-piperidinyl group, a 1-acetyl-4-piperidinyl group, a 1-[(2-methylphenyl)methyl]-4-piperidinyl group, a 1-[(3-chlorophenyl)methyl]-4-piperidinyl group, a 1-[(2-chlorophenyl)methyl]-4-piperidinyl group, a 1-[(3-nitrophenyl)methyl]-4-piperidinyl group, and 1-[[3-(trifluoromethyl)phenyl]methyl]-4-piperidinyl group are frequently used.

Examples of the "spacer wherein the number of atoms constituting a straight chain moiety is 1 to 4" represented by X in the aforementioned formula include a saturated divalent group and a divalent group wherein a part of a bond is converted into an unsaturated bond such as :

(1) $-(CH_2)_{f1}-$ (fl denotes an integer of 1 to 4),

(2)$-(CH_2)_{g1}-X^1-(CH_2)_{g2}-$ (g1 and g2 are the same or different and denote an integer of 0 to 3, provided that a sum of g1 and g2 is 1 to 3, and $X^1$ denotes NH, 0, S, SO or $SO_2$),

(3) $(CH_2)_{h1}-X^1-(CH_2)_{h2}-X^2-(CH_2)_{h3}-$(h1, h2 and h3 are the same or different and denote an integer of 0 to 2, provided that a sum of h1, h2 and h3 is 0 to 2, $X^1$ and $X^2$ denote NH, 0, S, SO or $SO_2$, respectively, provided that when h2 is 0, then at least one of $X^1$ and $X^2$ denotes preferably NH); and a divalent group wherein the number of atoms constituting a straight chain moiety is 0 to 4, such as -CO-, -O-, $-NR^{3a}-$, - S-, -SO-, $-SO_2-$, $-SO_2NR^{3a}-$, $-SO_2NHCONR^{3a}-$, $-SO_2NHC$ (=NH) $NR^{3a}-$, -CS-,$-CR^{3a}$ $(R^{3b})-$, $-C(-CR^{3a}$ $(R^{3b}))-$, $-C(-NR^{3a})-$, $-CONR^{3a}-$ (wherein $R^{3a}$ and $R^{3b}$ denote independently a hydrogen atom, a cyano group, hydroxyl group, an amino group, a $C_{1-6}$alkyl group or a $C_{1-6}$alkoxy group).

As X, -CO-, -O-, $-NR^{3a}-$, -S-, -SO-, $-SO_2-$, $-SO_2NR^{3a}-$, $-SO_2NHCONR^{3a}-$, $-SO_2NHC$ (=NH) $NR^{3a}-$, -CS-, $-CR^{3a}$ $(R^{3b})$ -, $-C(=CR^{3a}(R^{3b}))-$,$-C(=NR^{3a})-$, $-CONR^{3a}-$ (wherein $R^{3a}$ and $R^{3b}$ denote independently a hydrogen atom, a cyano atom, a hydroxyl group, an amino group, a $C_{1-6}$alkyl group or a $C_{1-6}$alkoxy group) are more preferable and, inter alia, -CO-, -O-, $-SO_2-$, $-SO_2NR^{3a}-$,-,$-CR^{3a}(R^{3b})-$, $-CONR^{3a}-$ are preferable, in particular, $-SO_2NR^{3a}-$,$-CONR^{3a}-$, -, $-CR^{3a}$ $(R^{3b})-$ are preferably used.

A divalent group represented by X may have a substituent group on an arbitrary position (preferably, on a carbon atom), and examples of such the substituent group include lower $(C_{1-6})$alkyl (e.g. methyl, ethyl, propyl, isopropyl, butyl, isobutyl, sec-butyl, tert-butyl, pentyl, isopentyl, neopentyl, hexyl etc.), lower $(C_{3-7})$cycloalkyl (e.g. cyclopropyl, cyclobutyl, cyclopentyl, cyclohexyl, cycloheptyl etc.), formyl, lower $(C_{2-7})$alkanoyl (e.g. acetyl, propionyl, butyryl etc.), lower $(C_{1-6})$lower alkoxy-carbonyl, lower $(C_{1-6})$lower alkoxy, hydroxyl group and oxo.

Among the compounds represented by the formula (I) or salts thereof, compounds represented by the formula (II):

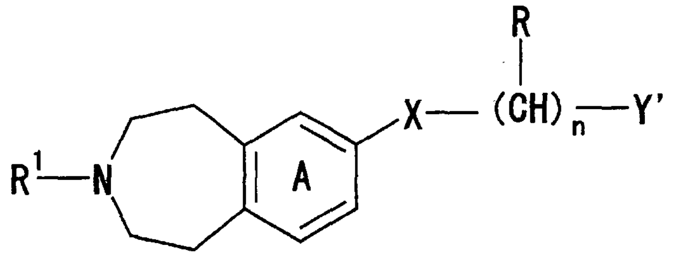

(II)

wherein $R^1$ denotes a hydrogen atom, an optionally substituted hydrocarbon group or an optionally substituted acyl group, ring A denotes a benzene ring optionally further having a substituent group, X denotes a spacer wherein the number of atoms constituting a straight chain moiety is 1 to 4 (excluding -CO-), n denotes an integer of 1 to 10, R is a hydrogen atom or an optionally substituted hydrocarbon group and may be the same or different in repetition of n, or R may be bound to ring A or a substituent group of ring A to form a ring, Y denotes an optionally substituted amino group, or salts thereof are preferably used.

Examples of salts of compounds having GPR 14-antagonistic activity to be used in the present invention [including compounds represented by formula (I) and (II)] preferably include pharmaceutically acceptable salts such as salts with inorganic base, organic base, inorganic acid, organic acid, or basic or acidic amino acid.

Preferable examples of salts with inorganic base include alkaline metal salts such as sodium salts and potassium salts; alkaline earth metal salts such as calcium salts and magnesium salts; and aluminium salts and ammonium salts, etc.

Preferable examples of salts with organic base include salts with, for example, trimethylamine, triethylamine, pyridine, picoline, ethanolamine, diethanolamine, triethanolamine, dicyclohexylamine or N,N'-dibenzylethylenediamine, etc.

Preferable examples of salts with inorganic acid include salts with, for example, hydrochloric acid, hydrobromic acid, nitric acid, sulfuric acid or phosphoric acid, etc.

Preferable examples of salts with organic acid include salts with, for example, formic acid, acetic acid, trifluoroacetic acid, fumaric acid, oxalic acid, tartaric acid, maleic acid, citric acid, succinic acid, malic acid, methansulfonic acid, benzenesulfonic acid or p-toluenesulfonic acid, etc.

Preferable examples of salts with basic amino acid include salts with, for example, arginine, lysine or ornithine, etc. Preferable examples of salts with acidic amino acid include salts with, for example, aspartic acid or glutamic acid, etc.

Compounds having GPR 14-antagonistic activity to be used in the present invention [including compounds represented by formula (I) and (II)] may be hydrates or non-hydrates. Compounds having GPR 14-antagonistic activity to be used in the present invention [including compounds represented by formula (I) and (II)] can be individually isolated by any known means for separation/purification as desired when they are present as configurational isomers, diastereoisomers or conformers. Compounds having GPR 14-antagonistic activity to be used in the present invention [including compounds represented by formula (I) and (II)] can be separated into S-compound and R-compound by any conventional optical resolution means when they are present as racemic compounds. All of those optically active compounds and racemic compounds are encompassed by the present invention.

Compounds having GPR 14-antagonistic activity to be used in the present invention and salts thereof [including compounds represented by formula (I) and (II) and salts thereof] [hereinafter sometimes referred to as GPR14 antagonist] may be use as prodrugs. Examples of such prodrug may include compounds which may be converted into GPR14 antagonist through, for example, enzyme- or gastric acid-mediated reaction *in vivo* under physiological conditions, i.e., compounds which may be enzymatically oxidized, reduced and/or hydrolyzed to be converted into GPR14 antagonist, and compounds which may be hydrolyzed by gastric acid and the like to be converted into GPR14 antagonist. Examples of prodrug of GPR14 antagonist include compounds comprising GPR 14 antagonist in which amino group or groups have been acylated, alkylated or phosphorylated (e.g., compounds comprising GPR14 antagonist in which amino group or groups have been eicosanoylated, alanylated, pentylaminocarbonylated, (5-methyl-2-oxo-1,3-dioxolene-4-yl) methoxycarbonylated, tetrahydrofuranylated, pyrrolidylmethylated, pivaloyloxymethylated, or tert-butylated); compounds comprising GPR 14 antagonist in which hydroxy group or groups have been acylated, alkylated, phosphorylated or borated (e.g., compounds comprising GPR14 antagonist in which hydroxy group or groups have been acetylated, palmitoylated, propanoylated, pivaloylated, succinylated, fumarylated, alanylated or dimethylamino methylcarbonylated); compounds comprising GPR 14 antagonist in which carboxyl group or groups have been esterified or amidated (e.g., compounds comprising GPR 14 antagonist in which carboxyl group or groups have been ethylesterified, phenylesterified, carboxymethyl esterified, dimethylaminomethyl esterified, pivaloyloxymethyl esterified,

ethoxycarbonyloxyethyl esterified, phthalidyl esterified, (5-methyl-2-oxo-1,3-dioxolene-4-yl)methyl esterified, cyclohexyloxycarbonylethyl esterified or methylamidated, etc.). These compounds can be prepared from GPR14 antagonist using any known method.

Further, prodrugs of GPR14 antagonist may be compounds which may be converted into GPR14 antagonist under physiological conditions as described in "Development of pharmaceuticals (Iyakuhinn no Kaihatsu)", vol. 7, Molecular Design pp.163-198, Hirokawa Shoten (1990).

GPR14 antagonist may be labeled with any suitable isotope such as $^3H$, $^{14}C$, $^{35}S$, $^{125}I$, etc.

GPR14 antagonist according to the present invention may be used alone or in combination with pharmaceutically acceptable carrier or carriers, to formulate solid (such as tablet, capsule, granule or powder) or liquid (such as syrup or injection) formulations which can then be administered orally or parenterally.

Dosage forms for parenteral administration include, for example, injection, instillation and suppository.

Examples of pharmaceutically acceptable carrier include various organic or inorganic carrier materials which have been conventionally used as formulation bases. Excipient, lubricant, binder and/or disintegrator may be used for solid formulations while solvent, dissolution adjuvant, suspending agent, isotonizing agent, buffer and/or soothing agent may be used for liquid formulations. Additive or additives may be added when required, including preservative, antioxidant, colorant and/or sweetening agent. Preferable examples of excipient include lactose, saccharose, D-mannitol, starch, crystalline cellulose or light anhydrous silicic acid, etc. Preferable examples of lubricant include, for example, magnesium stearate, calcium stearate, talc or colloidal silica, etc. Preferable examples of binder include, for example, crystalline cellulose, saccharose, D-mannitol, dextrin, hydroxypropylcellulose, hydroxypropylmethylcellulose, polyvinylpyrrolidone, etc. Preferable examples of disintegrator include, for example, starch, carboxymethyl cellulose, carboxy methylcellulose calcium, crosscarmellose sodium or sodium carboxymethyl starch. Preferable examples of solvent include, for example, water for injection, alcohol, propylene glycol, macrogol, sesame oil or corn oil. Preferable examples of dissolution adjuvant include, for example, polyethylene glycol, propylene glycol, D-mannitol, benzyl benzoate, ethanol, trisaminomethane, cholesterol, triethanolamine, sodium carbonate or sodium citrate. Preferable examples of suspending agent include:

surfactants such as stearyl triethanolamine, sodium lauryl sulfate, laurylamino propionate, lecitin, benzalkonium chloride, benzethonium chloride or glyceryl monostearate; and hydrophilic polymers such as polyvinyl alcohol, polyvinylpyrrolidone, sodium carboxymethylcellulose, methylcellulose, hydroxymethyl cellulose, hydroxyethyl cellulose, hydroxypropyl cellulose, etc. Preferable examples of isotonizing agent include, for example, sodium chloride, glycerine, D-mannitol, etc. Preferable examples of buffer include buffer solution of, for example, phosphate, acetate, carbonate, citrate, etc. Preferable examples of soothing agent include, for example, benzyl alcohol, etc. Preferable examples of preservative include, for example, p-hydroxybenzoic esters, chlorobutanol, benzyl alcohol, phenethyl alcohol, dehydroacetic acid and sorbic acid. Preferable examples of anti-oxidant include, for example, sulfite and ascorbic acid, etc.

The preparation method of the compounds represented by the formula (I) [including compounds represented by the formula (II) having a novel structure] or salts thereof will be described below.

The compounds represented by the formula (I) or salts thereof can be prepared by the method known per se. Alternatively, the compounds represented by the formula (I) or salts thereof can be prepared, for example, according to or substantially according to the method described below or in EP-A-487071, EP-A-560235, WO98/46590 and WO00/23437.

The compounds used in the following preparation methods may form salts similar to those of the compounds (I) as far as they do not have any adverse effect on the reactions. In addition, in the reactions described below, when starting compounds have an amino group, a carboxyl group or a hydroxyl group as a substituent group, a protecting group which is typically used in peptide chemistry may be introduced into these substituent groups, and the desired compound can be obtained by removing a protecting group after the reaction, if necessary.

As a protecting group for an amino group, for example, $C_{1-6}$alkylcarbonyl (e.g. acetyl, propionyl etc.), formyl, phenylcarbonyl, $C_{1-6}$alkyloxycarbonyl (e.g. methoxycarbonyl, ethoxycarbonyl, t-butoxycarbonyl etc.), phenyloxycarbonyl (e.g. benzoxycarbonyl etc.), $C_{7-10}$aralkyloxycarbonyl (e.g. benzyloxycarbonyl etc.) trityl and phthaloyl, which may have a substituent group, are used. As these substituent groups, halogen atom (e.g. fluorine, chlorine, bromine, iodine etc.), $C_{1-6}$alkylcarbonyl (e.g. acetyl, propionyl, butyryl etc.) and nitro group are used, and the number of substituent groups is around 1 to 3.

As a protecting group for a carboxyl group, for example, $C_{1-6}$alkyl (e.g. methyl, ethyl, propyl, isopropyl, butyl, tert-butyl etc.), phenyl, trityl, and silyl, which may have a substituent group, are used. As these substituent groups, halogen atom (e.g. fluorine, chlorine, bromine, iodine etc.), $C_{1-6}$alkylcarbonyl (e.g. acetyl, propionyl, butyryl etc.), formyl, and nitro group are used, and the number of substituent groups is around 1 to 3.

As a protecting group for a hydroxyl group, for example, $C_{1-6}$alkyl (e.g. methyl, ethyl, propyl, isopropyl, butyl, tert-butyl etc.), phenyl, $C_{7-10}$aralkyl (e.g. benzyl etc.), $C_{1-6}$alkylcarbonyl (e.g. acetyl, propionyl etc.), formyl, phenyloxycarbonyl, $C_{7-10}$aralkyloxycarbonyl (e.g. benzyloxycarbonyl etc.), pyranyl, furanyl, and silyl, which may have a substituent group, are used. As these substituent groups, halogen atom (e.g. fluorine, chlorine, bromine, iodine etc.), $C_{1-6}$alkyl,

phenyl, $C_{7-10}$aralkyl, and nitro group are used, and the number of substituent groups are around 1 to 4.

In addition, as a method of introducing and removing a protecting group, the method known per se or a similar method [for example, the method described in Protective Groups in Organic Chemistry, J.F.W.McOmie et al, Plenum Press] is used and, as a method for removal, methods treating with acid, base, reduction, ultra-violet, hydrazine, phenylhydrazine, sodium N-methyldithiocarbamate, tetrabutylammonium fluoride, or palladium acetate are used.

Method of preparation

When the compounds (I) of the present invention and compounds (raw material compounds or synthetic intermediates) for each step in the preparation of compounds (I) are free compounds, they can be converted into salts according to a conventional method and, when they form salts, they can be converted into free compounds or other salts according to a conventional method.

In addition, the compounds (I) of the present invention and respective raw material compounds or synthetic intermediates may be optical isomers, steric isomers, positional isomers or rotational isomers, or mixtures thereof, and these are included in compounds (I) of the present invention and raw material compounds or synthetic intermediates. For example, compounds (I) may be racemic compounds, or optical isomers resolved from racemic compounds. In addition, these can be isolated and purified by the separation method known per se.

Optical isomers can be prepared according to the means known per se. Specifically, optical isomers can be prepared by using optically active raw material compounds or synthetic intermediates, or by optically resolving racemic final compounds according to the conventional method. As an optical resolution method, the methods known per se, for example, a fractionation recrystallization method, an optically active column method, a diastereomer method and the like can be applied. Steric isomers, positional isomers and rotational isomers can be prepared by applying the methods known per se.

The following respective reactions can be performed without using a solvent, or by using a suitable solvent, if necessary. As the solvent, any solvents which can be generally used in a chemical reaction can be used as far as they do not inhibit a reaction and, for example, organic solvents such as hydrocarbon solvents (e.g. hexane, toluene etc.), ether solvent (e.g. ethyl ether, tetrahydrofuran, dioxane, dimethoxyethane), amide solvents (e.g. formamide, N, N-dimethylformamide, N,N-dimethylacetamide, hexamethylphosphoric triamide etc.), urea solvents (e.g. 1,3-dimethyl-2-imidazolidinone etc.), sulfoxide solvents (e.g. dimethyl sulfoxide etc.), alcohol solvents (e.g. methanol, ethanol, isopropanol, t-butanol etc.), nitrile solvents (e.g. acetonitrile, propionitrile etc.), pyridine and the like, and water and the like are used. An amount of the solvent to be used is usually about 0.5 ml to about 100ml, preferably about 3ml to about 30ml relative to 1 mmol of a compound. A reaction temperature is different depending on a kind of a solvent used, and is usually about -30°C to about 180°C, preferably about 0°C to about 120°C. A reaction time is different depending on a reaction temperature, and is usually about 0.5 hour to about 72 hours, preferably about 1 hour to about 24 hours. A reaction is carried out usually under a normal pressure and, if necessary, a reaction may be carried out under pressure at about 1 atm to about 100 atm.

A compound obtained in following each step is isolated and purified by the known means, for example, concentration, solution nature conversion, dissolution transference , solvent extraction, fractional distillation, distillation, crystallization, recrystallization, chromatography, fractional high performance liquid chromatography and the like, and is supplied as a raw material in the next reaction. Alternatively, the reaction mixture containing the compound may be used as a raw material without isolation or purification.

In the following explanation, a "condensation reaction" can be carried outin the presence of a base, if necessary. As the base, inorganic bases such as sodium carbonate, sodium bicarbonate, potassium carbonate, lithium carbonate, sodium hydroxide, potassium hydroxide, potassium hydride, sodium hydride, sodium methoxide, potassium t-butoxide and the like, and organic bases such as pyridine, lutidine, collidine, triethylamine and the like are used. An amount of the base to be used is usually an equivalent mole amount to an excessive amount, preferably about 1 mole equivalent to about 5 mole equivalent relative to a compound. Further, the present reaction may be promoted in the presence of a catalytic amount of an iodide compound, for example, sodium iodide, potassium iodide, or 4-dimethylaminopyridine and the like, if necessary.

Among compounds (I) of the present invention, the known compounds can be prepared by a synthetic method described below. Alternatively, those compounds can be prepared by the methods described in JP-A 6-166676, JP-A 11-310532, EP-A-487071, EP-A-560235, WO98/46590 and WO00/23437 or similar methods thereof.

On the other hand, novel compounds in the present invention, for example, compounds represented by the formula (II) or salts thereof can be prepared by a synthetic method described below.

1-1) Among compounds (II), compounds (IIa) wherein -X-is -O- or salts thereof can be prepared by the following reaction formula 1-1.

Reaction formula 1-1

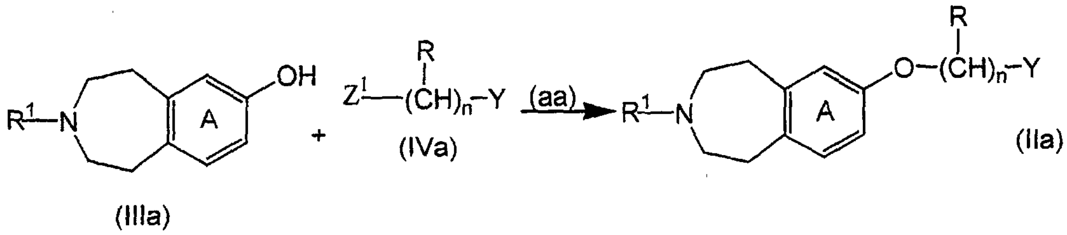


(IIIa) (IVa) (IIa)

In a step (aa), a compound (IIa) can be prepared by a condensation reaction between a compound represented by the formula (IIIa) [wherein each symbols denote the same meanings as those described above] (hereinafter, abbreviated as compound (IIIa) in some cases) and a compound represented by the formula (IVa)[wherein $Z^1$ denotes a leaving group, and other symbols denote the same meanings as those described above](hereinafter, abbreviated as compound (IVa) in some cases).

As the leaving group represented by $Z^1$, for example, a halogen atom (e.g. chloro, bromo, iodo etc.), a $C_{1-6}$alkylsulfonyloxy group (e.g. methanesulfonyloxy, ethanesulfonyloxy, trifluoromethanesulfonyloxy etc.), a $C_{6-10}$arylsulfonyloxy group (e.g. benzenesulfonyloxy, p-toluenesulfonyloxy etc.) and the like are used. In particular, for example, a halogen atom (e.g. bromo, iodo etc.) and the like are preferably used.

As a solvent for a condensation reaction between a compound (IIIa) and a compound (IVa), for example, alcohol solvents such as ethanol and the like, and nitrile solvents such as acetonitrile and the like are preferably used. A reaction temperature is different depending on a kind of a solvent used, and is preferably around about 0°C to about 120°C. A reaction time is different depending on a reaction temperature, and is preferably about 1 hour to about 24 hours. As the base, for example, sodium carbonate, potassium carbonate, triethylamine and the like are preferably used. An amount of the base to be used is preferably about 1 equivalent to about 3 equivalents relative to a compound (IVa). Further, the present reaction may be promoted in the presence of a catalytic amount to a compound (IVa) of an iodide compound (e.g. sodium iodide, potassium iodide etc.), or 4-dimethylaminopyridine or the like, if necessary. Specifically, for example, a reaction may be carried outin a solvent such as N,N-dimethylformamide and the like in the presence of potassium carbonate, sodium hydride or the like. An amount of the base to be used is preferably about 1 equivalent to about 3 equivalents relative to a compound (IVa).

A compound (IVa) can be prepared by the method known per se or a similar method thereof.

In addition, a raw material compound (IIIa) in a step (aa) or a salt thereof can be prepared, for example, according to the method described in WO00/23437.

1-2) Among compounds (II), compounds (IIb) wherein -X-is -$NR^{3a}$-or salts thereof can be prepared by the following reaction formula 2-1.

Reaction formula 2-1

Reaction formula 2-1

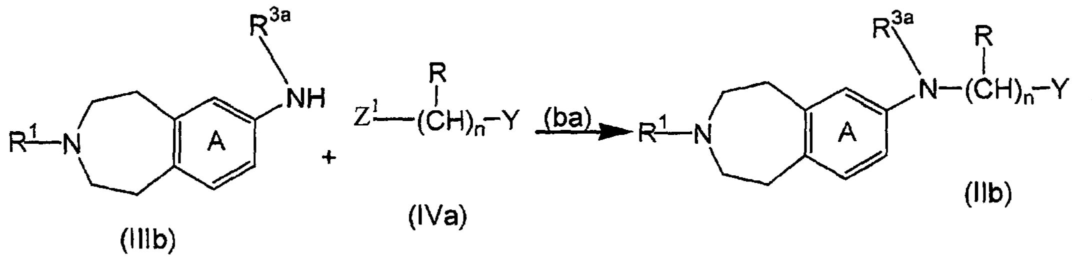


(IIIb) (IVa) (IIb)

In a step (ba), a compound (IIb) can be prepared by a condensation reaction between a compound represented by the formula (IIIb)[wherein each symbols denote the same meanings as those described above](hereinafter, abbreviated as compound (IIIb) in some cases) and a compound (IVa).

A condensation reaction between a compound (IIIb) and a compound (IVa) can be carried out, for example, in a solvent such as N,N-dimethylformamide and the like in the presence of potassium carbonate, sodium hydride or the like as a base. An amount of the base to be used is preferably about 1 equivalent to 3 equivalents relative to a compound (IVa).

In addition, a raw material (IIIb) in a step (ba) or a salt thereof can be prepared by the following reaction formula 2-2. That is, by successively carrying out:

a step (bb): a nitration reaction of a compound represented by the formula (Vb) [wherein each symbols denote the same meanings as those described above](hereinafter, abbreviated as compound (Vb) in some cases),

a step (bc): a reduction reaction of a compound represented by the formula (VIb) [wherein each symbols denote the same meanings as those described above](hereinafter, abbreviated as compound (VIb) in some cases), and

a step (bd): a condensation reaction of a compound represented by the formula (VIIIb)[wherein wach symbols denote the same meanings as those described above](hereinafter, abbreviated as compound (VIIIb) in some cases) and a compound represented by the formula (IXb)[wherein each symbols denote the same meanings as those described above](hereinafter, abbreviated as compound (IXb) in some cases), a compound (IIIb) can be prepared.

Reaction formula 2-2

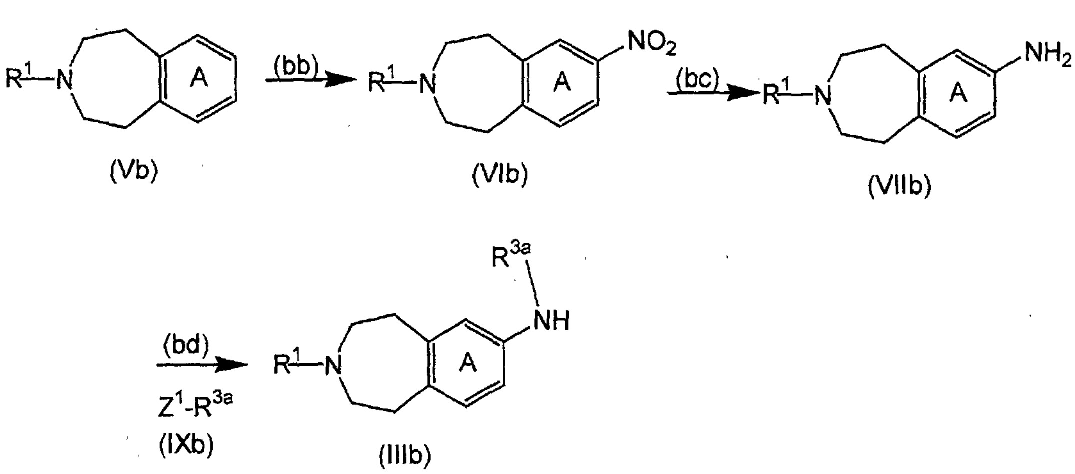


(Vb) (VIb) (VIIb) (IXb) (IIIb)

In a step (bb), a compound (VIb) can be prepared by nitrating a compound (Vb).

The present reaction can be carried out using a suitable nitrating reagent (e.g. nitric acid, nitric acid-sulfuric acid, nitronium trifluoroborate etc.) by the known method (method described in Synthesis, 217-238(1977), Chemistry of the Nitro and Nitroso Groups, p.1-48 Wiley (1970) etc.) or a similar method thereof.

A compound (Vb) can be prepared by the method known per se or a similar method thereof. For example, the compound (Vb) can be prepared by the methods described in J.Org.Chem, 34,2235(1969), J.Org.Chem., 54,5574 (1989), Tetrahedron Lett., 35,3023(1977), Bull.Chem.Soc.Jpn., 56,2300(1983), Indian, J.Chem., 2,211(1964), Indian. J.Chem., 12,247 1974, Bull.Chem.Soc.Jpn., 43,1824(1970), Chem.Pharm.Bull., 20,1328(1972), Chem.Pharm.Bull., 27,1982(1979), Helv.Chem.Acta,46,1696(1963), Synthesis, 541(1979), U.S.3,682,962, U.S. 3,911,126, Ger.Offen. 2,314,392, Ger.1,545,805, J.Chem.Soc., 1381(1949), Can.J.Chem., 42,2904(1964), J.Org.Chem., 28,3058(1963),J. Am.Chem.Soc., 76,3194(1954), 87,1397(1965), 88,4061(1966), JP-A 49-41539 and the like.

In a step (bc), a compound (VIIIb) can be prepared by a reduction reaction of a compound (VIb).

The present reaction can be carried outusing a suitable reduction reaction (e.g. a catalytic reduction reaction using a transition metal catalyst, a reduction reaction using a metal such as tin and the like in an acidic solvent etc.). Specifically, the reaction can be carried out by the known methods, for example, the methods described in Organic Syxthesis, Coll. Vol.5, 829-833(1973), Organic Synthesis, Coll. Vol.1, 456(1941), J. Am. Chem. Soc., 66, 1781(1944), or similar methods thereof.

In a step (bd), a compound (IIIb) can be prepared by a condensation reaction of a compound (VIIb) and a compound (IXb).

A condensation reaction of a compound (VIIb) and a compound (IXb) can be carried out, for example, in a same manner as that of the condensation reaction of a compound (IIIa) and a compound (IVa).

Further, a compound (IIIb) can be prepared using a compound (VIIb) as a raw material, for example, by a method such as reductive alkylation (e.g. the method desceibed in J. Am. Chem. Soc., 87, 2767(1965), Organic Synthesis, Coll. Vol.4, 283-285(1963) etc.) and a Michael addition reaction (e.g. the method described in Helv. Chem. Acta, 43, 1898(1960), J. Org. Chem., 39, 2044(1974), Synthesis, 5, 375(1981) etc.) or similar methods thereof.

1-3) Among compounds (II), compounds (IIc) wherein -X-is -$NR^{3a}CO$- or salts thereof can be prepared by the following reaction formula 3.

Reaction formula 3

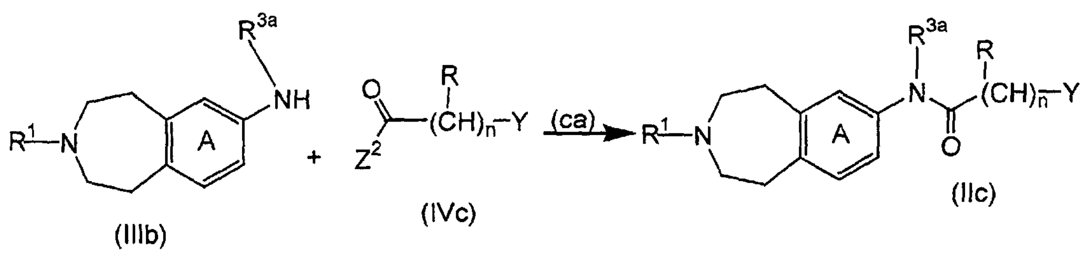


(IIIb) (IVc) (IIc)

In a step (ca), a compound (IIc) can be prepared by a amidation reaction of a compound (IIIb) and a compound represented by the formula (IVc)[wherein $Z^2$ denotes a leaving group, and other symbols denote the same meanings as those described above](hereinafter, abbreviated as compound (IVc) in some cases).

As the leaving group represented by $Z^2$, for example, a halogen atom (e.g. chloro, bromo, iodo etc.), a $C_{1-6}$alkyloxy group (e.g. methoxy, ethoxy, benzyloxy etc.), a $C_{6-10}$aryloxy group (e.g. phenoxy, p-nitrophenoxy etc.), a hydroxyl group and the like are used. In particular, a halogen atom (e.g. chloro etc.), a hydroxyl group and the like are preferably used.

An amidation reaction of a compound (IIIb) and a compound (IVc) can also be carried out using a suitable condensing agent or a base. For example, when $Z^2$ is a hydroxyl group, the present amidation reaction can be carried out by using a suitable condensing agent, for example, condensing agents which are conventionally used in the peptide chemistry, in particular, carbodiimides such as dicyclohexylcarbodiimide, 1-ethyl-3-(3-dimethylaminopropyl)carbodiimide and the like, phosphonic acids such as diphenylphosphorylazide, diethyl cyanophophonate and the like, phosgene equivalents such as 1-1'-carbonylbis-IH-imidazole and the like, and the like. An amount of the condensing agent to be used is usually about 1 equivalent to about 5 equivalents, preferably about 1 equivalent to about 1.5 equivalents relative to 1 mmol of a compound (IIIb).

In addition, for example, when $Z^2$ is a halogen atom, it is preferable to carry out a reaction using a suitable

base, for example, sodium carbonate, potassium carbonate, triethylamine and the like. An amount of the base to be used is usually about 1 equivalent to about 10 equivalents, preferably about 1 equivalent to about 2 equivalents relative to a compound (IIIb).

1-4) Among compound (II), compounds (IId) wherein -X-is -S-, -SO- or $-SO_2-$ or salts thereof can be prepared by the following reaction formula 4-1.

Reaction formula 4-1

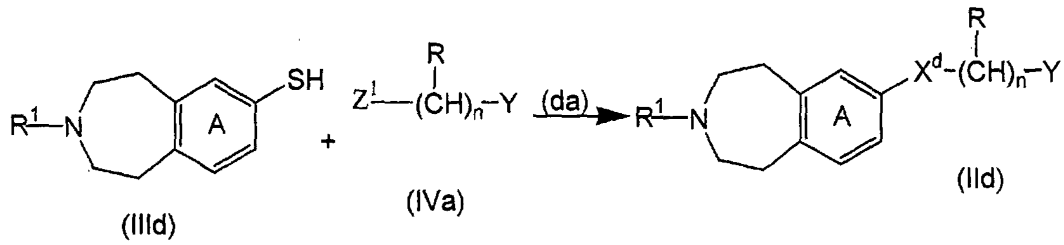


(IIId) (IVa) (IId)

In a step (da), a compound (IId) can be prepared by carrying out a condensation reaction of a compound (IIId) and a compound (IVa) and, if necessary, followed by carrying out an oxidation reaction [wherein $X^d$ denotes -S-, -SO- or $-SO_2-$, and other symbols denote the same meanings as those described above].

A condensation reaction of a compound (IIId) and a compound (IVa) can be carried out, for example, in a solvent such as N,N-dimethylformamide and the like in the presence of a base such as potassium carbonate, sodium hydride and the like. An amount of the base to be used is about 1 equivalent to about 3 equivalents relative to a compound (IVa).

A compound (IId) wherein $X^d$ is -S- can be derived into a compound (IId) wherein $X^d$ is -O- or $-SO_2-$ by carrying out an oxidation reaction, if necessary.

As an oxidizing agent, any oxidizing agents can be used as far as they are used as an oxidizing agent for sulfide and, preferably, for example, metachloroperbenzoic acid, peracetic acid, hydrogen peroxide, alkali metal periodate and the like are used. Particularly preferably, metachloroperbenzoic acid and hydrogen peroxide are used. An amount of the oxidizing agent to be used is particularly preferably about 1 equivalent to about 1.1 equivalents relative to a compound (IId) in the case of oxidation of S into SO. And the amount is particularly preferably about 2 to 2.5 equivalents relative to a compound (IVd) in the case of oxidation of S into $SO_2$. As a solvent for the present reaction, for example, dichloromethane, chloroform, acetic acid, ethyl acetate and the like are preferred.

A raw material compound (IIId) in a step (da) or a salt thereof can be prepared by the following reaction formula 4-2. That is, a compound (IIId) can be prepared by:

a step (db): a chlorosulfonylation reaction of a compound (Vb), and

a step (dc): a reduction reaction of a compound represented by the formula (VId)[wherein each symbols denote the same meanings as those described above](hereinafter, abbreviated as compound (VId)).

Reaction formula 4-2

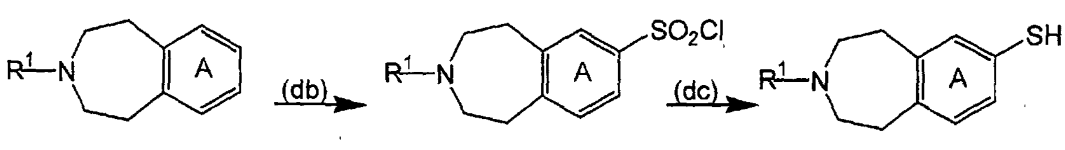


(Vb) (VId) (IIId)

In a step (db), a compound (VId) can be prepared by chlorosulfonylating a compound (Vb).

As an agent for the present chlorosulfonylation reaction, for example, chlorosulfonic acid, sulfuryl chloride, sulfur dioxide-copper chloride and the like can be used. In particular, chlorosulfonic acid is preferred. An amount of the chlorosulfonylating reagent to be used is about 1 equivalent to large excess. The present reaction can be carried out using a solvent or without a solvent. As a solvent used in the case where the reaction is carried out in a solvent, for

example, dichloromethane, 1,2-dichloroethane, carbon disulfide and the like are preferred. A reaction without a solvent is particularly preferred. As a reaction temperature, about -20°C to about 100°C is preferred.

In addition, a chlorosulfonyl group can be introduced into any position where a reaction can take place and, for example, when ring A is not substituted, a 7-position is mainly chlorosulfonylated. However, a compound in which a 6-position is chlorosulfonylated can be produced and separated.

In a step (dc), a compound (IIId) can be prepared by reducing a compound (VId).

The present reduction reaction can be carried out under a suitable reduction condition, for example, a combination of a metal and an acid such as zinc-acetic acid, tin-hydrochloric acid and the like, a catalytic reduction using a transition metal catalyst or a metal hydride such as lithium aluminium hydride and the like. Particularly preferable is a reduction reaction using zinc-acetic acid.

1-5) Among compounds (II), compounds (IIe) wherein -X-is $-SO_2NR^{3a}-$ or salts thereof can be prepared by the following reaction formula 5.

Reaction formula 5

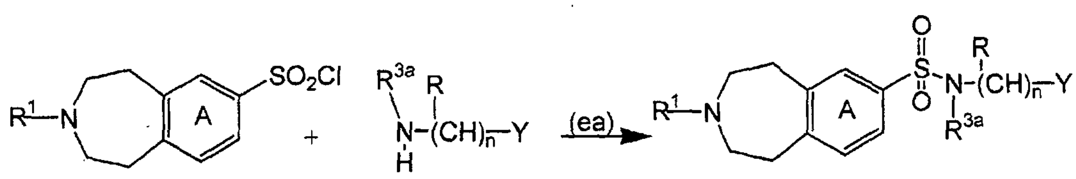


(VId) (IVe) (IIe)

In a step (ea), a compound (IIe) can be prepared by a condensation reaction of a compound (VId) and a compound represented by the formula (IVe) [wherein each symbols denote the same meanings as those described above](hereinafter, abbreviated as compound (IVe) in some cases).

A condensation reaction of a compound (VId) and a compound (IVe) can be carried out by the same manner as the amidation reaction of a compound (IIIb) and a compound (IVc).

A compound (IVe) or a salt thereof can be prepared by the method known per se or a similar method thereof. For example, it can be prepared by the methods described in J.Med. Chem., 33, 1880(1990) or similar methods thereof.

1-6) Among compounds (II), compounds (IIf) wherein -X-is $-SO_2NHCONR^{3a}-$ or salts thereof can be prepared by the following reaction formula 6.

Reaction formula 6

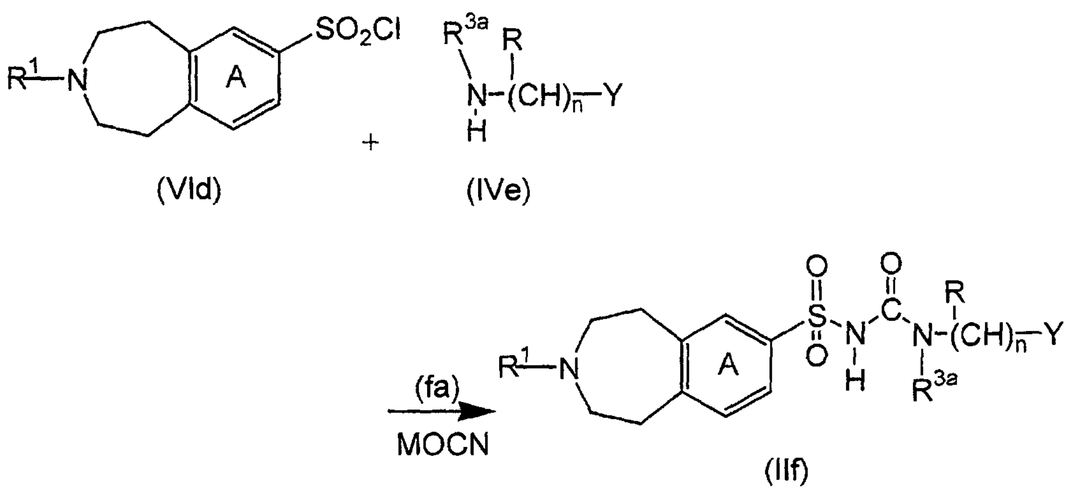


In a step (fa), a compound (IIf) can be prepared by acting an alkali metal isocyanate salt (MOCN; wherein M denotes an alkali metal) on a compound (VId) and followed by reacting a compound (IVe) therewith. The present reaction can be carried out by the methods described in EP-759431, JP-A 7-118267 and the like or similar methods thereof.

A reaction between a compound (VId) and an alkali metal isocyanate salt is carried out in the presence of a base, if needed. As a base to be used, pyridine, triethylamine and the like are particularly preferred. An amount of the base to be used is preferably about 1 equivalent to about 5 equivalents relative to a compound (VId). As a reaction solvent, in particular, acetonitrile and the like are preferably used. As an alkali metal, for example, potassium and the like are preferably used.

1-7) Among compounds (II), compounds (IIg) wherein -X-is $-SO_2NHC(=NH)NR^{3a}-$ or salts thereof can be prepared by the following reaction formula 7.

Reaction formula 7

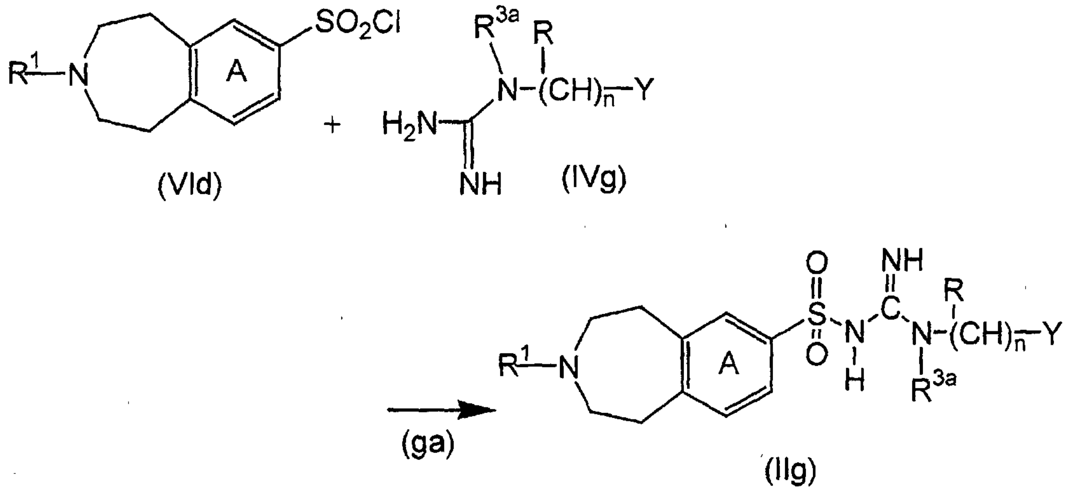

(VId) (IVg) (IIg)

In a step (ga), a compound (IIg) can be prepared by a condensation reaction of a compound (VId) and a compound represented by the formula (IVg) [wherein each symbols denote the same meanings as those described above](hereinafter, abbreviated as compound (IVg) in some cases).

A condensation reaction of a compound (VId) and a compound (IVg) can be carried out, for example, by the same manner as the amidation reaction of a compound (IIIb) and a compound (IVc).

A compound (IVg) can be prepared using a compound (IVe) by the method known per se or a similar method thereof. For example, a compound (IVg) can be prepared by a method of acting S-methylisothiourea on a compound (IVe)(e.g. the method described in J. Org. Chem., 13, 924 (1948) etc.), a method of acting cyanamide on a compound (IVe) (e.g. the method described in Helv. Chem. Acta, 29, 324(1946) etc.), and a method of acting 1,3-bis(t-butoxycarbonyl)-2-methyl-2-thiopseudourea on a compound (IVe) (e.g. the methods described in Tetrahedron Lett., 33, 6541-6542(1992), J. Org. Chem., 52, 1700-1703(1987) etc.) and the like.

1-8) Among compound (II), compounds (IIh) wherein -X-is $-CR^{3a}(R^{3b})-$ or a salts thereof can be prepared by the following reaction formula 8.

Reaction formula 8

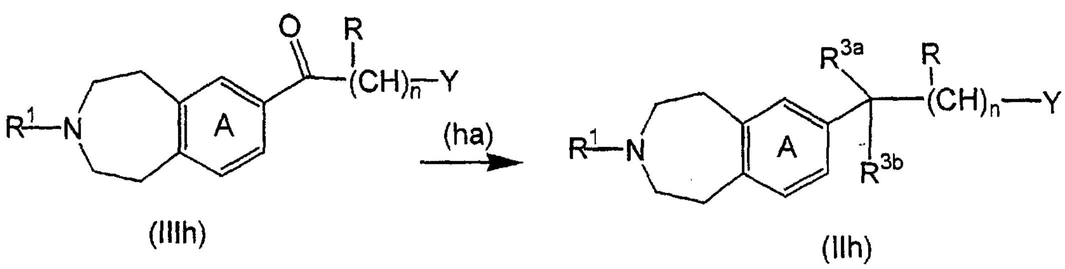

(IIIh) (IIh)

In a step (ha), a compound (IIh) can be prepared by reacting a compound represented by the formula (IIIh) [wherein each symbols denote the same meanings as those described above](hereinafter, abbreviated as compound (IIIh) in some cases) with a suitable reagent to convert a carbonyl group.

As a reagent used in a reaction of converting a carbonyl group, for example, reducing agents such as sodium borohydride, lithium aluminium hydride, triethylsilane and the like, organic metal reagents such as alkyllithium, alkylmagnesium halide and the like, and nucleophilic reactant such as hydrogen cyanide and the like are used.

Specifically, conversion of a carbonyl group into-CH(OH)- or $-CH_2-$ can be carried out, for example, using a reducing agent such as sodium borohydride, lithium aluminium hydride, triethylsilane and the like, under suitable reduction conditions (e.g. a combination of triethylsilane-trifluoroacetic acid, lithium aluminium hydride -aluminium chloride, zinc-hydrochloric acid and the like).

The present reaction can be carried out by the methods described in Reduction with Complex Metal Hydrides,

Interscience, New York(1956), Chem.Soc.Rev., 5,23(1976), Synthesis, 633(1974), J.Am.Chem.Soc. 91,2967(1969), J.Org. Chem., 29,121(1964), Org.Reactions, 1,155(1942), Angew.Chem., 71,726(1956), Synthesis,633(1974), J.Am. Chem.Soc., 80,2896(1958), Org.Reactions, 4,378(1948) and J.Am.Chem.Soc., 108,3385(1986) etc., or similar methods thereof.

In addition, conversion of a carbonyl group into-$CR^{3c}(OH)$- (wherein $R^{3c}$ denotes a $C_{1-6}$alkyl group) can be-carried out, for example, using an organic metal reagent such as alkyllithium, alkylmagnesium halide and the like by the methods described, for example, in Grignard Reactions of Nonmetallic Substances, Prentice-Hall: Englewood Cliffs, NJ, 1954, pp.138-528, Organolithium Methods, Academic Press: New York, 1988, pp.67-75 and the like or similar methods thereof.

In addition, conversion of a carbonyl group can be carried out by the method described in Advanced Organic Chemistry, 5th ed. Wiley-Interscience: New York, 1992, pp.879-981 and the like or similar methods thereof.

A compound (IIIh) can be prepared by the method known per se or a similar method thereof, for example, the method described in JP-A 5-14o149, JP-A 6-206875, J.Med.Chem. 37,2292(1994) and the like or similar methods thereof.

1-9) Among compounds (II), compound (IIi) wherein -X-is -$C(=CR^{3a}(R^{3b}))$- or salts thereof can be prepared by the following reaction formula 9.

Reaction formula 9

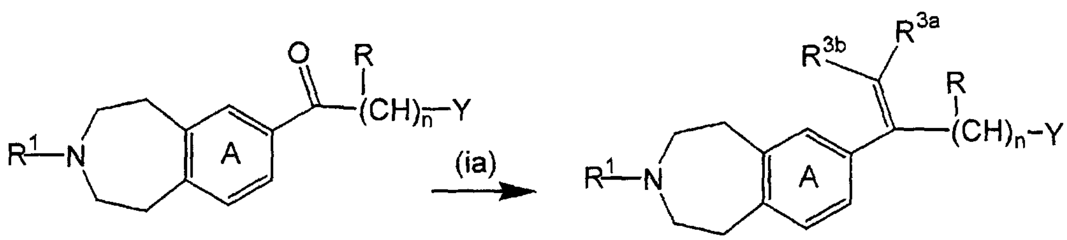


(IIIh) (IIi)

In a step of (ia), a compound (IIi) can be prepared by reacting a compound (IIIh) with a suitable reagent to convert a carbonyl group.

Examples of a conversion reaction of a carbonyl group include the Wittig reaction, the Horner-Wadsworth-Emmons reaction, the Peterson olefinization reaction, the Knoevenagel reaction and the like and, as a reagent, general reagents used for those reactions are used.

The present reaction can be carried out by the methods described, for example, in Advanced Organic Chemistry, 5th ed. Wiley-Interscience: New York, 1992, pp. 879-981, Organic Synthesis, coll. vol.5, 751(1973), Organic Synthesis, coll. vol.5, 509(1973), Synthesis, 384(1984), Org. Reactions, 15, 204(1967) and the like, or similar methods thereof.

1-10) Among compounds (II), compounds (IIj) wherein-X- is $-C(=NR^{3a})-$ or salts thereof can be prepared by the following reaction formula 10.

Reaction formula 10

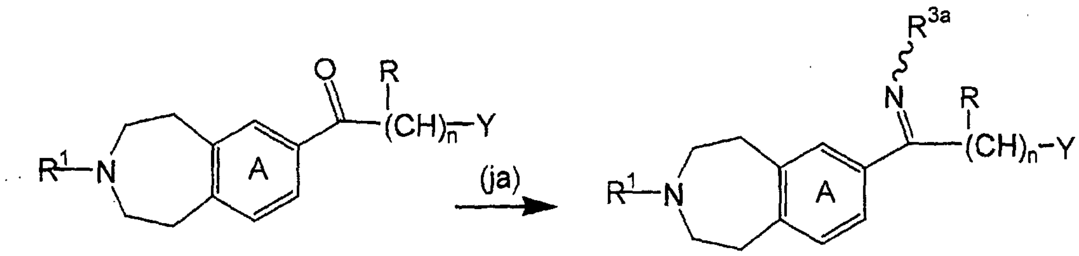


(IIIh)

(IIj)

In a step (ja), a compound (IIj) can be prepared by reacting a compound (IIIh) with a suitable reagent to convert a carbonyl group.

Examples of a reagent used for a conversion reaction of a carbonyl group include, for example, optionally substituted hydrazine and optionally substituted hydroxylamine. As the substituent group, a $C_{1-6}$alkyl group and the like are used.

The present reaction can be carried out by the methods described, for example, in Advanced Organic Chemistry, 5th ed. Wiley-Interscience: New York, 1992, pp. 904-907, Organic Functional Group Preparations, vol. III, Academic(1983), Rodd's Chemistry of Carbon Compounds, vol. 1, part C, Elsevier Publishing CO. (1965) and the like, or similar methods thereof.

1-11) Among compounds (II), compounds (IIk) wherein-X- is -CS- or salts thereof can be prepared by the following reaction formula 11.

Reaction formula 11

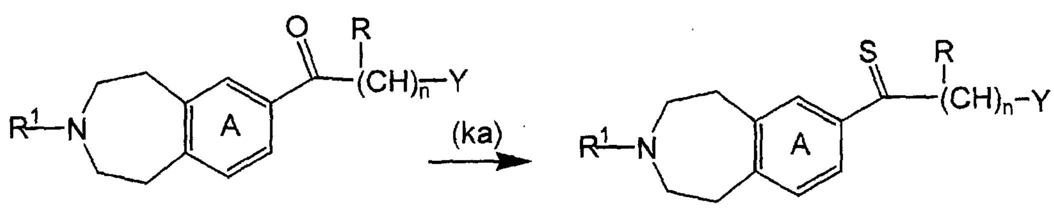


(IIIh)

(IIk)

In a step (ka), a compound (IIk) can be prepared by reacting a compound (IIIh) with a suitable reagent to convert a carbonyl group into a thiocarbonyl group.

Examples of a reagent used for converting a carbonyl group into a thiocarbonyl group include, for example, sulfurizing reagents such as Lawesson reagent, phosphorus pentasulfide, hydrogen sulfide-hydrochloric acid and the like.

The present reaction can be carried out by the methods described , for example, in Synthesis, 7, 543(1991), J. Am. Chem. Soc., 106, 934(1984), J. Am. Chem. Soc., 68, 769(1946) and the like, or similar methods thereof.

1-12) Among compounds (II), compounds (IIm) wherein-X- is $-CONR^{3a}-$ or salts thereof can be prepared by the following 12-1.

Reaction formula 12-1

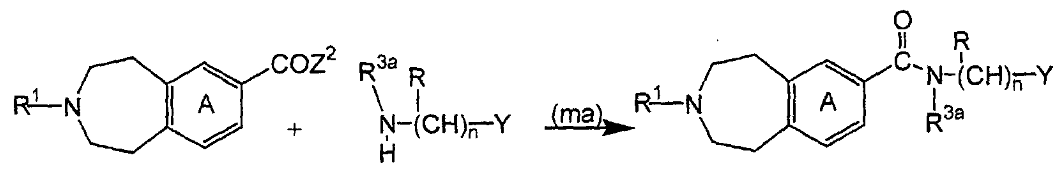


(IIIm) (IVe) (IIm)

In a step (ma), a compound (IIm) can be prepared by a condensation reaction of a compound represented by the formula (IIIm)[wherein each symbols denote the same meanings as those described above](hereinafter, abbreviated as compound (IIIm) in some cases) and a compound (IVe).
A reaction between a compound (IIIm) and a compound (IVe) can be carried out, for example, by the same manner as the amidation reaction of a compound (IIIb) and a compound (IVc).
In addition, a raw material compound (IIIm) for a step (ma) can be prepared by the following reaction formula 12-2. That is, a compound (IIIm) can be prepared by carrying out successively a step (mb): a acetylation of a compound (Vb), and a step (mc): a oxidation of a compound represented by the formula (VIm)[wherein each symbols denote the same meanings as those described above](hereinafter, abbreviated as compound (VIm) in some cases) and, if necessary, followed by conversion of a functional group.

Reaction formula 12-2

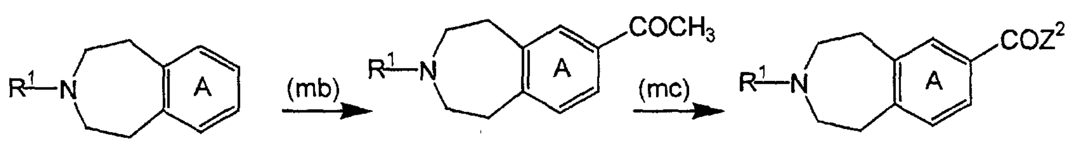


(Vb) (VIm) (IIIm)

In a step (mb), a compound (VIm) can be prepared by acetylating a compound (Vb).
The present reaction can be carried out under the general conditions for Friedel-Crafts reaction. As a reagent for acetylation, acetyl chloride, acetic anhydride and the like are used. Specifically, the compound can be prepared by the methods described, for example, in JP-A 5-140149, JP-A 6-206875, J. Med. Chem., 37, 2292(1994) and the like, or similar methods thereof.
In a step (mc), a compound (IIIm), in particular, a compound wherein $Z^2$ is a hydroxyl group can be prepared by oxidizing a compound (VIm).
Examples of an oxidizing agent used in the present reaction include, for example, hypochlorite, hypobromite, and halogen (e.g. bromine, iodine etc.) in the presence of a suitable base (e.g. sodium hydroxide etc.). Specifically, the present reaction can be carried out by the methods described, for example, in Org. Synthesis, Coll. Vol. 2, 428 (1943), J. Am. Chem. Soc., 66, 894(1944) and the like, or similar methods thereof.
In addition, if necessary, by converting a functional group of a hydroxyl group of a compound (IIIm) wherein $Z^2$ is a hydroxyl group, the compound can be converted into a compound (IIIm) wherein $Z^2$ is a halogen atom (e.g. chloro, bromo, iodo etc.), a $C_{1-6}$alkyloxy group (e.g. methoxy, ethoxy, benzyloxy etc.) or a $C_{6-10}$aryloxy group (e.g. phenoxy, p-nitrophenoxy etc.).
A method for conversion of a functional group can be carried out by the methods described, for example, in Advanced Organic Chemistry, 5th ed., Wiley-Interscience: New York, 1992, pp. 393-396, 437-438, Comprehensive Organic Transformations, VCH Publishers Inc. (1989) and the like, or similar methods thereof.
The thus obtained compound (II) can be isolated and purified by the known separation and purification means such as concentration, concentration under reduced pressure, solvent extraction, crystallization, recrystallization, dissolution transference, chromatography and the like.

Since compounds having GPR 14 antagonistic activity or salts thereof according to the present invention [including compounds represented by formula (I) and (II) or salts thereof] have a potent GPR 14 antagonistic activity, those can be used as therapeutic agents for expressing various vasoactivities (e.g. facilitation or inhibition of vasoconstriction), and preferably as vasoconstriction inhibitors.

Compounds having GPR 14 antagonistic activity or salts thereof according to the present invention [including compounds represented by formula (I) and (II) or salts thereof] can be used as a prophylactic and therapeutic agent for various diseases (e.g., cardiovascular diseases), more preferably as a prophylactic and therapeutic agent of hypertension, arteriosclerosis, hypertension, cardiomegaly, myocardial infarction, heart failure or septic shock, and particularly preferably as a prophylactic and therapeutic agent of ischemic myocardial infarction or congestive heart failure.

Further, compounds having GPR 14 antagonistic activity or salts thereof according to the present invention [including compounds represented by formula (I) and (II) or salts thereof] have very low toxicity and thus can be used safely.

Daily dose of compounds having GPR 14 antagonistic activity or salts thereof according to the present invention may be varied depending on various factors such as the condition and weight of the patient to be treated and administration manner. For oral administration, active ingredient (e.g., compound represented by formula (II) or salt thereof) can be administered to an adult (50kg) in an amount of approximately 0.1 to 100mg, preferably about 1 to 50mg, more preferably about 1 to 20mg in one portion, and may be administered in one to three divided portions a day.

Compounds having GPR 14 antagonistic activity or salts thereof according to the present invention [including compounds represented by formula (I) and (II) or salts thereof] may be used in combination with other therapeutic agent or agents (particularly with a prophylactic and therapeutic agent of hypertension). In this case, these agents may separately be formulated into different preparations, or may be formulated together into one preparation, by blending with any pharmaceutically acceptable carrier, excipient, binder and/or diluent, and administered orally or parenterally. When these agents are separately formulated into different preparations, these preparations may be administered to a subject after mixing together by using diluent just prior to use. Alternatively, these preparations may separately be administered to the subject simultaneously or with a certain time interval. A kit product for mixing separate preparations using diluent and the like just prior to use for administration (e.g., a kit for injection which contains two or more ampoules each containing a different powdery drug and a diluent for mixing the drugs just prior to use) as well as a kit product for administering separate preparations to a subject simultaneously or separately with a certain time interval (e.g., a kit for administering two or more types of separate tablets to a subject simultaneously or separately with a certain time interval wherein tablets each containing a different drug are packed in the same bag or different bags, and a column is provided on the bag in which a time interval for drug administration can be written) are encompassed by the pharmaceutical compositions of the present invention.

Particular examples of other therapeutic agents which can be used in combination with compounds having GPR 14 antagonistic activity or salts thereof according to the present invention include:

antihypertensive drugs: diuretic [e.g., furosemide (Lasix), bumetanide (Lunetoron) and azosemide (Diart) ], hypotensive drug [e.g., ACE inhibitor (enalapril maleate (Renivace), delapril hydrochloride) and Ca antagonist (manidipine, amlodipine), and $\alpha$- or $\beta$-receptor blocker];

therapeutic drugs of chronic heart failure: cardiotonic [e.g., cardiotonic glycoside (e.g., digoxin), $\beta$-receptor stimulant (catecholamine preparation such as denopamine and dobutamine) and PDE inhibitor], diuretic [e.g., furosemide (Lasix), spironolactone (Aldactone) ], ACE inhibitor [e.g., enalapril maleate (Renivace)], Ca antagonist [e.g., amlodipine] and $\beta$-receptor blocker;

antiarrhythmic: disopyramide, lidocaine, quinidine sulfate, flecainide acetate, mexiletine hydrochloride, amiodarone hydrochloride, as well as $\beta$-blocker, Ca antagonist;

prophylactic and therapeutic drugs of thrombogenesis: coagulation inhibitor [e.g., heparin sodium, heparin calcium, warfarin calcium (warfarin), blood coagulation factor Xa inhibitor and drugs capable of balancing coagulation fibrinolytic system], thrombolytic agent [e.g., tPA, urokinase, prourokinase, etc.], antiplatelet drug [e.g., aspirin, sulfinpyrazolo (Anturan), dipyridamole (Persantin), ticlopidine (Panaldine), cilostazol (Pletaal) and GP IIb/IIIa antagonist (ReoPro)];

coronary vasodilators: nifedipine, diltiazem, nicorandil or nitrite agent; and

protective drugs for cardiac muscle: opener for cardiac ATP-K, Na-H exchange inhibitor, endothelin antagonist and urotensin antagonist.

Although the present invention will be described in more detail by referring to Experimental Examples, Preparation Examples, Reference Example and Synthesis Examples, these examples are provided to illustrate the invention but not to limit its scope.

Brief description of SEQ ID NOS used herein will be provided below:

[SEQ ID NO: 1]

A synthetic DNA used for screening cDNA encoding human GPR14 protein.

[SEQ ID NO: 2]

Another synthetic DNA used for screening cDNA encoding human GPR14 protein.

[SEQ ID NO: 3]

The entire nucleotide sequence of cDNA encoding human GPR14 protein with nucleotide sequences which may be recognized by restriction enzymes Sal I and Spe I added at the 5'- and 3'-termini, respectively.

[SEQ ID NO: 4]

The amino acid sequence of human GPR14 protein confirmed in Reference Example 2.

Reference Example 1: Amplifying cDNA for human GPR14 receptor by PCR method using human skeletal muscle-derived cDNA

PCR amplification was performed by using cDNA derived from human skeletal muscle (Clontech) as a template and two synthetic DNA primers (SEQ ID NOS: 1 and 2). The synthetic DNA primers were designed so that the gene in the region which is to be translated into receptor protein would be amplified, and such that nucleotide sequences which may be recognized by restriction enzymes Sal I and Spe I were added at the 5'- and 3'-termini of the gene, respectively. Reaction solution included 2.5μl of cDNA template, synthetic DNA primers (0.2μM each), 0.2mM dNTPs, 1μl of Advantage 2 polymerase mix (Clontech) and the buffer appended to the enzyme (total reaction volume of 50μl). Thermocycler (Perkin-Elmer Corp.) was used for amplification. The amplification cycle consisted of heating at 95°C for 60 seconds, followed by 5 rounds of 95°C for 30 seconds and 72°C for 3 minutes, 5 rounds of 95°C for 30 seconds and 70°C for 3 minutes, and then 20 rounds of 95°C for 30 seconds and 68°C for 3 minutes, and finally heating at 68°C for 3 minutes. The resultant PCR amplification products were confirmed by purification by electrophoresis on a 0.8% agarose gel followed by staining with ethidium bromide.

Reference Example 2: Subcloning of PCR product into plasmid vector and confirming amplified cDNA by reading the nucleotide sequence of cDNA insert

PCR reaction products obtained in Reference Example 1 were separated on a 0.8% low-melting agarose gel, a gel containing bands was excised using a razor, and DNA was collected using GENECLEAN SPIN (BIO 101, Inc.). According to the prescription included in Eukaryotic TOPO™ TA Cloning kit (Invitrogen), the collected DNA was cloned into a plasmid vector for expression in animal cells, pcDNA3.1/V5/His, to construct a plasmid for protein expression, pcDNA3.1-hGPR14 which was then introduced into *Escherichia coli* DH5α competent cells (Toyobo Co., Ltd.) for transformation. Then, clone which contained cDNA insert fragment was selected on an ampicillin-containing LB agar medium, and separated using a sterilized toothpick to obtain transformant *E. coli* DH5α/pcDNA3.1-hGPR14. Each clone was cultured overnight on an ampicillin-containing LB medium, and Quiawell 8 Ultra Plasmid kit (Qiagen) was used to prepare plasmid DNA. Portion of DNA prepared was digested with restriction enzyme Sal I, and the size and direction of receptor cDNA fragment inserted were determined. The sequences of nucleotides were determined by using DyeDeoxy Terminator Cycle Sequence Kit (Perkin-Elmer Corp.) and then reading in a fluorescence automatic sequencer. The sequence of clone obtained was analyzed and confirmed to be consistent with a genetic sequence comprising the sequence of human GPR14 gene, of which entire sequence has been reported (EP 0 859 052 A1), and Sal I and Spe I recognition sequences added to the 5'- and 3'-termini of the sequence, respectively (SEQ ID NOS: 3 and 4). It should be noted that although the 1133rd base in the sequence of human GPR14 gene (SEQ ID NO: 3) was identified as C in the report (EP 0 859 052 A1) while it was identified as G in the present Example though the amino acids which would be translated from these sequences may be the same.

Reference Example 3: Preparing human GPR14-expressing CHO cell

After the transformant *E. coli* DH5α/pcDNA3.1-hGPR14 prepared in Reference Example 2 was cultured, plasmid DNA for pcDNA3.1-hGPR14 was prepared by using Plasmid Midi Kit (Qiagen). The plasmid DNA was introduced into CHO dhfr$^-$ cells using CellPhect Transfection Kit (Amersham Pharmacia Biotech) according to the protocol ap-

pended thereto. 10μg of DNA was co-precipitated with calcium phosphate to prepare a suspension which was then added to a 10cm petri dish on which 5 x $10^5$ or 1 x $10^6$ CHO dhfr⁻ cells had previously been inoculated 24 hours before then. Cells were cultured in a MEMα medium containing 10% fetal bovine serum for one day, subcultured, and cultured in a selection medium, a MEMα medium containing 0.4 mg/ml G418 (GIBCO BRL) and 10% dialysis fetal bovine serum. Colonies of transformed cells (CHO/hGPR14), which were human GPR14-expessing CHO cells growing in the selection medium, were selected.

Experimental Example 1: Preparing human GPR14-expressing cell fraction

To 1 x $10^8$ CHO/GPR14 cells were added 10 ml of homogenate buffer (10 mM $NaHCO_3$, 5 mM EDTA, 0.5 mM PMSF, lμg/ml pepstatin, 4μg/ml E64, 20μg/ml leupeptin), and disrupted using Polytron (12,000 rpm, 1 minute). Cell debris solution was centrifuged at 1,000g for 15 minutes to obtain a supernatant. The supernatant was then ultrasonicated (in Beckman type 30 rotor, 30,000 rpm, 1 hour), and the resultant precipitant was collected as human GPR14-expressing CHO cell fraction.

Experimental Example 2: Preparing isotope-labeled human urotensin II

Isotope-labeled human urotensin II to be used in experiments for testing inhibition of binding was prepared as described below. 5 μg of human urotensin II (available from Peptide Institute, Inc.) was dissolved in 25μl of 0.4M sodium acetate (pH 5.6). To the solution was added 200ng of lactoperoxidase (Wako Pure Chemical Industries, Ltd.) followed by 1 mCi [$^{125}$I]-sodium iodide (Amersham Pharmacia Biotech) and 200 ng of hydrogen peroxide (10μl). The solution was left to stand at room temperature for 10 minutes, another 200 ng of hydrogen peroxide (10μl) was added thereto and then the solution was left to stand for 10 minutes. The mixture was then purified by HPLC using TSKgel ODS-$SOT_s$ column (4.6 mm x 25 cm, Toso Co., Ltd.) to obtain [$^{125}$I]-labeled human urotensin II.

Experimental Example 3: Experiment for testing the ability of test compound to inhibit binding of urotensin II to GPR14 using human GPR14-expressing cell fraction and isotope-labeled urotensin II

Human GPR14-expressing CHO cell fraction was diluted in a membrane diluting buffer (20mM phosphate buffer (pH7.3), 150mM NaCl, 5mM $MgCl_2$, 0.1% BSA, 0.05% CHAPS, 0.5mM PMSF, 0.1μg/ml Pepstatin, 20μg/ml Leupeptin, 4μg/ml E-64) to prepare a solution of cell membrane fraction (protein concentration:3μg/ml) for assay. The membrane fraction solution for assay was dispensed in 96-well microplates (85μl each) which were left for stand for reaction at 25°C for 3 hours after adding: 10μl of membrane diluting buffer containing 1nM [$^{125}$I]-labeled human urotensin II and 5μl of di-methylsulfoxide diluted 5-times (by volume) in membrane diluting buffer for examining the total binding; 10μl of membrane diluting buffer containing 1nM [$^{125}$I]-labeled human urotensin II and 5μl of 20% dimethylsulfoxide-containing membrane diluting buffer containing 20μM human urotensin II without isotope-labeling for examining non-specific binding; and 5μl of a solution of test compound in di-methylsulfoxide diluted 5-times (by volume) in membrane diluting buffer and 10μl of membrane diluting solution containing 1nM [$^{125}$I]-labeled-human urotensin II for testing the ability of test compounds to inhibit binding. The mixture solution was filtrated through a filter plate (GF/C, Watman). Next, the filter was washed three times with membrane diluting buffer (0.2ml), added with 20μl of Microscinti 20 (Packard), and determined for radioactivity in Topcount (Packard). Specific-binding is calculated by subtracting non-specific binding from the total binding. The ability of test compound to inhibit binding of urotensin II to human GPR14 is represented by the ratio of [(total binding) - (the radio activity of the cell fraction to which test compound was added)] vs [specific binding]. Concentrations of test compounds at which the compounds showed 50% inhibition of human GPR14 binding activity are shown.

Results are shown in Table 1 below.

[Table 1]

| Test compound | Inhibitory concentration |
|---|---|
| compound of example 6 | 3.2nM |
| compound of example 75 | 8.6nM |
| compound of example 84 | 1.7nM |

Experimental Example 4: Change in calcium concentration in human GPR14-expressing CHO cell caused by test compound

GPR14-expressing CHO cells were inoculated on a 96-well plate at $1\times10^4$ cell/well, cultured for 48 hours, and then washed with 0.1ml of HBSS containing 20mM HEPES(pH7.4), 1% FCS and 1% penicillin-streptomycin (hereinafter referred to as "wash buffer"). Next, 100μl of another wash buffer containing 4μM Fluo3, 0.04% pluronic acid and 2.5mM probenicid (hereinafter referred to as "reaction buffer") was added thereto for reaction at 37°C for 1 hour. The reaction buffer was then removed and the plate was washed three times with 0.2ml of wash buffer. Then, 90μl of wash buffer and 10μl of a solution of test compound in dimethylsulfoxide diluted 10 times (by volume) in membrane diluting buffer were added for agonist activity assay, while, for antagonist activity assay, furthermore 10μl of 10nM urotensin II was additionally added to determine change in intracellular calcium concentration in FLIPR (Japan Molecular Device). The test compound (compound described in Example 12 of JP-A 6-166676) inhibited urotensin II-induced increase in intracellular calcium concentration.

Synthesis Example

In the following Examples, HPLC was measured under the following condition A or B.

Measuring apparatus: Shimazuseisakusho LC-10Avp System

Condition A

Column: CAPCELL PAK C18UG120, S-3 μm, 2.0×50mm
Solvent: A solution; 0.1% trifluoroacetic acid-containing water, B solution; 0.1% trifluoroacetic acid-containing acetonitrile
Gradient cycle: 0.00min. (A solution/B solution=90/10), 4.00min. (A solution/B solution=5/95), 5.50min. (A solution/B solution=5/95), 5.51min. (A solution/B solution=90/10), 8.00min. (A solution/B solution=90/10)
Injection amount: 2 μl, flow rate: 0.5ml/min., detection method: UV 220nm

Condition B

Column: CAPCELL PAK C18UG120, S-3 μm, 2.0 × 50mm
Solvent: A solution; 0.1% trifluoroacetic acid-containing water, B solution; 0.1% trifluoroacetic acid-containing acetonitrile
Gradient cycle: 0.00min. (A solution/B solution=100/0), 4.00min. (A solution/B solution=60/40), 5.50min. (A solution/B solution=60/40), 5.51min. (A solution/B solution=90/10), 8.00min. (A solution/B solution=90/10)
Injection amount: 2μl, flow rate: 0.5ml/min., detection method: UV 220nm

In the following Examples, mass spectrum (MS) was measured under the following conditions.
Measuring apparatus: Micromass Platform II
Ionization method: Atmospheric Pressure Chemical Ionization (APCI) or Electron Spray Ionization (ESI)
In the following Examples, purification by preparative HPLC was carried out under the following conditions.
Apparatus: Gilson High Throughput Purification System Column: YMC CombiPrep ODS-A, S-5μm, 50×20mm
Solvent: A solution; 0.1%trifluoroacetic acid-containing water, B solution; 0.1%trifluoroacetic acid-containing acetonitrile
Gradient cycle: 0.00min. (A solution/B solution=90/10), 1.00min. (A solution/ B solution=90/10), 4.20min. (A solution/B solution=10/90), 5.40min. (A solution/B solution=10/90), 5.50min. (A solution/B solution=90/10), 5.60min. (A solution/B solution=90/10)
Flow rate: 25ml/min., detection method: UV 220nm

Example 1

4-(4-phenyl-1-piperazinyl)-1(2,3,4,5-tetrahydro-1H-3-benzazepin-7-yl)-1-butanone trihydrochloride

1) 2,2,2-trifluoro-1-(1,2,4,5-tetrahydro-3H-3-benzazepin-3-yl)-1-ethanone

Trifluoroacetic anhydride (31g) was added to a solution of 2,3,4,5-tetrahydro-1H-3-benzazepine (15g) and triethylamine (51ml) in tetrahydrofuran (THF; 100ml) under ice-cooling. The reaction mixture was stirred at room temperature for 15 hours, IN hydrochloric acid was added to stop the reaction, and the reaction mixture was extracted with ethyl acetate. The extract was washed with water, dried over anhydrous magnesium sulfate, and the solvent was evaporated under reduced pressure. The residue was purified by silica gel column chromatography (n-hexane/ethyl acetate=4/1) to obtain the title compound (25g) .

$^1$H-NMR ($CDCl_3$) δ: 2.95-3.05(4H,m),3.65-3.85(4H,m),7.10-7.30(4H,m)

2) 4-bromo-1-[3-(2,2,2-trifluoroacetyl)-2,3,4,5-tetrahydro-1H-3-benzazepin-7-yl]-1-butanone

4-bromobutyryl chloride (4.8ml) and aluminium chloride (8.2g) were added to a solution of 2,2,2-trifluoro-1-(1,2,4,5-tetrahydro-3H-3-benazepin-3-yl)-1-ethanone (10g) in dichloromethane (70ml), and the mixture was stirred at room temperature for 3 hours. The reaction solution was poured in ice-water, and the solution was extracted with dichloromethane. The extract was washed with brine, dried over anhydrous magnesium sulfate, and the solvent was evaporated under reduced pressure. The residue was purified by silica gel column chromatography (n-hexane/ethyl acetate=4/1) to give the title compound (5.9g). $^1$H-NMR ($CDCl_3$) δ: 2.20-2.40(2H,m), 2.95-3.10 (4H,m), 3.17(2H,t,J=7.0 Hz), 3.56(2H,t,J=6.4 Hz), 3.65-3.85(4H,m), 7.20-7.30(1H,m), 7.75-7.85(2H,m)

3) 4-(4-phenyl-1-piperazinyl)-1-[3-(2,2,2-trifluoroacetyl)-2,3,4,5-tetrahydro-lH-3-benzazepin-7-yl]-1-butanon

A mixture of 4-bromo-1-[3-(2,2,2-trifluoroacetyl)-2,3,4,5-tetrahydro-1H-3-benzazepin-7-yl]-1-butanone (100mg), 1-phenylpiperazine (0.043ml), potassium carbonate (35mg) and N,N-dimethylformamide (DMF; 3ml) was stirred at 80°C for 2 hours. The reaction solution was diluted with water, and extracted with ethyl acetate. The extract was washed with brine, dried over anhydrous magnesium sulfate, and the solvent was evaporated under reduced pressure. The residue was purified by silica gel column chromatography (n-hexane/ethyl acetate=1/3) to give the title compound (72mg). $^1$H-NMR ($CDCl_3$) δ: 1.91-2.05(2H,m), 2.47(2H,t,J=6.8Hz), 2.55-2.65(4H,m), 2.95-3.05(6H,m), 3.10-3.20(4H,m), 3.60-3.80(4H,m), 6.80-6.95(3H,m), 7.20-7.30(3H,m), 7.75-7.85(2H,m)

MS(APCI+)= 474(M+H)

4) 4-(4-phenyl-1-piperazinyl)-1-(2,3,4,5-tetrahydro-1H-3-benzazepin-7-yl)-1-butanone trihydrochloride

A 1M aqueous potassium carbonate solution (0.24ml) was added to a solution of 4-(4-phenyl-1-piperazinyl)-1-[3-(2,2,2-trifluoroacetyl)-2,3,4,5-tetrahydro-1H-3-benzazepin-7-yl]-1-butanone (58mg) in methanol (1ml), and the mixture was stirred at room temperature for 1.5 hours. The methanol was evaporated under reduced pressure, followed by extraction with ethyl acetate. The extract was washed with brine, dried over anhydrous magnesium sulfate, and the solvent was evaporated under reduced pressure to give 4-(4-phenyl-1-piperadinyl)-1-(2,3,4,5-tetrahydro-lH-3-benzazepin-7-yl)-1-butanone. This was treated with a IN hydrogen chloride solution in ethyl acetate to give an objective compound (22mg). $^1$H-NMR (DMSO-$d_6$) δ: 2.00-2.20(2H,m), 3.10-3.40(16H,m), 3.50-3.65(2H, m), 3.70-3.90(2H,m), 6.87(1H,t,J=8.0Hz), 7.00(2H,d,J=8.0Hz), 7.27(2H,t,J=8.0Hz), 7.38(1H,d,J=8.4Hz), 7.80-7.85(2H,m)

MS(APCI+): 378(M+H) The following compounds were prepared as in Example 1.

Example 2

4-[4-(1,3-benzodioxol-5-ylmethyl)-1-piperazinyl]-1-(2,3,4,5-tetrahydro-1H-3-benzazepin-7-yl)-1-butanone trihydrochloride

$^1$H-NMR (DMSO-$d_6$) δ: 2.00-2.15(2H,m), 3.00-3.20(12H,m), 3.25-3.80(10H,m), 6.07(2H,s), 6.98(1H,d, J=8.0Hz), 7.05-7.15(1H,m), 7.27(1H,m), 7.37(1H,d,J=8.0Hz), 7.75-7.85(2H,m) MS(ESI+): 436(M+H)

Example 3

4-(4-benzhydryl-1-piperazinyl)-1-(2,3,4,5-tetrahydro-1H-3-benzazepin-7-yl)-1-butanone

$^1$H-NMR ($CDCl_3$) δ: 1.55(4H,m), 2.20-2.60(12H,m), 2.80-2.30(8H,m), 4.21(1H,s), 6.85-7.60(13H,m) MS(ESI+): 454(M+H)

Example 4

4-(4-benzhydryl-1-piperazinyl)-1-(2,3,4,5-tetrahydro-1H-3-benzazepin-7-yl)-1-butanone trihydrochloride

$^1$H-NMR(DMSO-$d_6$) δ: 1.40-1.80(4H,m), 3.00-3.40(12H,m), 3.50-4.00(9H,m), 7.00-7.80(13H,m) MS(ESI+): 454(M+H)

Example 5

4-{4-[bis(4-fluorophenyl)methyl]-1-piperazinyl}-1-(2,3,4,5-tetrahydro-1H-3-benzazepin-7-yl)-1-butanone

$^1$H-NMR ($CDCl_3$) δ: 1.80-2.00(2H,m), 2.25-2.55(10H,m), 3.90-4.00(10H,m), 4.18(1H,s), 6.90-7.00(4H,m), 7.15(1H,d,J=8.2Hz), 7.25-7.50(4H,m), 7.50-7.80(2H,m) MS(ESI+): 504(M+H)

Example 6

4-{4-[bis(4-fluorophenyl)methyl]-1-piperazinyl}-1-(2,3,4,5-tetrahydro-1H-3-benzazepin-7-yl)-1-butanone trihydrochloride

$^1$H-NMR (DMSO-$d_6$) δ: 1.90-2.15(2H,m), 2.60-3.80(21H,m), 7.10-7.30(4H,m), 7.37(1H,d,J=8.4z), 7.40-7.95 (6H,m) MS(ESI+): 504(M+H)

Example 7

4-{4-(4-chlorobenzyl)-1-piperazinyl}-1-(2,3,4,5-tetrahydro-lH-3-benzazepin-7-yl)-1-butanone

$^1$H-NMR (DMSO-$d_6$) δ: 1.80-2.00(2H,m), 2.30-2.55(10H,m), 2.85-3.00(10H,m), 3.45(2H,s), 7.10-7.30(5H,m), 7.65-7.75(2H,m)
MS(ESI+): 426(M+H)

Example 8

4-{4-(4-chlorobenzyl)-1-piperazinyl}-1-(2,3,4,5-tetrahydro-lH-3-benzazepin-7-yl)-1-butanone trihydrochloride

$^1$H-NMR (DMSO-$d_6$) δ: 1.95-2.10(2H,m), 3.00-3.95(20H,m), 4.20-4.40(2H,m), 7.38(1H,d,J=8.4Hz), 7.53(2H, d,J=8.4Hz), 7.68(2H,d,J=8.4Hz), 7.75-7.85(2H,m)
MS(APCI+): 426(M+H)

Example 9

4-{4-(1-naphthylmethyl)-1-piperazinyl}-1-(2,3,4,5-tetrahydro-1H-3-benzazepin-7-yl)-1-butanone

$^1$H-NMR (DMSO-$d_6$) δ: 1.85-2.00(2H,m), 2.21(2H,m), 2.35-2.60(8H,m), 2.80-3.00(10H,m), 3.88(2H,s), 7.14-7.19(1H,m), 7.40-7.55(4H,m), 7.65-7.90(4H,m), 8.25-8.35(1H,m) MS(APCI+): 442(M+H)

Example 10

4-{4-(1-naphthylmethyl}-1-piperazinyl}-1-(2,3,4,5-tetrahydro-1H-3-benzazepin-7-yl)-1-butanone trihydrochloride

$^1$H-NMR (DMSO-$d_6$) δ: 1.90-2.10(2H,m), 3.00-4.00(22H,m), 7.30-7.40(1H,m), 7.50-7.70(2H,m), 7.75-8.15 (6H,m), 8.35-8.45(1H,m)

MS(APCI+): 442(M+H)

Example 11

4-[4-(4-chlorobenzyl)-1-piperazinyl]-1-(2,3,4,5-tetrahydro-1H-3-benzazepin-7-yl)-1-butanone tritrifluoroacetate

A mixture of 4-bromo-1-[3-(2,2,2-trifluoroacetyl)-2,3,4,5-tetrahydro-1H-3-benzazepin-7-yl]-1-butanone (100mg), 1-(4-chlorobenzyl)piperazine (81mg), triethylamine (0.053ml) and DMF (3ml) was stirred at 80°C for 15 hours, and polystyrene methylisocyanate (255mg) was added, followed by further stirring for 1 hour. After the resin was filtered off, the filtrate was concentrated under reduced pressure. Dichloromethane (1.5ml) and water (1.5ml) were added to the residue, and the layers were separated using Filter Tube (Whatman; catalogue No. 6984-0610). The dichloromethane solution was concentrated under reduced pressure. The residue was dissolved in methanol (1ml), a 1M aqueous potassium carbonate solution (0.51ml) was added, and the mixture was stirred at room temperature for 1.5 hours. The methanol was evaporated under reduced pressure, dichloromethane (lml) was added, and the layers were separated using Filter Tube (same as above). The dichloromethane solution was concentrated under reduced pressure, and the residue was purified by preparative HPLC to give an objective compound (24mg).
$^1$H-NMR (Acetone-$d_6$) δ: 2.10-2.25(2H,m), 3.15-3.80(20H,m), 4.05(2H,s), 7.30-7.60(5H,m), 7.80-7.90(2H,m) HPLC analysis (Condition A): purity 95% (retention time: 2.021min.)
MS (APCI+): 426 (M+H)

The following compounds were prepared as in Example 11.

Example 12

4-{4-[bis(4-fluorophenyl)methyl]-1-piperazinyl}-1-(2,3,4,5-tetrahydro-1H-3-benzazepin-7-yl)-1-butanone tritrifluoroacetate

yield :31mg
HPLC analysis (Condition A): purity 89% (retention time: 2.725min.)
MS(APCI+): 504 (M+H)

Example 13

tert-butyl 4-[4-oxo-4-(2,3,4,5-tetrahydro-1H-3-benzazepin-7-yl)butyl]-1-piperazinecarboxylate tritrifluoroacetate

yield :32mg
HPLC analysis (Condition A): purity 95% (retention time: 1.088min.)
MS(APCI+): 402 (M+H)

Example 14

4-{4-[(5-phenyl-1,2,4-oxadiazol-3-yl)methyl]-1-piperazinyl}-1-(2,3,4,5-tetrahydro-1H-3-benzazepin-7-yl)-1-butanone tritrifluoroacetate

yield :42mg
HPLC analysis (Condition A): purity 91% (retention time: 1.898min.)
MS(APCI+): 460 (M+H)

Example 15

4-{4-([1,1'-biphenyl]-4-ylmethyl)-1-piperazinyl}-1-(2,3,4,5-tetrahydro-1H-3-benzazepin-7-yl)-1-butanone tritrifluoroacetate

yield :40mg
HPLC analysis (Condition A): purity 94% (retention time: 2.249min.)
MS(APCI+): 468 (M+H)

Example 16

4-{4-(4-methoxybenzyl)-1-piperazinyl}-1-(2,3,4,5-tetrahydro-lH-3-benzazepin-7-yl)-1-butanone tritrifluoroacetate

yield :15mg
HPLC analysis (Condition A): purity 98% (retention time: 0.722min.)
MS(APCI+): 422 (M+H)

Example 17

4-{4-(4-fluorobenzyl}-1-piperazinyl}-1-(2,3,4,5-tetrahydro-1H-3-benzazepin-7-yl)-1-butanone tritrifluoroacetate

yield :28mg
HPLC analysis (Condition A): purity 93% (retention time: 0.840min.)
MS(APCI+): 410 (M+H)

Example 18

4-({4-[4-oxo-4-(2,3,4,5-tetrahydro-1H-3-benzazepin-7-yl)butyl]-1-piperazinyl}methyl)benznitrile tritrifluoroacetate

yield :47mg
HPLC analysis (Condition A): purity 70% (retention time: 1.022min.)
MS(APCI+): 417 (M+H)

Example 19

4-{4-(4-methylbenzyl)-1-piperazinyl}-1-(2,3,4,5-tetrahydro-1H-3-benzazepin-7-yl)-1-butanone tritrifluoroacetate

yield :20mg
HPLC analysis (Condition A): purity 97% (retention time: 0.965min.)
MS(APCI+): 406 (M+H)

Example 20

4-{4-(1-naphtylmethyl)-1-piperazinyl}-1-(2,3,4,5-tetrahydro-1H-3-benzazepin-7-yl)-1-butanone tritrifluoroacetate

yield :6.1mg
HPLC analysis (Condition A): purity 83% (retention time: 2.110min.)
MS(APCI+): 442 (M+H)

Example 21

4-{4-(1-isoquinolinylmethyl)-1-piperazinyl}-1-(2,3,4,5-tetrahydro-lH-3-benzazepin-7-yl)-1-butanone tritrifluoroacetate

yield :4.4 mg
HPLC analysis (Condition A): purity 94% (retention time: 0.745min.)
MS(APCI+): 443 (M+H)

Example 22

4-{4-(4-pyridylmethyl)-1-piperazinyl}-1-(2,3,4,5-tetrahydro-1H-3-benzazepin-7-yl)-1-butanone tritrifluoroacetate

yield :12 mg
MS(APCI+): 393 (M+H)

Example 23

4-{4-ethyl-1-piperazinyl}-1-(2,3,4,5-tetrahydro-1H-3-benzazepin-7-yl)-1-butanone tritrifluoroacetate

yield :47 mg
HPLC analysis (Condition B): purity 99% (retention time: 0.787min.)
MS(APCI+): 330 (M+H)

Example 24

4-{4-[(E)-3-phenyl-2-propenoyl]-1-piperazinyl}-1-(2,3,4,5-tetrahydro-1H-3-benzazepin-7-yl)-1-butanone tritrifluoroacetate

yield :33 mg
HPLC analysis (Condition A): purity 93% (retention time: 3.551min.)
MS(APCI+): 418 (M+H)

Example 25

4-{4-acetyl-1-piperazinyl}-1-(2,3,4,5-tetrahydro-1H-3-benzazepin-7-yl)-1-butanone tritrifluoroacetate

yield :20 mg
HPLC analysis (Condition B): purity 87% (retention time: 4.676min.)
MS(APCI+): 344 (M+H)

Example 26

4-{4-(2-furylmethyl)-1-piperazinyl}-1-(2,3,4,5-tetrahydro-1H-3-benzazepin-7-yl)-1-butanone tritrifluoroacetate

yield :30 mg
HPLC analysis (Condition B): purity 98% (retention time: 5.192min.)
MS(APCI+): 382 (M+H)

Example 27

4-{4-(1-piperidinyl)-1-piperidinyl}-1-(2,3,4,5-tetrahydro-1H-3-benzazepin-7-yl)-1-butanone tritrifluoroacetate

yield :52 mg
HPLC analysis (Condition B): purity 97% (retention time: 5.073min.)
MS(APCI+): 384 (M+H)

Example 28

4-(4-phenethyl-1-piperazinyl)-1-(2,3,4,5-tetrahydro-1H-3-benzazepin-7-yl)-1-butanone tritrifluoroacetate

yield :63 mg
HPLC analysis (Condition A): purity 95% (retention time: 1.549min.)
MS(APCI+): 406 (M+H)

Example 29

4-[4-(1-phenylethyl)-1-piperazinyl]-1-(2,3,4,5-tetrahydro-1H-3-benzazepin-7-yl)-1-butanone tritrifluoroacetate

yield :70 mg
HPLC analysis (Condition A): purity 91% (retention time: 1.443min.)
MS(APCI+): 406 (M+H)

Example 30

4-[4-(ethylsulfonyl)-1-piperazinyl]-1-(2,3,4,5-tetrahydro-1H-3-benzazepin-7-yl)-1-butanone tritrifluoroacetate

yield :24 mg
HPLC analysis (Condition A): purity 96% (retention time: 0.942min.)
MS(APCI+): 394 (M+H)

Example 31

4-{4-[2-(dimethylamino)ethyl]-1-piperazinyl}-1-[2,3,4,5-tetrahydro-1H-3-benzazepin-7-yl)-1-butanone tritrifluoroacetate

yield :4.1 mg
MS(APCI+): 373 (M+H)

Example 32

4-{4-[4-(1H-1,2,3,4-tetrazol-1-yl)benzyl]-1-piperazinyl}-1-(2,3,4,5-tetrahydro-1H-3-benzazepin-7-yl)-1-butanone tritrifluoroacetate

yield :31 mg
HPLC analysis (Condition A): purity 96% (retention time: 1.428min.)
MS(APCI+): 460 (M+H)

Example 33

4-[4-(3,5-dimethyl-4-isoxazolyl)-1-piperazinyl]-1-(2,3,4,5-tetrahydro-1H-3-benzazepin-7-yl)-1-butanone tritrifluoroacetate

yield :17 mg
HPLC analysis (Condition A): purity 97% (retention time: 1.066min.)
MS(APCI+): 411 (M+H)

Example 34

4-[4-(cyclohexylmethyl)-1-piperazinyl]-1-(2,3,4,5-tetrahydro-1H-3-benzazepin-7-yl)-1-butanone tritrifluoroacetate

yield :28 mg
HPLC analysis (Condition A): purity 91% (retention time: 1.565min.)
MS(APCI+): 398 (M+H)

Example 35

4-(4-benzyl-1-piperidinyl)-1-(2,3,4,5-tetrahydro-1H-3-benzazepin-7-yl)-1-butanone ditrifluoroacetate

yield :32 mg
HPLC analysis (Condition A): purity 97% (retention time: 2.463min.)
MS(APCI+): 391 (M+H)

Example 36

4-[4-(4-fluorobenzyl)-1-piperidinyl)-1-(2,3,4,5-tetrahydro-1H-3-benzazepin-7-yl)-1-butanone ditrifluoroacetate

yield :42 mg
HPLC analysis (Condition A): purity 94% (retention time: 2.528min.)
MS(APCI+): 409 (M+H)

Example 37

4-[4-(4-benzhydroxy)-1-piperidinyl)-1-[2,3,4,5-tetrahydro-1H-3-benzazepin-7-yl)-1-butanone ditrifluoroacetate

yield :29 mg
HPLC analysis (Condition A): purity 93% (retention time: 2.909min.)
MS(APCI+): 483 (M+H)

Example 38

1-(4-fluorobenzyl)-4-[4-oxo-4-(2,3,4,5-tetrahydro-1H-3-benzazepin-7-yl)butyl]-2-piperazinone ditrifluoroacetate

yield :14 mg
HPLC analysis (Condition A): purity 84% (retention time: 2.043min.)
MS(APCI+): 424 (M+H)

Example 39

4-[4-(4-methoxyphenyl)-1-piperazinyl)-1-(2,3,4,5-tetrahydro-1H-3-benzazepin-7-yl)-1-butanone tritrifluoroacetate

yield :56 mg
HPLC analysis (Condition A): purity 93% (retention time: 2.124min.)
MS(APCI+): 408 (M+H)

Example 40

1-(2,3,4,5-tetrahydro-1H-3-benzazepin-7-yl)-4-{4-[3-(trifluoromethyl)phenyl]-1-piperazinyl}-1-butanone tritrifluoroacetate

yield :33 mg
HPLC analysis (Condition A): purity 95% (retention time: 2.593min.) MS(APCI+): 446 (M+H)

Example 41

4-[4-(4-fluorophenyl)-1-piperazinyl)-1-(2,3,4,5-tetrahydro-1H-3-benzazepin-7-yl)-1-butanone tritrifluoroacetate

yield :30 mg
HPLC analysis (Condition A) : purity 83% (retention time: 2.240min.)
MS(APCI+): 396 (M+H)

Example 42

4-[4-(4-acetylphenyl)-1-piperazinyl)-1-(2,3,4,5-tetrahydro-1H-3-benzazepin-7-yl)-1-butanone tritrifluoroacetate

yield :40mg
HPLC analysis (Condition A) : purity 92% (retention time: 2.003min.)
MS(APCI+): 420 (M+H)

Example 43

4-[4-(2,3-dimethylphenyl)-1-piperazinyl)-1-(2,3,4,5-tetrahydro-1H-3-benzazepin-7-yl)-1-butanone tritrifluoroacetate

yield :20mg
HPLC analysis (Condition A): purity 86% (retention time: 2.600min.)
MS(APCI+): 406 (M+H)

Example 44

4-[4-(2-pyrimidinyl)-1-piperazinyl)-1-(2,3,4,5-tetrahydro-1H-3-benzazepin-7-yl)-1-butanone tritrifluoroacetate

yield :32mg
HPLC analysis (Condition A): purity 95% (retention time: 1.365min.)
MS(APCI+): 380 (M+H)

Example 45

4-[4-(3,5-dichloro-4-pyridinyl)-1-piperazinyl)-1-(2,3,4,5-tetrahydro-1H-3-benzazepin-7-yl)-1-butanone tritrifluoroacetate

yield :27mg
HPLC analysis (Condition A): purity 90% (retention time: 2.000min.)
MS(APCI+): 447 (M+H)

Example 46

4-[4-(1H-indol-4-yl)-1-piperazinyl)-1-(2,3,4,5-tetrahydro-1H-3-benzazepin-7-yl)-1-butanone tritrifluoroacetate

yield :14mg
HPLC analysis (Condition A): purity 95% (retention time: 2.076min.)
MS(APCI+): 417 (M+H)

Example 47

1-(2,3,4,5-tetrahydro-1H-3-benzazepin-7-yl)-4-{4-[4-(trifluoromethoxy)phenyl]-1-piperadinyl}-1-butanone tritrifluoroacetate

yield :50mg
HPLC analysis (Condition A): purity 96% (retention time: 2.688min.)
MS(APCI+): 462 (M+H)

Example 48

4-[4-(1-naphthyl)-1-piperazinyl]-1-(2,3,4,5-tetrahydro-1H-3-benzazepin-7-yl)-1-butanone tritrifluoroacetate

yield :8.7mg
HPLC analysis (Condition A): purity 90% (retention time: 2.682min.)
MS(APCI+): 428 (M+H)

Example 49

4-(4-[1,1'-biphenyl]-4-yl-1-piperazinyl)-1-(2,3,4,5-tetrahydro-1H-3-benzazepin-7-yl)-1-butanone tritrifluoroacetate

yield :9.3mg
HPLC analysis (Condition A): purity 93% (retention time: 2.861min.)
MS(APCI+): 454 (M+H)

Example 50

4-(4-benzoyl-1-piperazinyl)-1-(2,3,4,5-tetrahydro-1H-3-benzazepin-7-yl)-1-butanone tritrifluoroacetate

yield :23mg
HPLC analysis (Condition A): purity 92% (retention time: 1.740min.)
MS(APCI+): 406 (M+H)

Example 51

4-[3,4-dihydro-2(1H)-isoquinolinyl]-1-(2,3,4,5-tetrahydro-1H-3-benzazepin-7-yl)-1-butanone ditrifluoroacetate

yield :15mg
HPLC analysis (Condition A): purity 95% (retention time: 2.008min.)
MS(APCI+): 349 (M+H)

Example 52

4-(4-phenyl-1-piperidinyl)-1-(2,3,4,5-tetrahydro-1H-3-benzazepin-7-yl)-1-butanone ditrifluoroacetate

yield :4.6mg
HPLC analysis (Condition A): purity 77% (retention time: 2.372min.)
MS(APCI+): 377 (M+H)

Example 53

4-[4-(2-methoxyphenyl)-1-piperidinyl]-1-(2,3,4,5-tetrahydro-1H-3-benzazepin-7-yl)-1-butanone ditrifluoroacetate

yield :16mg
HPLC analysis (Condition A): purity 83% (retention time: 2.475min.)
MS(APCI+): 407 (M+H)

Example 54

4-[spiro(1H-indene-1,4'-piperidinyl)]-1-(2,3,4,5-tetrahydro-1H-3-benzazepin-7-yl)-1-butanone ditrifluoroacetate

yield :2.2mg
HPLC analysis (Condition A): purity 89% (retention time: 2.548min.)
MS(APCI+): 401 (M+H)

Example 55

4-[4-(2-fluorobenzyl)-1-piperidinyl]-1-(2,3,4,5-tetrahydro-1H-3-benzazepin-7-yl)-1-butanone ditrifluoroacetate

yield :5.9mg
HPLC analysis (Condition A): purity 100% (retention time: 2.582min.)
MS(APCI+): 409 (M+H)

Example 56

4-[4-(4-trifluoromethylbenzyl)-1-piperidinyl]-1-(2,3,4,5-tetrahydro-1H-3-benzazepin-7-yl)-1-butanone ditrifluoroacetate

yield :19mg
HPLC analysis (Condition A): purity 97% (retention time: 2.886min.)
MS(APCI+): 459 (M+H)

Example 57

4-[4-{[4-(tert-butyl)phenyl]sulfonyl}-1-piperidinyl]-1-(2,3,4,5-tetrahydro-1H-3-benzazepin-7-yl)-1-butanone ditrifluoroacetate

yield :20mg
HPLC analysis (Condition A): purity 82% (retention time: 2.784min.)
MS(APCI+): 497 (M+H)

Example 58

4-[{2-[benzyl(methyl)amino]ethyl}(methyl)amino]-1-(2,3,4,5-tetrahydro-1H-3-benzazepin-7-yl)-1-butanone tritrifluoroacetate

yield :15mg
HPLC analysis (Condition A): purity 88% (retention time: 1.461min.)
MS(APCI+): 394 (M+H)

Example 59

4-{4-[(4-chlorophenyl)(phenyl)methyl]-1-piperazinyl}-1-(2,3,4,5-tetrahydro-1H-3-benzazepin-7-yl)-1-butanone tritrifluoroacetate

yield :22mg
HPLC analysis (Condition A): purity 72% (retention time: 3.045min.)
MS(APCI+): 502 (M+H)

Example 60

4-[4-(1,3-benzodioxol-5-ylmethyl)-1-piperidinyl]-1-(2,3,4,5-tetrahydro-1H-3-benzazepin-7-yl)-1-butanone ditrifluoroacetate

yield :6.1mg
HPLC analysis (Condition A): purity 91% (retention time: 2.523min.)
MS(APCI+): 435 (M+H)

Example 61

4-[4-(phenylsulfanyl)-1-piperidinyl]-1-(2,3,4,5-tetrahydro-lH-3-benzazepin-7-yl)-1-butanone ditrifluoroacetate

yield :4.0mg
HPLC analysis (Condition A): purity 91% (retention time: 2.545min.)
MS(APCI+): 409 (M+H)

Example 62

4-[{2-[benzhydryl(methyl)amino]ethyl}(methyl)amino]-1-(2,3,4,5-tetrahydro-1H-3-benzazepin-7-yl)-1-butanone tritrifluoroacetate

yield :5.7mg
HPLC analysis (Condition A): purity 75% (retention time: 2.424min.)
MS(APCI+): 470 (M+H)

Example 63

4-[4-(2,4-difluorobenzyl)-1-piperidinyl]-1-(2,3,4,5-tetrahydro-1H-3-benzazepin-7-yl)-1-butanone ditrifluoroacetate

yield :9.9mg
HPLC analysis (Condition A): purity 97% (retention time: 2.655min.)
MS(APCI+): 427 (M+H)

Example 64

4-[4-{2-(1H-1,2,3,4-tetrazol-1-yl)benzyl}-1-piperidinyl]-1-(2,3,4,5-tetrahydro-1H-3-benzazepin-7-yl)-1-butanone ditrifluoroacetate

yield :18mg

HPLC analysis (Condition A): purity 73% (retention time: 2.265min.)
MS(APCI+): 459 (M+H)

Example 65

4-[4-(4-methoxybenzyl)-1-piperidinyl]-1-(2,3,4,5-tetrahydro-1H-3-benzazepin-7-yl)-1-butanone ditrifluoroacetate

yield :8.8mg
HPLC analysis (Condition A): purity 80% (retention time: 2.545min.)
MS(APCI+): 421 (M+H)

Example 66

4-({1-[4-oxo-4-(2,3,4,5-tetrahydro-1H-3-benzazepin-7-yl)butyl]-4-piperidinyl}methyl)benzenesulfonamide ditrifluoroacetate

yield :3.2mg
HPLC analysis (Condition A): purity 79% (retention time: 2.073min.)
MS(APCI+): 470 (M+H)

Example 67

N,N-dimethyl-4-({1-[4-oxo-4-(2,3,4,5-tetrahydro-1H-3-benzazepin-7-yl)butyl]-4-piperidinyl}methyl) benzenesulfonamide ditrifluoroacetate

yield :33mg
HPLC analysis (Condition A): purity 93% (retention time: 2.440min.)
MS(APCI+): 498 (M+H)

Example 68

methyl 4-({1-[4-oxo-4-(2,3,4,5-tetrahydro-1H-3-benzazepin-7-yl)butyl]-4-piperidinyl}methyl)benzoate ditrifluoroacetate

yield :10mg
HPLC analysis (Condition A): purity 81% (retention time: 2.538min.)
MS(APCI+): 449 (M+H)

Example 69

4-(4-{[(4-fluorophenyl)sulfanyl]methyl}-1-piperidinyl)-1-(2,3,4,5-tetrahydro-1H-3-benzazepin-7-yl)-1-butanone ditrifluoroacetate

yield :14mg
HPLC analysis (Condition A): purity 89% (retention time: 2.724min.)
MS(APCI+): 441 (M+H)

Example70

4-[4-(3-fluorobenzyl)-1-piperidinyl]-1-(2,3,4,5-tetrahydro-1H-3-benzazepin-7-yl)-1-butanone ditrifluoroacetate

yield :10mg
HPLC analysis (Condition A): purity 88% (retention time: 2.605min.)
MS(APCI+): 409 (M+H)

Example 71

4-(4-benzhydryl-1-piperazinyl)-1-(3-benzyl-2,3,4,5-tetrahydro-1H-3-benzazepin-7-yl)-1-butanone tritrifluoroacetate

A mixture of 4-(4-benzhydryl-1-piperazinyl)-1-(2,3,4,5-tetrahydro-lH-3-benzazepin-7-yl)-1-butanone (95mg), benzyl bromide (0.027ml), potassium carbonate (31mg) and DMF (5ml) was stirred at 70°C for 14hours. The reaction mixture was diluted with water, and extracted with ethyl acetate. The extract was washed with water, dried over anhydrous magnesium sulfate, and the solvent was evaporated under reduced pressure. The residue was purified by preparative HPLC to give an objective compound (28mg). $^1$H-NMR(Acetone-$d_6$) δ: 2.00-2.25(2H,m), 2.80-4.00(20H,m), 4.50(2H,s), 4.58(1H,s),7.20-8.00(18H,m)
MS (ESI+):558 (M+H)

Example 72

4-(4-benzhydryl-1-piperazinyl)-1-(3-methyl-2,3,4,5-tetrahydro-1H-3-benzazepin-7-yl)-1-butanone tritrifluoroacetate

This was prepared as in Example 71.
yield 4.0mg
$^1$H-NMR(Acetone-$d_6$) δ: 2.10-2.25(2H,m), 2.98(3H,s), 3.00-3.40(6H,m), 3.40-4.00(14H,m), 4.49(1H,s), 7.20-7.40(7H, m), 7.50-7.60(4H,m), 7.85-7.90(2H,m).
MS (ESI+):482 (M+H)

Example 73

7-[4-(4-benzhydryl-1-piperazinyl)butyl]-2,3,4,5-tetrahydro-1H-3-benzazepine trihydrochloride

1) 1-{7-[4-(4-benzhydryl-1-piperazinyl)butyl]-1,2,4,5-tetrahydro-3H-3-benzazepin-3-yl}-2,2,2-trifluoro-1-ethanone

Triethylsilane (0.34ml) was added to a solution of 4-(4-benzhydryl-1-piperazinyl)-1-[3-(2,2,2-trifluoroacetyl)-2,3,4,5-tetrahydro-lH-3-benzazepin-7-yl]-1-butanone (150mg) in trifluoroacetic acid (5ml), and the mixture was stirred at room temperature for 17 hours. After the solution was concentrated under reduced pressure, ethyl acetate was added to the residue, and the mixture was successively washed with aqueous saturated sodium bicarbonate solution and brine. After dried over anhydrous magnesium sulfate, the solvent was evaporated under reduced pressure. The residue was purified by silica gel column chromatography (n-hexane/ethyl acetate=3/1) to give the title compound (40mg).
$^1$H-NMR($CDCl_3$) δ: 1.40-1.70(4H,m), 2.25-2.65(12H,m), 2.93(4H,m), 3.60-3.80(4H,m), 4.20(1H,s), 6.90-7.50(13H,m)

2) 7-[4-(4-benzhydryl-1-piperazinyl)butyl]-2,3,4,5-tetrahydro-1H-3-benzazepine trihydrochloride

This was prepared as in 4) in Example 1.
yield 16mg
$^1$H-NMR(DMSO-$d_6$) δ: 1.40-1.80(4H,m), 3.00-3.40(12H,m), 3.50-4.00(9H,m), 7.00-7.80(13H,m)
MS (ESI+):454 (M+H)

Example 74

N-[2-(4-benzyl-1-piperazinyl)ethyl]-2,3,4,5-tetrahydro-1H-3-benzazepine-7-sulfonamide

1) 3-(2,2,2-trifluoroacetyl)-2,3,4,5-tetrahydro-1H-3-benzazepine-7-sulfonyl chloride

Chlorosulfonic acid (4.65ml) was added to a solution of 2,2,2-trifluoro-1-(1,2,4,5-tetrahydro-3H-3-benzazepin-3-yl)-1-ethanone (2.43g) in dichloroethane (10ml), and the mixture was stirred at room temperature for 10 minutes. The reaction solution was poured into water, and extracted with diethyl ether. The extract was washed with brine, dried over anhydrous magnesium sulfate, and the solvent was evaporated under reduced pressure to give the title compound (1.70g).
$^1$H-NMR($CDCl_3$) δ: 3.05-3.20(4H,m), 3.70-3.90(4H,m), 7.42(1H,d,J=5.6,8.2Hz), 7.82-7.90(2H,m)

2) N-[2-(4-benzyl-1-piperazinyl)ethyl]-3-(2,2,2-trifluoroacetyl)-2,3,4,5-tetrahydro-1H-3-benzazepine-7-sulfonamide

1-(2-aminoethyl)-4-benzylpiperazine (213mg) and triethylamine (0.14ml) were added to a solution of 3-(2,2,2-trifluoroacetyl)-2,3,4,5-tetrahydro-1H-3-benzazepine-7-sulfonyl chloride (301mg) in THF (5ml), and the mixture was stirred at room temperature for 15 hours. The reaction solution was diluted with water, and extracted with diethyl ether. The extract was washed with brine, dried over anhydrous magnesium sulfate, and the solvent was evaporated under reduced pressure. The residue was purified by silica gel column chromatography (n-hexane/ethyl acetate=1/1) to give the title compound (305mg).
$^1$H-NMR($CDCl_3$) δ: 2.30-2.50(10H,m), 2.90-3.10(6H,m), 3.49(2H,s), 3.65-3.80(4H,m), 7.25-7.35(6H,m), 7.60-7.70(2H,m)
MS (APCI+):525 (M+H

3) N-[2-(4-benzyl-1-piperazinyl)ethyl]-2,3,4,5-tetrahydro-1H-3-benzazepine-7-sulfonamide

A 1M aqueous potassium carbonate solution (1.72ml) was added to a solution of N-[2-(4-benzyl-1-piperazinyl)ethyl]-3-(2,2,2-trifluoroacetyl)-2,3,4,5-tetrahydro-1H-3-benzazepine-7-sulfonamide (300mg) in methanol (4ml), and the mixture was stirred at room temperature for 1.5 hours. The methanol was evaporated under reduced pressure, followed by extraction with ethyl acetate. The extract was washed with brine, dried over anhydrous magnesium sulfate, and the solvent was evaporated under reduced pressure to give objective compound (192mg).
$^1$H-NMR($CDCl_3$) δ: 2.20-2.45(10H,m), 2.96(10H,m), 3.49(2H,s), 7.15-7.35(6H,m), 7.55-7.65(2H,m)
MS (APCI+):429 (M+H)

Example 75

N-[2-(4-benzyl-1-piperazinyl)ethyl]-2,3,4,5-tetrahydro-1H-3-benzazepine-7-sulfonamide trihydrochloride

N-[2-(4-benzyl-1-piperazinyl)ethyl]-2,3,4,5-tetrahydro-1H-3-benzazepine-7-sulfonamide was treated with a IN hydrogen chloride solution in ethyl acetate to give an objective compound (212mg).
$^1$H-NMR($CDCl_3$) δ: 3.0-3.90(20H,m), 4.34(2H,s), 7.40-7.55(4H,m), 7.60-7.75(4H,m), 8.05(1H,m) MS (APCI+):429 (M+H)

Example 76

N-[2-(4-benzhydryl-1-piperazinyl)ethyl]-2,3,4,5-tetrahydro-1H-3-benzazepine-7-sulfonamide

This was prepared as in Example 74.
yield:171mg
$^1$H-NMR(DMSO-$d_6$) δ: 2.20-2.40(10H,m), 2.80-3.30(10H,m), 4.19(1H,s), 7.10-7.31(8H,m), 7.35-7.45(3H,m), 7.55-7.60 (2H,m)
MS (ESI+):505 (M+H)

Example 77

N-[2-(4-benzhydryl-1-piperazinyl)ethyl]-2,3,4,5-tetrahydro-1H-3-benzazepine-7-sulfonamide trihydrochloride

This was prepared as in Example 75.
yield:180mg
$^1$H-NMR(DMSO-$d_6$) δ: 3.00-3.60(21H,m), 7.20-7.45(7H,m), 7.45-7.80(6H,m), 8.09(1H,m)
MS (ESI+):505 (M+H)

Example 78

N-{2-[4-(4-chlorobenzyl)-1-piperazinyl]ethyl}-2,3,4,5-tetrahydro-1H-3-benzazepine-7-sulfonamide

This was prepared as in Example 74.
yield:166mg
$^1$H-NMR(DMSO-$d_6$) δ: 2.25-2.45(10H,m), 2.96(10H,m), 3.44(2H,s), 7.15-7.35(6H,m), 7.55-7.65(2H,m)
MS (ESI+):463(M+H)

Example 79

N-{2-[4-(4-chlorobenzyl)-1-piperazinyl]ethyl}-2,3,4,5-tetrahydro-1H-3-benzazepine-7-sulfonamide trihydrochloride

This was prepared as in Example 75.
yield:190mg
$^1$H-NMR(DMSO-$d_6$) δ: 2.80-3.80(20H,m), 4.32(2H,m), 7.43-7.55(3H,m), 7.64-7.70(4H,m),8.07(1H,m)
MS (ESI+):463(M+H)

Example 80

4-[{2-[[bis(4-fluorophenyl)methyl](methyl)amino]ethyl}(methyl)amino]-1-(2,3,4,5-tetrahydro-lH-3-benzazepine-7-yl) butan-1-one

This was prepared as in Example 1.
$^1$H-NMR(DMSO-$d_6$) δ: 1.80-2.00(2H,m), 2.14(3H,s), 2.15(3H,s), 2.30-2.60(8H,m), 2.90-3.10(8H,m), 4.37(1H,s) 6.85-7.00(4H,m), 7.15-7.40(5H,m), 7.50-7.70(2H,m)
MS (APCI+):506(M+H)

Example 81

N-[2-(4-benzylpiperazin-1-yl)ethyl]-2,3,4,5-tetrahydro-1H-3-benzazepine-7-carboxamide trihydrochloride

1) 1,2,4,5-tetrahydro-3H-3-benzazepine-3-carboaldehyde

Acetic anhydride (18ml) was added to formic acid (54ml), and the mixture was stirred at room temperature for 1 hour. To this mixture was poured dropwise a solution of 2,3,4,5-tetrahydro-1H-3-benzazepine (9.5g) in ethyl acetate (5ml) under ice-cooling. The mixture was stirred at room temperature for 30 minutes, and the solvent was evaporated under reduced pressure. Ethyl acetate and an aqueous saturated sodium bicarbonate solution were added to the residue, and the mixture was extracted with ethyl acetate. The extract was washed with brine, dried over anhydrous magnesium sulfate, and the solvent was evaporated under reduced pressure to give the title compound (9.37g).
$^1$H-NMR($CDCl_3$) δ: 2.85-3.00(4H,m), 3.45-3.50(2H,m), 3.64-3.70(2H,m), 7.10-7.20(4H,m), 8.15(1H,s)

2) 7-acetyl-1,2,4,5-tetrahydro-3H-3-benzazepine-3-carboaldehyde

Aluminium chloride (12.0g) was added to a solution of 1,2,4,5-tetrahydro-3H-3-benzazepine-3-carboaldehyde (4.50g) and acetyl chloride (2.01ml) in dichloroethane (25ml). The reaction mixture was stirred at room temperature for 15 hours, poured into ice-water, and extracted with ethyl acetate. The extract was washed with brine, dried over anhydrous magnesium sulfate, and the solvent was evaporated under reduced pressure. The residue was purified by silica gel column chromatography (ethyl acetate) to give the title compound (3.26g).
$^1$H-NMR($CDCl_3$) δ: 2.60(3H,s), 2.90-3.05(4H,m), 3.45-3.55(2H,m), 3.65-3.75(2H,m), 7.20-7.30(1H,m), 7.50-7.80(2H, m), 8.16(1H,s)

3) 3-formyl-2,3,4,5-tetrahydro-1H-3-benzazepine-7-carboxylic acid

An aqueous solution (70ml) of sodium hydroxide (4.78g) was added to a solution of 7-acetyl-1,2,4,5-tetrahydro-3H-3-benzazepine-3-carboaldehyde (3.24g) in dioxane (50ml), and bromine (2.31ml) was added dropwise under ice-cooling. The reaction mixture was stirred for 30 minutes under ice-cooling, and acetone was added to stop the reaction. After the solvent was evaporated under reduced pressure, the aqueous layer was extracted with ethyl acetate, and 5N hydrochloric acid was added to the extract. The precipitated crystals were filtered, and washed successively with water and ether to give the title compound (2.11g).
$^1$H-NMR(DMSO-$d_6$) δ: 2.85-3.00(4H,m), 3.45-3.60(4H,m), 7.32(1H,dd,J=2.2,7.6Hz), 7.72-7.80(2H,m), 8.12(1H,s)

4) 2,3,4,5-tetrahydro-1H-3-benzazepine-7-carboxylic acid

A solution of 3-formyl-2,3,4,5-tetrahydro-1H-3-benzazepine-7-carboxylic acid (1.0g) in concentrated hydrochloric acid (50ml) was stirred at 100°C for 12 hours. The solution was concentrated under reduced pressure, the resulting solid was filtered, and washed successively with water and ether to obtain the title compound

(990mg). $^1$H-NMR($CDCl_3$) δ: 3.18(4H,m), 3.46(4H,m), 7.33(1H,d,J=7.8Hz), 7.76(1H,d,J=7.8Hz), 7.78(1H,s)

5) 3-(tert-butoxycarbonyl)-2,3,4,5-tetrahydro-1H-3-benzazepine-7-carboxylic acid

2,3,4,5-tetrahydro-1H-3-benzazepine-7-carboxylic acid (300mg) was dissolved in a mixture of IN aqueous sodium hydroxide solution (2.64ml), water (2.5ml) and tetrahydrofuran (2.5ml), and di-tert-butyl dicarbonate (0.33ml) was added, and the mixture was stirred at room temperature for 2 hours. After tetrahydrofuran was evaporated under reduced pressure, the aqueous layer was made acidic with a 5% aqueous potassium hydrogen sulfate solution, and was extracted with ethyl acetate. The extract was washed with brine, dried over anhydrous magnesium sulfate, and the solvent was evaporated under reduced pressure to give the title compound (344mg). $^1$H-NMR($CDCl_3$) δ: 1.49(9H, s), 2.95-3.00(4H,m), 3.55-3.60(4H,m),7.23(1H,d,J=8.4Hz),7.86(1H,s),7.89(1H,d,J=8.4Hz)

6) tert-butyl 7-({[2-(4-benzylpiperazin-1-yl)ethyl]amino}carbonyl)-1,2,4,5-tetrahydro-3H-3-benzazepine-3-carboxylate

Diethyl cyanophosphate (0.086ml) was added to a solution of 3-(tert-butoxycarbonyl)-2,3,4,5-tetrahydro-1H-3-benzazepine-7-carboxylic acid (150mg), 2-(4-benzylpiperazin-1-yl)ethylamine (124mg) and triethylamine (0.079ml) in DMF (5ml). The reaction mixture was stirred at room temperature for 15 hours, and diluted with water. After extracted with ethyl acetate, the extract was washed with brine, dried over anhydrous magnesium sulfate, and the solvent was evaporated under reduced pressure. The residue was purified by silica gel column chromatography (n-hexane/ethyl acetate=1/2) to give the title compound (199mg).
$^1$H-NMR($CDCl_3$) δ: 1.49(9H,s), 2.50-2.65(8H,m), 2.59(2H,t,J=6.0Hz), 2.90-3.00(4H,m), 3.53(2H,s), 3.45-3.60(6H,m), 6.81(1H,m), 7.15-7.35(6H,m), 7.45-7.60(2H,m)
MS(ESI+):493(M+H)

7) N-[2-(4-benzylpiperazin-1-yl)ethyl]-2,3,4,5-tetrahydro-1H-3-benzazepine-7-carboxamide trihydrochloride

tert-butyl 7-({[2-(4-benzylpiperazin-1-yl)ethyl]amino}carbonyl)-1,2,4,5-tetrahydro-3H-3-benzazepine-3-carboxylate (199mg) was treated with a 1N hydrogen chloride solution in ethyl acetate to give an objective compound (126mg).
$^1$H-NMR(DMSO-$d_6$) δ: 3.00-4.00(20H,m), 4.35(2H,m), 7.30(1H,d,J=7.8Hz), 7.40-7.50(3H,m), 7.60-7.70(2H,m), 7.70-7.80(2H,m), 8.84(1H,m)
MS(ESI+):393(M+H)

Compounds of Examples 82-88 were prepared as in Example 81.

Example 82

N-[2-(4-benzhydrylpiperazin-1-yl)ethyl]-2,3,4,5-tetrahydro-1H-3-benzazepine-7-carboxamide trihydrochloride

yield:238mg
$^1$H-NMR(DMSO-$d_6$) δ: 3.00-4.00(21H,m), 7.25-7.40(8H,m), 7.60-7.90(5H,m), 8.89(1H,m)
MS(APCI+):469(M+H)

Example 83

N-[2-[4-(4-chlorobenzyl)piperazin-1-yl]ethyl]-2,3,4,5-tetrahydro-1H-3-benzazepine-7-carboxamide trihydrochloride
yield:198mg
$^1$H-NMP(DMSO-$d_6$) δ: 3.00-4.00(20H,m), 4.31(2H,m), 7.30(1H,d,J=7.8Hz), 7.45-7.80(6H,m), 8.85(1H,m)
MS(APCI+):427(M+H)

Example 84

N-(2-{4-[bis(4-fluorophenyl)methyl]piperazin-1-yl}ethyl)-2,3,4,5-tetrahydro-1H-3-benzazepine-7-carboxamide trihydrochloride

yield:148mg
$^1$H-NMR(DMSO-$d_6$) 5: 3.00-3.45(16H,m), 3.50-3.80(5H,m), 7.15-7.40(5H,m), 7.50-8.00(6H,m), 8.90(1H,m) MS(APCI+):505(M+H)

Example 85

N-[2-(4-benzylpiperazin-1-yl)ethyl]-2,3,4,5-tetrahydro-1H-2-benzazepine-8-carboximde trihydrochloride

yield:139mg
$^1$H-NMR(DMSO-$d_6$) δ: 1.80-2.00(2H,m), 3.00-4.20(18H,m), 4.37(2H,m), 7.30-7.80(6H,m), 7.80-8.05(2H,m), 8.95(1H, m) MS(ESI+):393(M+H)

Example 86

N-[2-(4-benzhydrylpiperazin-1-yl)ethyl]-2,3,4,5-tetrahydro-1H-2-benzazepine-8-carboxamide trihydrochloride

yield:201mg
$^1$H-NMR(DMSO-$d_6$) δ: 1.75-1.95(2H,m), 2.95-4.20(18H,m), 4.35(1H,s), 7.30-7.45(7H,m), 7.60-8.00(6H,m), 8.97(1H, m)
MS(ESI+):469(M+H)

Example 87

N-[2-[4-(4-chlorobenzyl)piperazin-1-yl]ethyl]-2,3,4,5-tetrahydro-1H-2-benzazepine-8-carboxamide trihydrochloride
yield:205mg
$^1$H-NMR(DMSO-$d_6$) δ:1.80-2.00(2H,m), 3.00-4.00(18H,m), 4.36(2H,s), 7.36(1H,d,J=8.0Hz), 7.52(1H,d,J=8.4Hz), 7.69 (1H,d,J=8.4Hz), 7.89(1H,d,J=8.0Hz), 8.00(1H,s), 8.94(1H,m) MS(ESI+):427(M+H)

Example 88

N-(2-{4-[bis(4-fluorophenyl)methyl]piperazin-1-yl}ethyl)-2,3,4,5-tetrahydro-1H-2-benzazepine-8-carboxamide trihydrochloride

yield:325mg
$^1$H-NMR(DMSO-$d_6$) δ: 1.80-2.00(2H,m), 3.00-4.50(19H,m), 7.20-7.40(5H,m), 7.60-8.10(5H,m), 8.97(1H,m)
MS(ESI+):505(M+H)

Example 89

2-benzyl-N-(2-{4-[bis(4-fluorophenyl)methyl]piperazin-1-yl}ethyl)-2,3,4,5-tetrahydro-1H-2-benzazepine-8-carboxamide trihydrochloride

A mixture of 2,3,4,5-tetrahydro-1H-2-benzazepine-8-carboxylic acid (200mg) synthesized according to the same manner as that described in Example 81 1)-4), and benzyl bromide (0.23ml), potassium carbonate (267mg) and DMF (10ml) was stirred at room temperature for 24 hours, and diluted with water. The aqueous layer was washed with ethyl acetate, followed by acidification with 1N hydrochloric acid, and extracted with dichloromethane. The extract was washed with brine, dried over anhydrous magnesium sulfate, and the solvent was evaporated under reduced pressure to obtain 2-benzyl-2,3,4,5-tetrahydro-lH-2-benzazepine-8-carboxylic acid (89mg). From this, the title compound (104mg) was synthesized according to the same manner as that described in Example 81 6)-7).
$^1$H-NMR(DMSO-$d_6$) δ: 1.85-2.05(2H,m), 3.00-4.70(21H,m), 7.23(4H,m), 7.35-7.50(4H,m), 7.60-7.80(6H,m), 7.90-8.00 (2H,m), 8.97(1H,m)
MS(ESI+):595(M+H)

Example 90

N-[2-(4-benzhydrylpiperazin-1-yl)ethyl]-N-benzyl-2,3,4,5-tetrahydro-1H-3-benzazepine-7-carboxamide trihydrochloride

A mixture of 2-(4-benzhydrylpiperazin-1-yl)ethylamine (275mg), benzaldehyde (0.15ml), molecular sieves (1g) and methanol (5ml) was stirred at room temperature for 2 hours. After molecular sieves were filtered off, the filtrate was concentrated under reduced pressure. Sodium tetrahydroborate (56mg) was added to a solution of the resulting residue in methanol-THF (3:2; 5ml), and the mixture was stirred at room temperature for 17 hours. The solvent was

evaporated under reduced pressure. And brine was added to the residue. After extracting with ethyl acetate, the extract was washed with brine, and dried over anhydrous magnesium sulfate. The solvent was evaporated under reduced pressure to give N-[2-(4-benzhydrylpiperazin-1-yl)ethyl]-N-benzylamine (245mg). From this, the title compound (154mg) was synthesized according to the same manner as that described in Example 81 6)-7).
$^1$H-NMR(DMSO-$d_6$) δ: 2.90-4.00(21H,m), 4.58(2H,m), 7.10-7.50(12H,m), 7.50-7.90(3H,m)
MS(ESI+):559(M+H)

Example 91

N-benzyl-N-{2-[4-(4-chlorobenzyl)piperazin-1-yl]ethyl}-2,3,4,5-tetrahydro-1H-3-benzazepine-7-carboxamide trihydrochloride

This was prepared as in Example 90.
$^1$H-NMR(DMSO-$d_6$) δ: 3.00-3.80(20H,m), 4.37(2H,m), 4.59(2H,m), 7.10-7.50(5H,m), 7.53(2H,d,J=8.0Hz), 7.70(2H,d, J=8.0Hz)
MS(ESI+):517(M+H)

Example 92

3-(4-benzylpiperazin-1-yl)-N-(2,3,4,5-tetrahydro-1H-3-benzazepine-7-yl)propionamide trihydrochloride

1) 7-nitro-3-(trifluoroacetyl)-2,3,4,5-tetrahydro-1H-3-benzazepine

Potassium nitrate (229mg) was added to a solution of 3-(trifluoroacetyl)-2,3,4,5-tetrahydro-1H-3-benzazepine (500mg) in sulfuric acid (3ml) under ice-cooling. After stirring for 3 hours under ice-cooling, the mixture was poured into ice-water, and extracted with ethyl acetate. The extract was washed with an aqueous saturated sodium bicarbonate solution and brine, dried over anhydrous magnesium sulfate, and the solvent was evaporated under reduced pressure. The residue was purified by silica gel column chromatography (n-hexane/ethyl acetate=4/1) to obtain the title compound (295mg).
$^1$H-NMR($CDCl_3$) δ: 3.05-3.15(4H,m), 3.70-3.86(4H,m), 7.30-7.38(1H,m), 8.02-8.10(2H,m)
MS(APCI-):287(M-H)

2) 3-(trifluoroacetyl)-2,3,4,5-tetrahydro-1H-3-benzazepine-7-amine

A mixture of 7-nitro-3-(trifluoroacetyl)-2,3,4,5-tetrahydro-1H-3-benzazepine (100mg), tin chloride (II) dihydrate (391mg) and DMF (2ml) was stirred at room temperature for 5 hours. The mixture was diluted with water, and extracted with ethyl acetate. The extract was washed with an aqueous saturated sodium bicarbonate solution and brine, dried over anhydrous magnesium sulfate, and the solvent was evaporated under reduced pressure to give the title compound (85mg).
$^1$H-NMR($CDCl_3$) δ: 2.80-3.00(4H,m), 3.60-3.80(6H,m), 6.45-6.52(2H,m), 6.85-6.98(1H,m)
MS(APCI+):259(M+H)

3) 3-(4-benzylpiperazin-1-yl)-N-[3-(trifluoroacetyl)-2,3,4,5-tetrahydro-1H-3-benzazepine-7-yl]propionamide

Diethyl cyanophosphate (0.050ml) was added to a solution of 3-(trifluoroacetyl)-2,3,4,5-tetrahydro-1H-3-benzazepine-7-amine (77mg), 3-(4-benzylpiperazin-1-yl)propionic acid (105mg) and triethylamine (0.137ml) in DMF (3ml). The reaction mixture was stirred at room temperature for 15 hours, and diluted with water. After extracting with ethyl acetate, the extract was washed with brine, dried over anhydrous magnesium sulfate, and the solvent was evaporated under reduced pressure. The residue was purified by silica gel column chromatography (n-hexane/ethyl acetate=2/3) to give the title compound (71mg).
$^1$H-NMR($CDCl_3$) δ: 2.40-2.80(12H,m), 2.90-3.00(4H,m), 3.95(2H,s), 3.65-3.85(4H,m), 7.00-7.50(8H,m)
MS(APCI+):489(M+H)

4) 3-(4-benzylpiperazin-1-yl)-N-(2,3,4,5-tetrahydro-1H-3-benzazepin-7-yl)propionamide

A 1M aqueous potassium carbonate solution (0.39ml) was added to a solution of 3-(4-benzylpiperazin-1-yl) -N-[3-(trifluoroacetyl)-2,3,4,5-tetrahydro-1H-3-benzazepin-7-yl]propionamide (64mg) in methanol (1ml), and the mixture was stirred at room temperature for 1.5 hours. The methanol was evaporated under reduced pressure, followed

by extraction with ethyl acetate. The extract was washed with brine, dried over anhydrous magnesium sulfate, and the solvent was evaporated under reduced pressure to give the title compound (31mg).
$^1$H-NMR($CDCl_3$) δ: 2.35-2.80(12H,m), 2.85-3.00(8H,m), 3.51(2H,s), 7.03(1H,d,J=8.0Hz), 7.15-7.35(7H,m)
MS(APCI+):393(M+H)

5) 3-(4-benzylpiperazin-1-yl)-N-(2,3,4,5,-tetrahydro-1H-3-benzazepin-7-yl)propionamide trihydrochloride

3-(4-benzylpiperazin-1-yl)-N-(2,3,4,5-tetrahydro-1H-3-benzazepin-7-yl)propionamide (27mg) was treated with a 1N solution of hydrogen chloride in ethyl acetate to give an objective compound (4.0mg).
MS(APCI+):393(M+H)
Compounds of Examples 93 and 94 were prepared as in Example 92

Example 93

3-(4-benzhydrylpiperazin-1-yl)-N-(2,3,4,5-tetrahydro-1H-3-benzazepin-7-yl)propionamide trihydrochloride

yield:24mg
$^1$H-NMR(DMSO-$d_6$) δ: 2.80-3.80(21H,m), 7.10-7.70(13H,m),
10.30(1H,m)
MS(ESI+):469(M+H)

Example 94

3-[4-(4-chlorobenzyl)piperazin-1-yl]-N-(2,3,4,5-tetrahydro-1H-3-benzazepin-7-yl)propionamide trihydrochloride

yield:73mg
$^1$H-NMR(DMSO-$d_6$) δ: 2.80-4.00(20R,m), 4.33(2H,m), 7.12(1H,d,J=8.0Hz), 7.35-7.60(4H,m), 7.60-7.75(2H,m), 10.36(1H,m)
MS(ESI+):427(M+H)
Compounds of Examples 95-106 were prepared as in Example 11.

Example 95

4-(4-benzylpiperizin-1-yl)-1-(2,3,4,5-trtrahydro-1H-2-benzazepin-8-yl)butan-1-one

yield: 41mg
HPLC analysis (Condition B): purity 99% (retention time: 1.675min.)
MS (APCI+): 392 (M+H)

Example 96

4-(4-benzhydrylpiperazin-1-yl)-1-(2,3,4,5-trtrahydro-1H-2-benzazepin-8-yl)butan-1-one

yield: 89mg
HPLC analysis (Condition A): purity 93% (retention time: 2.632min.)
MS (APCI+): 468 (M+H)

Example 97

4-{4-[bis(4-fluorophenyl)methyl]piperazin-1-yl}-1-(2,3,4,5-trtrahydro-1H-2-benzazepin-8-yl)butan-1-one

yield: 40mg
HPLC analysis (Condition A): purity 94% (retention time: 2.779min.)
MS (APCI+): 504 (M+H)

Example 98

4-{4-[(4-chlorophenyl)(phenyl)methyl]piperazin-1-yl}-1-(2,3,4,5-trtrahydro-1H-2-benzazepin-8-yl)butan-1-one

yield: 42mg
HPLC analysis (Condition A): purity 99% (retention time: 2.973min.)
MS (APCI+): 502 (M+H)

Example 99

4-[4-(4-chlorobenzyl)piperazin-1-yl]-1-(2,3,4,5-trtrahydro-1H-2-benzazepin-8-yl)butan-1-one

yield: 11mg
HPLC analysis (Condition A): purity 93% (retention time: 2.073min.)
MS (APCI+): 426 (M+H)

Example 100

4-[4-(1-naphthylmethyl)piperazin-1-yl]-1-(2,3,4,5-trtrahydro-1H-2-benzazepin-8-yl)butan-1-one

yield: 28mg
HPLC analysis (Condition A): purity 96% (retention time: 2.261min.)
MS (APCI+): 442 (M+H)

Example 101

4-[4-(4-fluorobenzyl)piperazin-1-yl]-1-(2,3,4,5-trtrahydro-1H-2-benzazepin-8-yl)butan-1-one

yield: 18mg
HPLC analysis (Condition B): purity 77% (retention time: 1.701min.)
MS (APCI+): 410 (M+H)

Example 102

4-[(2-{[bis(4-fluorophenyl)methyl]amino}ethyl)amino]-1-(2,3,4,5-trtrahydro-1H-2-benzazepin-8-yl)butan-1-one

yield: 18mg
HPLC analysis (Condition A): purity 75% (retention time: 2.667min.)
MS (APCI+): 506 (M+H)

Example 103

4-(4-benzylpiperidin-1-yl)-1-(2,3,4,5-trtrahydro-1H-2-benzazepin-8-yl)butan-1-one

yield: 59mg
HPLC analysis (Condition A): purity 96% (retention time: 2.463min.)
MS (APCI+): 391 (M+H)

Example 104

4-[4-(4-fluorobenzyl)piperidin-1-yl]-1-(2,3,4,5-trtrahydro-1H-2-benzazepin-8-yl)butan-1-one

yield: 43mg
HPLC analysis (Condition A): purity 98% (retention time: 2.538min.)
MS (APCI+): 409 (M+H)

Example 105

4-(4-phenylpiperazin-1-yl)-1-(2,3,4,5-trtrahydro-1H-2-benzazepin-8-yl)butan-1-one

yield: 41mg
HPLC analysis (Condition A): purity 98% (retention time: 2.154min.)
MS (APCI+): 378 (M+H)

Example 106

4-[4-(benzhydryloxy)piperidin-1-yl]-1-(2,3,4,5-trtrahydro-1H-2-benzazepin-8-yl)butan-1-one

yield: 48mg
HPLC analysis (Condition A): purity 99% (retention time:
2.874min.)
MS (APCI+): 483 (M+H)

Vasoactive agents (e.g. prophylactic and therapeutic agent of cardiac infarction, prophylactic and therapeutic agent of heart failure etc.) containing the compound having the GPR14 antagonistic activity in the present invention or a salt thereof as an active ingredient can be prepared, for example, by the following formulations.

Preparation Example

1. Capsule

(1)Compound obtained in Example 1 40mg
(2)Lactose 70mg
(3)Microcrystalline cellulose 9mg
(4)Magnesium stearate 1mg
1 capsule 120mg

(1), (2) and (3) as well as 1/2 of (4) were kneaded, and granulated. To this granule is added the remaining (4), and the whole is capsulated into a gelatin capsule.

2.Tablet

| | |
|---|---|
| (1)Compound obtained in Example 1 | 40mg |
| (2)Lactose | 58mg |
| (3)Corn starch | 18mg |
| (4)Microcrystalline cellulose | 3.5mg |
| (5)Magnesium stearate | 0.5mg |
| 1 Tablet | 120mg |

(1), (2), (3), 2/3 of (4) and 1/2 of (5) are kneaded, and granulated. Then, the remaining (4) and (5) are added to the granule and the mixture is formulated into tablets by compression molding.

Industrial Applicability

The compounds having the GPRI14 antagonistic activity of the present invention [including compounds represented by the formula (I) and the formula (II)] or salts thereof have potent GPR14 antagonistic activity and, therefore, can be used advantageously as a variety of vasoactive agents (preferably vasoconstriction inhibitor) as well as in treatment of various diseases (preferably ischemic myocardial infarction or congestive heart failure).

Sequence Listing Free Text

SEQ ID No: 1

DNA for screening a cDNA encoding a human GPR14 protein

SEQ ID No: 2

DNA for screening a cDNA encoding a human GPR14 protein

**Claims**

1. A GPR14 antagonistic agent comprising a compound represented by the formula (I) :

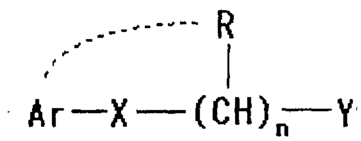

(I)

wherein Ar denotes an optionally substituted aryl group, X denotes a spacer wherein the number of atoms constituting a straight chain moiety is 1 to 4, n denotes an integer of 1 to 10, R is a hydrogen atom or an optionally substituted hydrocarbon group, and may be the same or different in repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

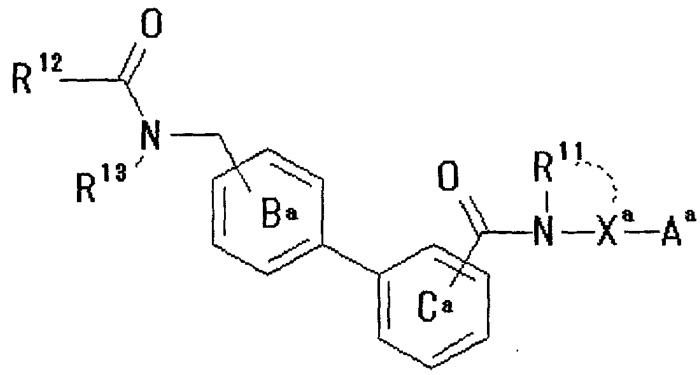

wherein $R^{11}$ denotes a hydrogen atom or an optionally substituted hydrocarbon group, $X^a$ denotes a spacer wherein the number of atoms constituting a straight chain moiety is 1 to 12, $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively.

2. The agent according to claim 1, wherein Ar is an optionally substituted phenyl group.

3. The agent according to claim 1, wherein Ar is a group represented by the formula:

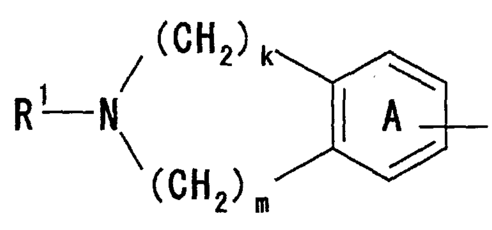

wherein $R^1$ denotes
(1) a hydrogen atom,
(2) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a

nitro group, (iii) a cyano group, (iv) an oxo group, (v) a hydroxyl group, (vi) a $C_{1-6}$alkyl group (this $C_{1-6}$ alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a formyl group, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group having 1 to 6 hetero atoms selected from a nitrogen atom, an oxygen atom and a sulfur atom (this heterocyclic group may be substituted with a substituent group selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group and (xxi') a $C_{1-6}$alkylsulfonyl group (hereinafter, abbreviated as a substituent group P)), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or $C_{1-6}$alkoxy group) or a $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xviii) an amidino group (this amidino group may be mono- or di-substituted with a $C_{1-6}$alkly group or a $C_{6-14}$aryl group (this $C_{6-14}$aryl group may be substituted with a nitro group), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocabonyl and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsufonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphophoryl group, (xxxxii) a $C_{1-4}$alkylenedioxy, (xxxxiii) phenylthio (this phenylthio may be substituted with halogen) and (xxxxiv) phenoxy (this phenoxy may be substituted with halogen), or

(3) an acyl group selected from -(C=O)-$R^{2c}$, -$SO_2$-$R^{2c}$, -SO-$R^{2c}$, - (C=O)N$R^{3c}R^{2c}$, -(C=O)O-$R^{2c}$, -(C=S)O-$R^{2c}$ or - (C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$ alkyl group may be substituted with phenyl), (vii') a $C_{1-6}$alkoxy group (this $C_{1-6}$alkoxy group may be substituted with phenyl, (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may have substituent(s) selected from the substituent group P above), (xxv') phenylthio (this phenylthio may be substituted with halogen) or (xxvi') phenoxy (this phenoxy may be substituted with halogen) (hereainafter, abbreviated as substituent group A). or (iii) a monocyclic or 2 to 4 cyclic heterocyclic group containing 1 to 6 hetero atoms selected from a nitrogen atom, an oxygen atom and sulfur atom (this heterocyclic group may have 1 to 5 substituents selected from the substituent group A above), or $R^{2c}$ and $R^{3c}$ may be bound to each other to form a

5 to 9 membered nitrogen-containing saturated heterocyclic group together with an adjacent nitrogen atom (this nitrogen-containing saturated heterocyclic group may have 1 to 5 substituents selected from the substituent group A above)], ring A denotes a benzene ring optionally having substituent(s) selected from (i) an amino group, (ii) a mono-$C_{1-6}$alkylamino group, (iii) a di-$C_{1-6}$alkylamino group, (iv) a 5 to 7 membered cyclic amino group optionally having 1 to 3 hetero atoms selected from a nitrogen atom, an oxygen atom and a sulfur atom in addition to one nitrogen atom, (v) a $C_1$-$_6$alkyl-carbonylamino group, (vi) an aminocarbonyloxy group, (vii) a mono-$C_1$-$_6$alkylamino-carbonyloxy group, (viii) a di-$C_{1-6}$alkylamino-carbonyloxy group, (ix) a $C_{1-6}$alkylsulfonylamino group, (x) phenyl-$C_{1-6}$alkylamino, (xi) a phenyl-$C_{1-6}$alkyl-sulfonylamino group, (xii) a phenylsulfonylamino group, (xiii) a halogen atom, (xiv) an optionally halogenated $C_1$-$_6$alkyl group, and (vx) an optionally halogenated $C_{1-6}$alkoxy group, k and m denote independently an integer of 0 to 5, and $1 < k+m < 5$.

**4.** The agent according to claim 1, wherein Ar is a group represented by the formula:

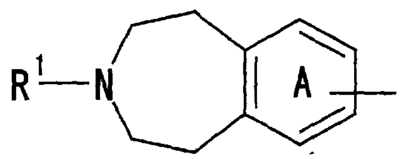

wherein $R^1$ denotes (1) a hydrogen atom,
(2) a straight or branched $C_{1-6}$alkyl group, a straight or branched $C_{2-6}$alkenyl group, a straight or branched $C_2$-$_6$alkynyl group, a $C_{3-8}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group having 1 to 6 hetero atoms selected from a nitrogen atom, an oxygen atom and a sulfur atom(this heterocyclic group may be substituted with substituent (s) selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as a substituent group Q), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group, or a $C_{1-6}$alkoxy group) or a $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono-or di-substituted with a $C_{1-6}$alkyl group or a $C_{6-14}$aryl group (this $C_{6-14}$aryl group may be substituted with a nitro group)), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino) carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or a nitro), (xxxiv) a sulfo group, (xxxv) a sulfino group, (xxxvi)

a sulfeno group, (xxxvii) a $C_{1-6}$alkylsulfo group, (xxxviii) a $C_{1-6}$alkylsulfino, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen), or (3) an acyl group selected from -(C=O)-$R^{2c}$, -$SO_2$-$R^{2c}$, -SO-$R^{2c}$, -(C=O)$NR^{3c}R^{2c}$, -(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx' ) a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii'') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may have substituent(s) selected from the substituent group Q above), (xxv') phenylthio (this phenylthio may be substituted with halogen) or (xxvi') phenoxy (this phenoxy may be substituted with halogen), (hereinafter, abbreviated as substituent group B ), or (iii) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substitutent group B above), or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from the substituent group B above)], ring A denotes a benzene ring optionally having substituent(s) selected from (i) an amino group, (ii) a mono-$C_{1-6}$alkylamino group, (iii) a di-$C_{1-6}$alkylamino group, (iv) a 5 to 7 membered cyclic amino group optionally having 1 to 3 hetero atoms selected from a nitrogen atom, an oxygen atom and a sulfur atom in addition to one nitrogen atom, (v) a $C_{1-6}$alkyl-carbonylamino group, (vi) an aminocarbonyloxy group, (vii) a mono-$C_{1-6}$alkylamino-carbonyloxy group, (viii) a di-$C_{1-6}$alkylamino-carbonyloxy group, (ix) a $C_{1-6}$alkylsulfonylamino group, (x) phenyl-$C_{1-6}$alkylamino, (xi) a phenyl-$C_{1-6}$alkyl-sulfonylamino group, (xii) a phenylsulfonylamino group, (xiii) a halogen atom, (xiv) an optionally halogenated $C_{1-6}$alkyl group and (xv) optionally halogenated $C_{1-6}$alkoxy group.

**5.** The agent according to claim 1, wherein X is a group represented by -CO-, -O-, -$NR^{3a}$-, -$NR^{3a}$CO-, -S-, -SO-, -$SO_2$-, -$SO_2NR^{3a}$-, -$SO_2NHCONR^{3a}$-, -$SO_2NHC(=NH)NR^{3a}$-, -CS-,-$CR^{3a}(R^{3b})$-, -$C(=CR^{3a}(R^{3b}))$-, -$C(=NR^{3a})$- or -$CONR^{3a}$- (wherein $R^{3a}$ and $R^{3b}$ denote independently a hydrogen atom, a cyano group, a hydroxyl group, an amino group, a $C_{1-6}$alkyl group or a $C_{1-6}$alkoxy group).

**6.** The agent according to claim 5, wherein X is a group represented by -CO-, -O-, -$SO_2$-, -$SO_2NR^{3a}$-, -$CR^{3a}(R^{3b})$-or -$CONR^{3a}$- (wherein $R^{3a}$ and $R^{3b}$ denote independently a hydrogen atom, a cyano group, a hydroxyl group, an amino group, a $C_{1-6}$alkyl group or a $C_{1-6}$alkoxy group).

**7.** The agent according to claim 5, wherein X is a group represented by -$CONR^{3a}$- (wherein $R^{3a}$ denotes a hydrogen atom, a cyano group, a hydroxyl group, an amino group, a $C_{1-6}$alkyl group or a $C_{1-6}$alkoxy group).

**8.** The agent according to claim 1, wherein R is a hydrogen atom.

**9.** The agent according to claim 1, wherein Y is a group represented by the formula:

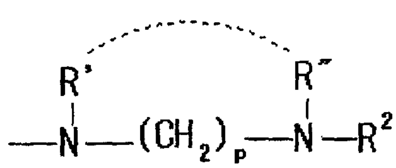

wherein $R^2$ denotes (1) a hydrogen atom,

(2) an acyl group selected from -(C=O)-$R^{2c}$, -$SO_2$-$R^{2c}$, -SO-$R^{2c}$, -(C=O)$NR^{3c}R^{2c}$, -(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i') a halogen atom, (ii') a nitro group, (iii') a cyano group, (iv') an oxo group, (v') a hydroxyl group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx' ) a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) selected from (i") a halogen atom, (ii") a nitro group, (iii") a cyano group, (iv") an oxo group, (v") hydroxyl group, (vi") a $C_{1-6}$alkyl group, (vii") a $C_{1-6}$alkoxy group, (viii") a $C_{1-6}$alkylthio group, (ix") an amino group, (x") a mono-$C_{1-6}$alkylamino group, (xi") a di-$C_{1-6}$alkylamino group, (xii") a 5 to 7 membered cyclic amino group optionally having 1 to 3 hetero atoms selected from a nitrogen atom, an oxygen atom and a sulfur atom in addition to carbon atom and one nitrogen atom, (xiii") a $C_{1-6}$alkyl-carbonylamino group, (xiv") a $C_{1-6}$alkyl-carbonylamino group, (xv") a $C_{1-6}$alkoxy-carbonyl group, (xvi") a carboxyl group, (xvii") a $C_{1-6}$alkyl-carbonyl group, (xviii") a carbamoyl group, (xix") a mono-$C_{1-6}$alkylcarbamoyl group, (xx") a di-$C_{1-6}$alkylcarbamoyl group and (xxi") a $C_{1-6}$alkylsulfonyl group (hereinafter, abbreviated as substituent group R)), (xxv') phenylthio (this phenylthio may be substituted with halogen) or (xxvi') phenoxy (this phenoxy may be substituted with halogen) (hereinafter, abbreviated as substituent group C), or (iii) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group C above), or $R^{2c}$ and $R^{3c}$ may be bound to each other to form an optionally substituted 5 to 9 membered nitrogen-containing saturated heterocyclic group together with an adjacent atom (this nitrogen-containing saturated heterocyclic group may have 1 to 5 substituents selected from the substituent group C above)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group R above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4-(4-nitrophenyl)piperazino] carbonyl, (4-benzylpiperazino)carbonyl, morpholinocarbonyl and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkylcarbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group D), or

(4) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group D above),

p denotes an integer of 1 to 3,

R' and R" denote a hydrogen atom or a $C_{1-6}$alkyl group (this $C_{1-6}$alkyl group may have 1 to 5 substituents selected from the aforementioned substituent group D), or R' and R" may be bound to each other to form a 5 to 9 membered nitrogen-containing heterocyclic ring optionally containing one hetero atom selected from a nitrogen atom, an oxygen atom and a sulfur atom in addition to carbon atom and two nitrogen atoms.

**10.** The agent according to claim 1, wherein, Y is a group represented by the formula:

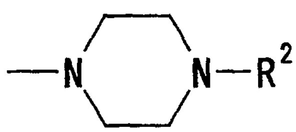

wherein $R^2$ denotes (1) a hydrogen atom, (2) an acyl group selected from -(C=O)$R^{2c}$, -$SO_2$-$R^{2c}$, -SO-$R^{2c}$, -(C=O)$NR^{3c}R^{2c}$,-(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl , a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl or (iii) a monocyclic or 2 to 4 cyclic heterocyclic group containing 1 to 6 hetero atoms selected from a nitrogen atom, an oxygen atom or a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkylcarbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom (this heterocyclic group may be substituted with substituent (s) selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as substituent group S), (xxv) phenylthio (this phenylthio may be substituted with halogen) or (xxvi) phenoxy (this phenoxy may be substituted with halogen)]

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl

group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group S above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or a $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group E), or

(4) a monocyclic or 2 to 4 cyclic heterocyclic group containing 1 to 6 hetero atoms selected from a nitrogen atom, an oxygen atom or a sulfur atom (this heterocyclic group may have 1 to 5 substituents selected from the substituent group E above).

**11.** The agent according to claim 1, wherein Y is a group represented by the formula:

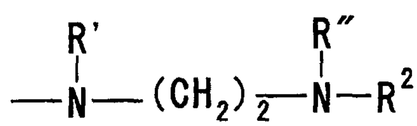

wherein $R^2$ denotes:

(1) a hydrogen atom,

(2) an acyl group selected from -(C=O)-$R^{2c}$, -$SO_2$-$R^{2c}$, -SO-$R^{2c}$, -(C=O)$NR^{3c}R^{2c}$, -(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$Cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group

optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom)(this heterocyclic group may be substituted with substituent(s) group selected from (i') a halogen atom, (ii') a nitro group, (iii') a cyano group, (iv') an oxo group, (v') a hydroxyl group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from an nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as a substituent group T), (xxv) phenylthio (this phenylthio may be substituted with halogen and (xxvi) phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group T above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group, or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4-(4-nitrophenyl)piperazino] carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group F), or

(4) a monocyclic or 2 to 4 cyclic heterocyclic group containing 1 to 6 hetero atoms selected from a nitrogen atom, an oxygen atom and a sulfur atom (this heterocyclic group may have 1 to 5 substituents selected from the substituent group F above),

R' and R" denote a hydrogen atom or a $C_{1-6}$alkyl group respectively (this $C_{1-6}$alkyl group may have 1 to 5 substituents selected from the substituent group F above).

**12.** The agent according to claim 1, wherein Y is a piperidino group (this piperidino group may be substituted with:
(1) a straight or branched $C_{1-6}$alkyl group, a straight or branched $C_{2-6}$alkenyl group, a straight or branched

$C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxyl group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may be substituted with substituent (s) selected from (i') a halogen atom, (ii') a nitro group, (iii') a cyano group, (iv') an oxo group, (v') a hydroxy group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a di-$C_{1-6}$alkylcarbamoyl group and (xxi') a $C_{1-6}$alkylsulfonyl group (hereinafter, abbreviated as substituent group U), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro group), (xxix) a guanidino group (this guanidino group may be mono- or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl)piperidino]carbonyl, (4-methylpiperazino)carbonyl, (4-phenylpiperazino)carbonyl, [4-(4-nitrophenyl)piperazino]carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) an aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkyl-carbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group G),

(2) an acyl group selected from $-(C=O)-R^{2c}$, $-SO_2-R^{2c}$, $-SO-R^{2c}$, $-(C=O)NR^{3c}R^{2c}$, $-(C=O)O-R^{2c}$, $-(C=S)O-R^{2c}$ or $-(C=S)NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom) (this heterocyclic group may be substituted with substituent(s) selected from the substituent group U above), (xxv) phenylthio (this phenylthio may be substituted with halogen) and (xxvi) phenoxy (this phenoxy may be substituted with halogen) ], or
(3) a monocyclic or a 2 to 4 cyclic heterocyclic group containing 1 to 6 hetero atoms selected from a nitrogen atom, an oxygen atom and a sulfur atom (this heterocyclic group may have 1 to 5 substituents selected from the substituent group G above).

**13.** The agent according to claim 1, wherein n is an integer of 1 to 5.

**14.** The agent according to claim 1, which is a vasoconstriction inhibitor.

**15.** The agent according to claim 1, which is a prophylactic and/or therapeutic agent of hypertension, arteriosclerosis, cardiac hypertrophy, cardiac infarction or heart failure.

**16.** A compound represented by the formula (II):

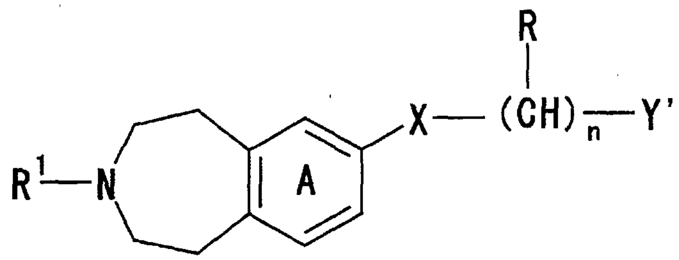

(II)

wherein $R^1$ denotes a hydrogen atom, an optionally substituted hydrocarbon group or an optionally substituted acyl group, ring A denotes a benzene ring optionally further having a substituent, X denotes a spacer wherein the number of atoms constituting a straight chain moiety is 1 to 4 (provided that -CO- is excluded), n denotes an integer of 1 to 10, R is a hydrogen atom or an optionally substituted hydrocarbon group and may be the same or different in the repetition of n, or R may be bound to ring A or a substituent of ring A to form a ring, and Y' denotes an optionally substituted amino group, or a salt thereof.

**17.** A prodrug of the compound or a salt thereof according to claim 16.

**18.** The compound according to claim 16, wherein $R^1$ is a hydrogen atom or an optionally substituted hydrocarbon group.

**19.** The compound according to claim 16, wherein $R^1$ is a hydrogen atom.

**20.** The compound according to claim 16, wherein X is a group represented by the formula: -O-, $-NR^{3a}-$, $-NR^{3a}CO-$, -S-, -SO-, $-SO_2-$, $-SO_2NR^{3a}-$, $-SO_2NHCONR^{3a}-$, $-SO_2NHC(=NH)NR^{3a}-$, -CS-, $-CR^{3a}(R^{3b})-$, $-C(=CR^{3a}(R^{3b}))-$, $-C(=NR^{3a})-$ or $-CONR^{3a}-$ (wherein $R^{3a}$ and $R^{3b}$ denote independently a hydrogen atom, a cyano group, a hydroxy group, an amino group, a $C_{1-6}$alkyl group or a $C_{1-6}$alkoxy group respectively).

**21.** The compound according to claim 20, wherein X is a group represented by the formula: $-SO_2NR^{3a}-$, $-CONR^{3a}-$ or-$CR^{3a}(R^{3b})-$ (wherein $R^{3a}$ and $R^{3b}$ denote independently a hydrogen atom, a cyano group, a hydroxy group, an amino group, a $C_{1-6}$alkyl group or a $C_{1-6}$alkoxy group respectively).

**22.** The compound according to claim 20, wherein X is a group represented by the formula: $-CONR^{3a}-$ (wherein $R^{3a}$ denotes a hydrogen atom, a cyano group, a hydroxy group, an amino group, a $C_{1-6}$alkyl group or a $C_{1-6}$alkoxy group).

**23.** The compound according to claim 16, wherein R is a hydrogen atom.

**24.** The compound according to claim 16, wherein Y' is a group represented by the formula:

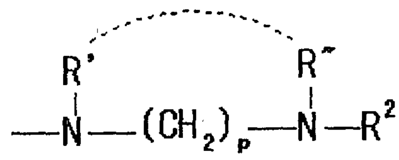

wherein $R^2$ denotes (1) a hydrogen atom,

(2) an acyl group selected from -(C=O)-$R^{2c}$, -$SO_2$-$R^{2c}$, -SO-$R^{2c}$, - (C=O)$NR^{3}CR^{2c}$, -(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or a 2 to 4 cyclic heterocyclic group containing 1 to 6 hetero atoms selected from a nitrogen atom, an oxygen atom and a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i') a halogen atom, (ii') a nitro group, (iii') a cyano group, (iv') an oxo group, (v') a hydroxy group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-sulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group, (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) selected from (i") a halogen atom, (ii") a nitro group, (iii") a cyano group, (iv") an oxo group, (v") hydroxy group, (vi") a $C_{1-6}$alkyl group, (vii") a $C_{1-6}$alkoxy group, (viii") a $C_{1-6}$alkylthio group, (ix") an amino group, (x") a mono-$C_{1-6}$alkylamino group, (xi") a di-$C_{1-6}$alkylamino group, (xii") a 5 to 7 membered cyclic amino group optionally having 1 to 3 hetero atoms selected from a nitrogen atom, an oxygen atom and a sulfur atom in addition to carbon atom and one nitrogen atom, (xiii") a $C_{1-6}$alkyl-carbonylamino group, (xiv") a $C_{1-6}$alkyl-carbonylamino group, (xv") a $C_{1-6}$alkoxy-carbonyl group, (xvi") a carboxyl group, (xvii") a $C_{1-6}$alkyl-carbonyl group, (xviii") a carbamoyl group, (xix") a mono-$C_{1-6}$alkylcarbamoyl group, (xx") a di-$C_{1-6}$alkylcarbamoyl group and (xxi") a $C_{1-6}$alkylsulfonyl group (hereinafter, abbreviated as substituent group V)), (xxv') phenylthio (this phenylthio may be substituted with halogen) or (xxvi') phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with halogen or phenyl), (vii) a $C_{1-6}$alkoxy group (this $C_{1-6}$alkoxy group may be substituted with halogen or phenyl), (viii) a $C_{1-6}$alkylthio group (this $C_{1-6}$alkylthio group may be substituted with halogen or phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) formyl, (xviii) a $C_{1-6}$alkyl-carbonyl group, (xix) a carbamoyl group, (xx) a mono-$C_{1-6}$alkyl-carbamoyl group, (xxi) a di-$C_{1-6}$alkyl-carbamoyl group, (xxii) a $C_{1-6}$alkylsulfonyl group, (xxiii) a $C_{1-6}$alkoxy-carbonyl-$C_{1-6}$alkyl group, (xxiv) a carboxyl-$C_{1-6}$alkyl group, (xxv) a monocyclic or 2 to 4 cyclic heterocyclic group containing 1 to 6 hetero atoms selected from a nitrogen atom, an oxygen atom and a sulfur atom (this heterocyclic group may have substituent(s) selected from the substituent group V above), (xxvi) an ureido group (this ureido group may be substituted with a $C_{1-6}$alkyl group, a $C_{6-14}$aryl group (this $C_{6-14}$aryl group may be substituted with halogen, a $C_{1-6}$alkyl group, a halo$C_{1-6}$alkyl group or a $C_{1-6}$alkoxy group) or $C_{7-16}$aralkyl group), (xxvii) a thioureido group (this thioureido group may be substituted with a $C_{1-6}$alkyl group, a $C_{6-14}$aryl group (this $C_{6-14}$aryl group may be substituted with halogen, a $C_{1-6}$alkyl group or a $C_{1-6}$alkoxy group) or a $C_{7-16}$aralkyl group), (xxviii) an amidino group (this amidino group may be mono- or di-substituted with a $C_{1-6}$alkyl group or a $C_{6-14}$aryl group (this $C_{6-14}$aryl group may be substituted with a nitro

group), (xxix) a guanidino group (this guanidino group may be mono-or di-substituted with a $C_{1-6}$alkyl group), (xxx) a cyclic aminocarbonyl group selected from pyrrolidinocarbonyl, piperidinocarbonyl, (4-methylpiperidino)carbonyl, (4-phenylpiperidino)carbonyl, (4-benzylpiperidino)carbonyl, (4-benzoylpiperidino)carbonyl, [4-(4-fluorobenzoyl) piperidino]carbonyl, (4-methylpiperazino)carbonyl, (4-phenylpiperazino)carbonyl, [4-(4-nitrophenyl)piperazino] carbonyl, (4-benzylpiperazino)carbonyl, morpholinocarbonyl, and thiomorpholinocarbonyl, (xxxi) an aminothiocarbonyl group (this aminothiocarbonyl group may be mono- or di-substituted with a $C_{1-6}$alkyl group), (xxxii) aminosulfonyl (this aminosulfonyl may be mono- or di-substituted with a $C_{1-6}$alkyl group), (xxxiii) phenylsulfonylamino (this phenylsulfonylamino may be substituted with a $C_{1-6}$alkyl group, halogen, a $C_{1-6}$alkoxy group, a $C_{1-6}$alkylcarbonylamino group or nitro), (xxxiv) a sulfo group, (xxxv) a sulfino group, (xxxvi) a sulfeno group, (xxxvii) a $C_{1-6}$alkylsulfo group, (xxxviii) a $C_{1-6}$alkylsulfino group, (xxxix) a $C_{1-6}$alkylsulfeno group, (xxxx) a phosphono group, (xxxxi) a di$C_{1-6}$alkoxyphosphoryl group, (xxxxii) $C_{1-4}$alkylenedioxy, (xxxxiii) phenylthio (this phenylthio may be substituted with halogen) or (xxxxiv) phenoxy (this phenoxy may be substituted with halogen) (hereinafter, abbreviated as a substituent group H), or

(4) a monocyclic or 2 to 4 cyclic heterocyclic group containing 1 to 6 hetero atoms selected from a nitrogen atom, an oxygen atom and a sulfur atom (this heterocyclic group may have 1 to 5 substituents selected from the substituent group H above),

p denotes an integer of 1 to 3,

R' and R" each denote a hydrogen atom or a $C_{1-6}$alkyl group (this $C_{1-6}$alkyl group may have 1 to 5 substituents selected from the aforementioned substituent group H), or R' and R" may be bound to form a 5 to 9 membered nitrogen-containing heterocyclic ring optionally containing one hetero atom selected from a nitrogen atom, an oxygen atom and a sulfur atom in addition to carbon atom and two nitrogen atoms.

**25.** The compound according to claim 16, wherein, Y' is a group represented by the formula:

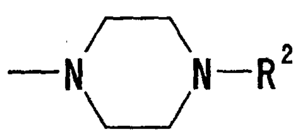

wherein $R^2$ denotes (1) a hydrogen atom,

(2) an acyl group selected from -(C=O)-$R^{2c}$, -$SO_2$-$R^{2c}$, -SO-$R^{2c}$, -(C=O)N$R^{3c}R^{2c}$, -(C=O)O-$R^{2c}$, -(C=S)O-$R^{2c}$ or -(C=S)N$R^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl , a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl or (iii) a monocyclic or 2 to 4 cyclic heterocyclic group containing 1 to 6 hetero atoms selected from a nitrogen atom, an oxygen atom and a sulfur atom, or $R^{2c}$ and $R^{3c}$ may be bound to each other to form a 5 to 9 membered nitrogen-containing saturated heterocyclic group together with an adjacent nitrogen atom (this nitrogen-containing saturated heterocyclic group may have 1 to 5 substituents selected from (i) a halogen atom, (ii) a nitro group, (iii) a cyano group, (iv) an oxo group, (v) a hydroxy group, (vi) a $C_{1-6}$alkyl group (this $C_{1-6}$alkyl group may be substituted with phenyl), (vii) a $C_{1-6}$alkoxy group (this $C_{1-6}$alkoxy group may be substituted with phenyl), (viii) a $C_{1-6}$alkylthio group (this $C_{1-6}$alkylthio group may be substituted with phenyl), (ix) an amino group, (x) a mono-$C_{1-6}$alkylamino group, (xi) a di-$C_{1-6}$alkylamino group, (xii) a 5 to 7 membered cyclic amino group optionally having 1 to 3 hetero atoms selected from a nitrogen atom, an oxygen atom and a sulfur atom in addition to carbon atom and one nitrogen atom, (xiii) a $C_{1-6}$alkyl-carbonylamino group, (xiv) a $C_{1-6}$alkyl-sulfonylamino group, (xv) a $C_{1-6}$alkoxy-carbonyl group, (xvi) a carboxyl group, (xvii) a $C_{1-6}$alkyl-carbonyl group, (xviii) a carbamoyl group, (xix) a mono-$C_{1-6}$alkyl-carbamoyl group, (xx) a di-$C_{1-6}$alkyl-carbamoyl group, (xxi) a $C_{1-6}$alkylsulfonyl group, (xxii) a $C_{1-6}$alkoxy-carbonyl-$C_{1-6}$alkyl group, (xxiii) a carboxyl-$C_{1-6}$alkyl group, (xxiv) a 4 to 14 membered heterocyclic group containing 1 to 4 hetero atoms selected from a nitrogen atom, an oxygen atom and a sulfur atom (this heterocyclic group may be substituted with substituent(s) selected from (i') a halogen atom, (ii') a nitro group, (iii') a cyano group, (iv') an oxo group, (v') a hydroxy group, (vi') a $C_{1-6}$alkyl group, (vii') a $C_{1-6}$alkoxy group, (viii') a $C_{1-6}$alkylthio group, (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkyl-carbonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkylcarbamoyl group, (xx') a

di-$C_{1-6}$alkylcarbamoyl group and (xxi') a $C_{1-6}$alkylsulfonyl group), (xxv) phenylthio (this phenylthio may be substituted with halogen) or (xxvi) phenoxy (this phenoxy may be substituted with halogen)],

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituent groups selected from (i) a halogen atom, (ii) a nitro group, (iii) a cyano group, (iv) a hydroxy group, (v) a $C_{1-6}$alkyl group and (vi) a $C_{1-6}$alkoxy group,or

(4) a monocyclic or 2 to 4 cyclic heterocyclic group containing 1 to 6 hetero atoms selected from a nitrogen atom, an oxygen atom or a sulfur atom].

**26.** The compound according to claim 25, wherein $R^2$ is a $C_{7-16}$aralkyl group optionally substituted with a halogen atom.

**27.** The compound according to claim 25, wherein $R^2$ is benzyl optionally substituted with a halogen atom, or diphenylmethyl optionally substituted with a halogen atom.

**28.** The compound according to claim 16, wherein Y' is a group represented by the formula:

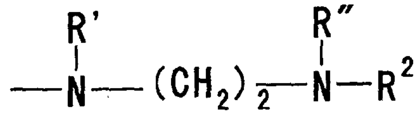

wherein $R^2$ denotes:

(1) a hydrogen atom,

(2) an acyl group selected from -(C=O)-$R^{2c}$, -$SO_2$-$R^{2c}$, -SO-$R^{2c}$, -(C=O)$NR^{3c}R^{2c}$, -(C=O)O-$R^{2c}$, -(C=S)O-$R^{2c}$ or -(C=S)$NR^{3c}R^{2c}$ [$R^{2c}$ and $R^{3c}$ are the same or different and denote (i) a hydrogen atom, (ii) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, or (iii) a monocyclic or 2 to 4 cyclic heterocyclic group containing 1 to 6 hetero atoms selected from a nitrogen atom, an oxygen atom and sulfur atom, or $R^{2c}$ and $R^{3c}$ may be bound to each other to form an optionally substituted 5 to 9 membered nitrogen-containing saturated heterocyclic group together with an adjacent nitrogen atom (this nitrogen-containing saturated heterocyclic group may have 1 to 5 substituents selected from (i') a halogen atom, (ii') a nitro group, (iii') a cyano group, (iv') an oxo group, (v') a hydroxy group, (vi') a $C_{1-6}$alkyl group (this $C_{1-6}$alkyl group may be substituted with phenyl), (vii') a $C_{1-6}$alkoxy group (this $C_{1-6}$alkoxy group may be substituted with phenyl), (viii') a $C_{1-6}$alkylthio group (this $C_{1-6}$alkylthio group may be substituted with phenyl), (ix') an amino group, (x') a mono-$C_{1-6}$alkylamino group, (xi') a di-$C_{1-6}$alkylamino group, (xii') a 5 to 7 membered cyclic amino group optionally having 1 to 3 hetero atoms selected from a nitrogen atom, an oxygen atom and a sulfur atom in addition to carbon atom and one nitrogen atom, (xiii') a $C_{1-6}$alkyl-carbonylamino group, (xiv') a $C_{1-6}$alkylsulfonylamino group, (xv') a $C_{1-6}$alkoxy-carbonyl group, (xvi') a carboxyl group, (xvii') a $C_{1-6}$alkyl-carbonyl group, (xviii') a carbamoyl group, (xix') a mono-$C_{1-6}$alkyl-carbamoyl group, (xx') a di-$C_{1-6}$alkyl-carbamoyl group, (xxi') a $C_{1-6}$alkylsulfonyl group (xxii') a $C_{1-6}$alkoxy-carbonyl-$C_{1-6}$alkyl group, (xxiii') a carboxyl-$C_{1-6}$alkyl group, (xxiv') a 4 to 14 membered heterocyclic group having 1 to 4 hetero atoms selected from a nitrogen atom, an oxygen atom and a sulfur atom (this heterocyclic group may be substituted with substituent(s) group selected from (i") a halogen atom, (ii") a nitro group, (iii") a cyano group, (iv") an oxo group, (v") a hydroxy group, (vi") a $C_{1-6}$alkyl group, (vii") a $C_{1-6}$alkoxy group, (viii") a $C_{1-6}$alkylthio group, (ix") an amino group, (x") a mono-$C_{1-6}$alkylamino group, (xi") a di-$C_{1-6}$alkylamino group, (xii") a 5 to 7 membered cyclic amino group optionally having 1 to 3 hetero atoms selected from a nitrogen atom, an oxygen atom and a sulfur atom in addition to carbon atom and one nitrogen atom, (xiii") a $C_{1-6}$alkyl-carbonylamino group, (xiv") a $C_{1-6}$alkyl-carbonylamino group, (xv") a $C_{1-6}$alkoxy-carbonyl group, (xvi") a carboxyl group, (xvii") a $C_{1-6}$alkyl-carbonyl group, (xviii") a carbamoyl group, (xix") a mono-$C_{1-6}$alkylcarbamoyl group, (xx") a di-$C_{1-6}$alkylcarbamoyl group and (xxi") a $C_{1-6}$alkylsulfonyl group), (xxv') phenylthio (this phenylthio may be substituted with halogen) or (xxvi') phenoxy (this phenoxy may be substituted with halogen)];,

(3) a straight or branched $C_{1-6}$alkyl group, a straight or branched $C_{2-6}$alkenyl group, a straight or branched $C_{2-6}$alkynyl group, a $C_{3-6}$cycloalkyl group, a bridged cyclic $C_{8-14}$ saturated hydrocarbon group, a $C_{6-14}$aryl group, a $C_{7-16}$aralkyl group, a $C_{6-14}$aryl-$C_{2-12}$alkenyl group, a $C_{6-14}$aryl-$C_{2-12}$alkynyl group, a $C_{3-7}$cycloalkyl-$C_{1-6}$alkyl group, biphenyl or biphenyl-$C_{1-10}$alkyl, each optionally having 1 to 5 substituents selected from (i) a halogen atom, (ii) a nitro group, (iii) a cyano group, (iv) a hydroxyl group, (v) a $C_{1-6}$alkyl group and (vi) a $C_{1-6}$alkoxy group, or

(4) a monocyclic or 2 to 4 cyclic heterocyclic group containing 1 to 6 hetero atoms selected from a nitrogen atom, an oxygen atom or a sulfur atom,
R' and R" each denote a hydrogen atom or a $C_{1-6}$alkyl group.

**29.** The compound according to claim 16, wherein Y' is a piperidino group (this piperidino group may be substituted with (i) phenyl-$C_{1-6}$alkyl optionally substituted with $C_{1-6}$alkyl, $C_{1-6}$alkoxy, halogen atom, nitro, mono- or di-$C_{1-6}$alkyl-carbamoyloxy, hydroxyl, cyano, carboxyl, $C_{1-6}$alkoxycarbonyl, carbamoyl, cyclic aminocarbonyl, amino, $C_{1-6}$alkylcarbonylamino, phenylsulfonylamino, $C_{1-6}$alkylsulfonylamino, amidino, ureido or heterocycle, (ii) $C_{1-6}$alkyl group optionally substituted with halogen atom, hydroxyl, $C_{1-6}$alkoxy, amino, mono- or di-$C_{1-6}$alkylamino, carboxyl, cyano or $C_{1-6}$alkoxy-carbonyl, or (iii) $C_{1-6}$alkylcarbonyl group optionally substituted with mono or di-$C_{1-6}$alkylamino or $C_{1-6}$alkoxy-carbonyl.

**30.** The compound according to claim 16, wherein n is an integer of 1 to 5.

**31.** N-[2-(4-benzhydrylpiperazin-1-yl)ethyl]-2,3,4,5-tetrahydro-1H-3-benzazepine-7-carboxamide or a salt thereof.

**32.** N-[2-[4-(4-chlorobenzyl)piperazin-1-yl]ethyl]-2,3,4,5-tetrahydro-1H-3-benzazepine-7-carboxamide or a salt thereof.

**33.** N-[2-{4-[bis(4-fluorophenyl)methyl]-1-piperazinyl}ethyl]-2,3,4,5-tetrahydro-1H-3-benzazepine-7-carboxamide or a salt thereof.

**34.** A pharmaceutical composition comprising the compound according to claim 16 or a salt thereof or a prodrug thereof.

**35.** A GPR14 antagonistic agent comprising the compound according to claim 16 or a salt thereof or a prodrug thereof.

**36.** The composition according to claim 34, which is a vasoconstriction inhibitor.

**37.** The composition according to claim 34, which is a prophylactic and/or therapeutic agent of hypertension, arteriosclerosis, cardiac hypertrophy, cardiac infarction or heart failure.

**38.** A GPR14 antagonizing method, which comprises: administering to a mammal an effective dose of a compound represented by the formula (I):

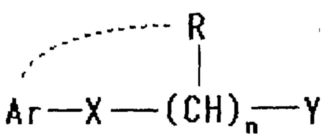

(I)

wherein Ar denotes an optionally substituted aryl group, X denotes a spacer wherein the number of atoms constituting a straight chain moiety is 1 to 4, n denotes an integer of 1 to 10, R denotes a hydrogen atom or an optionally substituted hydrocarbon group and may be the same or different in the repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

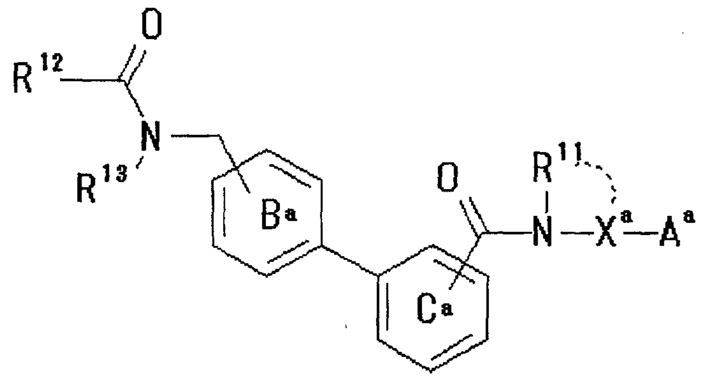

wherein $R^{11}$ denotes a hydrogen atom or an optionally substituted hydrocarbon group, $X^a$ denotes a spacer wherein the number of atoms constituting a straight chain moiety is 1 to 12 chain moiety , $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively.

**39.** Use of a compound represented by the formula (I) :

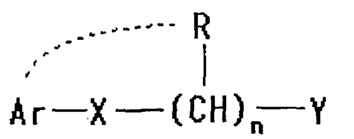

(I)

wherein Ar denotes an optionally substituted aryl group, X denotes a spacer wherein the number of atoms constituting a straight chain moiety is 1 to 4, n denotes an integer of 1 to 10, R denotes a hydrogen atom or an optionally substituted hydrocarbon group and may be the same or different in the repetition of n, or R may be bound to Ar or a substituent of Ar to form a ring, Y denotes an optionally substituted amino group or an optionally substituted nitrogen-containing heterocyclic group, or a salt thereof, provided that a compound having the following formula is excluded:

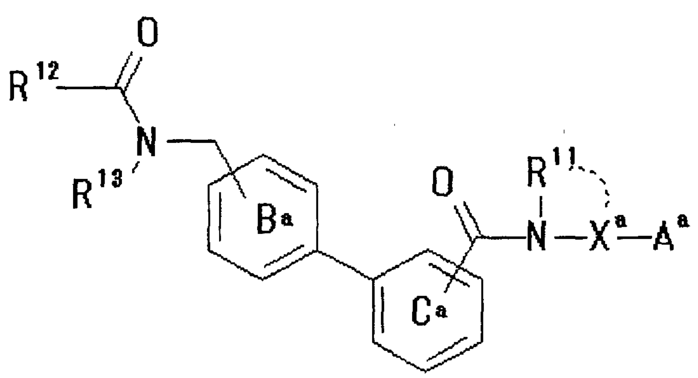

wherein $R^{11}$ denotes a hydrogen atom or an optionally substituted hydrocarbon group, $X^a$ denotes a spacer wherein the number of atoms constituting a straight chain moiety is 1 to 12, $R^{11}$ and $X^a$ may be bound to form a ring, $A^a$ denotes an optionally substituted amino group or an optionally substituted nitrogen-containing heterocyclic group, $R^{12}$ denotes an optionally substituted hydrocarbon group or an optionally substituted amino group, $R^{13}$ denotes an optionally substituted hydrocarbon group, and ring $B^a$ and ring $C^a$ denote an optionally further substituted benzene ring, respectively, for the manufacture of a GPR14 antagonistic agent.

**40.** A process for manufacturing a compound represented by the formula:

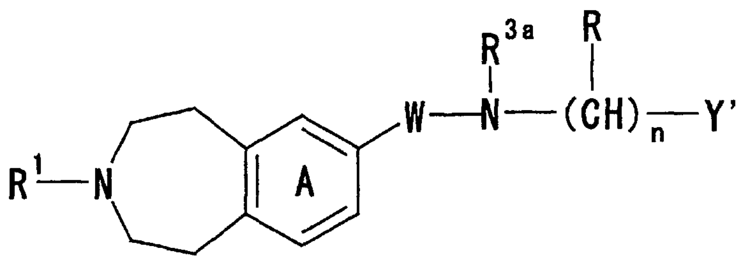

wherein $R^1$ denotes the same meaning as that described in claim 1, W denotes $-SO_2-$ or -CO-, $R^{3a}$ denotes a hydrogen atom, a cyano group, a hydroxyl group, an amino group, a $C_{1-6}$alkyl group or a $C_{1-6}$alkoxy group, R denotes a hydrogen atom or an optionally substituted hydrocarbon group, Y' denotes an optionally substituted amino group, and n denotes an integer of 1 to 10, or a salt thereof, which comprises: reacting a compound represented by the formula:

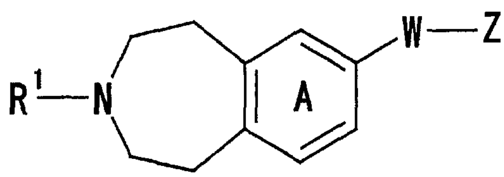

wherein Z denotes a leaving group and other symbols denote the same meanings as those described above, or a salt thereof with a compound represented by the formula:

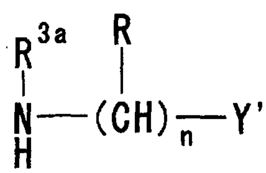

wherein respective symbols denote the same meanings as those described above, or a salt thereof.

# INTERNATIONAL SEARCH REPORT

International application No.
PCT/JP01/05784

**A. CLASSIFICATION OF SUBJECT MATTER**

Int.Cl[7] C07D223/16, 401/12, 405/12, 413/12, A61K31/55, A61P9/08, 9/10, 9/12, 43/00

*According to International Patent Classification (IPC) or to both national classification and IPC*

**B. FIELDS SEARCHED**

Minimum documentation searched (classification system followed by classification symbols)

Int.Cl[7] C07D223/16, 401/12, 405/12, 413/12, A61K31/55, A61P9/08, 9/10, 9/12, 43/00

Documentation searched other than minimum documentation to the extent that such documents are included in the fields searched

| | | | |
|---|---|---|---|
| Jitsuyo Shinan Koho | 1926-1992 | Toroku Jitsuyo Shinan Koho | 1994-1996 |
| Kokai Jitsuyo Shinan Koho | 1971-1992 | Jitsuyo Shinan Toroku Koho | 1996-2001 |

Electronic data base consulted during the international search (name of data base and, where practicable, search terms used)

CA (STN), REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 98/46590 A1 (Takeda Chemical Industries, Ltd.), 22 October, 1998 (22.10.98), Full text & JP 11-310532 A | 1-37,39,40 |
| A | EP 560235 A1 (Takeda Chemical Industries, Ltd.), 15 September, 1993 (15.09.93), Full text & JP 6-166676 A | 1-37,39,40 |
| A | EP 487071 A1 (Takeda Chemical Industries, Ltd.), 27 May, 1992 (27.05.92), Full text & JP 5-140149 A | 1-37,39,40 |
| A | WO 00/23437 A1 (Takeda Chemical Industries, Ltd.), 27 April, 2000 (27.04.00), Full text & JP 2000-186088 A | 1-37,39,40 |

☒ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| | |
|---|---|
| Date of the actual completion of the international search<br>28 September, 2001 (28.09.01) | Date of mailing of the international search report<br>09 October, 2001 (09.10.01) |
| Name and mailing address of the ISA/<br>Japanese Patent Office<br><br>Facsimile No. | Authorized officer<br><br>Telephone No. |

Form PCT/ISA/210 (second sheet) (July 1992)

**INTERNATIONAL SEARCH REPORT**

International application No.
PCT/JP01/05784

**Box I Observations where certain claims were found unsearchable (Continuation of item 1 of first sheet)**

This international search report has not been established in respect of certain claims under Article 17(2)(a) for the following reasons:

1. ☒ Claims Nos.: 38
because they relate to subject matter not required to be searched by this Authority, namely:

Claim 38 involves a method for treatment of the human body by surgery or therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP01/05784

Claims 1 to 15 involve an extremely large number of compounds. However, the compounds which are supported by the description in the meaning of Article 6 of the PCT and are disclosed in the meaning of Article 5 of the PCT are limited to an extremely small part of the compounds claimed.

A search was hence made with respect to the part which is supported by and disclosed in the description, that is, the compounds in which Ar is benzazepine and R is not bonded to Ar or a substituent of Ar to form a ring.

A complete search was made with respect to claims 16-37, 39, and 40.

Form PCT/ISA/210 (extra sheet) (July 1992)

**INTERNATIONAL SEARCH REPORT**

International application No.
PCT/JP01/05784

C (Continuation). DOCUMENTS CONSIDERED TO BE RELEVANT

| Category* | Citation of document, with indication, where appropriate, of the relevant passages | Relevant to claim No. |
|---|---|---|
| A | WO 00/32627 A1 (Takeda Chemical Industries, Ltd.), 08 June, 2000 (08.06.00), Full text & JP 2001-128688 A | 1-37,39,40 |

Form PCT/ISA/210 (continuation of second sheet) (July 1992)

Sequence Listing

<110> Takeda Chemical Industries, Ltd.

<120> Vasoactive agent

<130> 662683

<150> JP 2000-206865

<151> 2000-07-04

<160> 4

<210> 1

<211> 37

<212> DNA

<213> Artificial Sequence

<220>

<223> Designed DNA used for screening cDNA encoding human GPR14 protein

<400> 1

TCGTGAGTCG ACCACCATGG CGCTGACCCC CGAGTCC 37

<210> 2

<211> 33

<212> DNA

<213> Artificial Sequence

<220>

<223> Designed DNA used for screening cDNA encoding human GPR14 protein

<400> 2

GCCTGGACTA GTGCCGCCCC TCCGCGTGCT CAC 33

<210> 3

<211> 1215

<212> DNA

<213> Human

<400> 3

TCGTGAGTCG ACCACCATGG CGCTGACCCC CGAGTCCCCG AGCAGCTTCC CTGGGCTGGC 60

CGCCACCGGC AGCTCTGTGC CGGAGCCGCC TGGCGGCCCC AACGCAACCC TCAACAGCTC 120

CTGGGCCAGC CCGACCGAGC CCAGCTCCCT GGAGGACCTG GTGGCCACGG GCACCATTGG 180

GACTCTGCTG TCGGCCATGG GCGTGGTGGG CGTGGTGGGC AACGCCTACA CGCTGGTGGT 240

CACCTGCCGC TCCCTGCGTG CGGTGGCCTC CATGTACGTC TACGTGGTCA ACCTGGCGCT 300

GGCCGACCTG CTGTACCTGC TCAGCATCCC CTTCATCGTG GCCACCTACG TCACCAAGGA 360

GTGGCACTTC GGGGACGTGG GCTGCCGCGT GCTCTTCGGC CTGGACTTCC TGACCATGCA 420
CGCCAGCATC TTCACGCTGA CCGTCATGAG CAGCGAGCGC TACGCTGCGG TGCTGCGGCC 480
GCTGGACACC GTGCAGCGCC CCAAGGGCTA CCGCAAGCTG CTGGCGCTGG GCACCTGGCT 540
GCTGGCGCTG CTGCTGACGC TGCCCGTGAT GCTGGCCATG CGGCTGGTGC GCCGGGGTCC 600
CAAGAGCCTG TGCCTGCCCG CCTGGGGCCC GCGCGCCCAC CGCGCCTACC TGACGCTGCT 660
CTTCGCCACC AGCATCGCGG GGCCCGGGCT GCTCATCGGG CTGCTCTACG CGCGCCTGGC 720
CCGCGCCTAC CGCCGCTCGC AGCGCGCCTC CTTCAAGCGG GCCCGGCGGC CGGGGGCGCG 780
CGCGCTGCGC CTGGTGCTGG GCATCGTGCT GCTCTTCTGG GCCTGCTTCC TGCCCTTCTG 840
GCTGTGGCAG CTGCTCGCCC AGTACCACCA GGCCCCGCTG GCGCCGCGGA CGGCGCGCAT 900
CGTCAACTAC CTGACCACCT GCCTCACCTA CGGCAACAGC TGCGCCAACC CCTTCCTCTA 960
CACGCTGCTC ACCAGGAACT ACCGCGACCA CCTGCGCGGC CGCGTGCGGG GCCCGGGCAG 1020
CGGGGGAGGC CGGGGGCCCG TTCCCTCCCT GCAGCCCCGC GCCCGCTTCC AGCGCTGTTC 1080
GGGCCGCTCC CTGTCTTCCT GCAGCCCACA GCCCACTGAC AGCCTCGTGC TGGCCCCAGC 1140
GGCCCCGGCC CGACCTGCCC CCGAGGGTCC CAGGGCCCCG GCGTGAGCAC GCGGAGGGGC 1200
GGCACTAGTC CAGGC 1215

<210> 4

<211> 389

<212> PRT

<213> Human

<400> 4

Met Ala Leu Thr Pro Glu Ser Pro Ser Ser Phe Pro Gly Leu Ala Ala
1 5 10 15

Thr Gly Ser Ser Val Pro Glu Pro Pro Gly Gly Pro Asn Ala Thr Leu

20 25 30

Asn Ser Ser Trp Ala Ser Pro Thr Glu Pro Ser Ser Leu Glu Asp Leu

35 40 45

Val Ala Thr Gly Thr Ile Gly Thr Leu Leu Ser Ala Met Gly Val Val

50 55 60

Gly Val Val Gly Asn Ala Tyr Thr Leu Val Val Thr Cys Arg Ser Leu

65 70 75 80

Arg Ala Val Ala Ser Met Tyr Val Tyr Val Val Asn Leu Ala Leu Ala

85 90 95

Asp Leu Leu Tyr Leu Leu Ser Ile Pro Phe Ile Val Ala Thr Tyr Val

100 105 110

Thr Lys Glu Trp His Phe Gly Asp Val Gly Cys Arg Val Leu Phe Gly

115 120 125

Leu Asp Phe Leu Thr Met His Ala Ser Ile Phe Thr Leu Thr Val Met

130 135 140

Ser Ser Glu Arg Tyr Ala Ala Val Leu Arg Pro Leu Asp Thr Val Gln

145 150 155 160

Arg Pro Lys Gly Tyr Arg Lys Leu Leu Ala Leu Gly Thr Trp Leu Leu

165 170 175

Ala Leu Leu Leu Thr Leu Pro Val Met Leu Ala Met Arg Leu Val Arg

180 185 190

Arg Gly Pro Lys Ser Leu Cys Leu Pro Ala Trp Gly Pro Arg Ala His

195 200 205

Arg Ala Tyr Leu Thr Leu Leu Phe Ala Thr Ser Ile Ala Gly Pro Gly

210 215 220

Leu Leu Ile Gly Leu Leu Tyr Ala Arg Leu Ala Arg Ala Tyr Arg Arg

225 230 235 240

Ser Gln Arg Ala Ser Phe Lys Arg Ala Arg Arg Pro Gly Ala Arg Ala

245 250 255

Leu Arg Leu Val Leu Gly Ile Val Leu Leu Phe Trp Ala Cys Phe Leu

260 265 270

Pro Phe Trp Leu Trp Gln Leu Leu Ala Gln Tyr His Gln Ala Pro Leu

275 280 285

Ala Pro Arg Thr Ala Arg Ile Val Asn Tyr Leu Thr Thr Cys Leu Thr

290 295 300

Tyr Gly Asn Ser Cys Ala Asn Pro Phe Leu Tyr Thr Leu Leu Thr Arg

305 310 315 320

Asn Tyr Arg Asp His Leu Arg Gly Arg Val Arg Gly Pro Gly Ser Gly

325 330 335

Gly Gly Arg Gly Pro Val Pro Ser Leu Gln Pro Arg Ala Arg Phe Gln

340 345 350

Arg Cys Ser Gly Arg Ser Leu Ser Ser Cys Ser Pro Gln Pro Thr Asp

355 360 365

Ser Leu Val Leu Ala Pro Ala Ala Pro Ala Arg Pro Ala Pro Glu Gly

370 375 380

Pro Arg Ala Pro Ala

385